(19) Europäisches Patentamt
European Patent Office
Office européen des brevets

(11) EP 1 119 551 B1

(12) EUROPEAN PATENT SPECIFICATION

(45) Date of publication and mention of the grant of the patent: **29.12.2004 Bulletin 2004/53**

(21) Application number: **99947738.3**

(22) Date of filing: **04.10.1999**

(51) Int Cl.[7]: **C07D 211/24**, A61K 31/445, C07C 255/57, C07C 235/66, C07D 417/12, C07D 211/26, C07F 9/59, C07D 413/10, C07D 401/04, C07D 211/22, C07D 211/34, C07D 211/20

(86) International application number: **PCT/GB1999/003274**

(87) International publication number: **WO 2000/020389 (13.04.2000 Gazette 2000/15)**

(54) **NAPHTHALENECARBOXAMIDES AS TACHYKININ RECEPTOR ANTAGONISTS**

NAPHTHALIN-CARBOXAMIDE ALS TACHYKININ REZEPTORANTAGONISTEN

NAPHTALENE-CARBOXAMIDES UTILISES EN TANT QU'ANTAGONISTES DES RECEPTEURS DES TACHYKININES

(84) Designated Contracting States:
**AT BE CH CY DE DK ES FI FR GB GR IE IT LI LU MC NL PT SE**
Designated Extension States:
**LT LV MK RO SI**

(30) Priority: **07.10.1998 GB 9821699**
**17.03.1999 GB 9906278**
**30.04.1999 GB 9909839**

(43) Date of publication of application: **01.08.2001 Bulletin 2001/31**

(60) Divisional application:
**04006920.5 / 1 433 783**
**04007194.6**

(73) Proprietor: **AstraZeneca AB**
**151 85 Södertälje (SE)**

(72) Inventors:
- **BERNSTEIN, Peter, Robert**
  **Wilmington, DE 19850-5437 (US)**
- **DEDINAS, Robert, Frank**
  **Wilmington, DE 19850-5437 (US)**
- **OHNMACHT, Cyrus, John**
  **Wilmington, DE 19850-5437 (US)**
- **RUSSELL, Keith**
  **Wilmington, DE 19850-5437 (US)**
- **SHEWOOD, Scott, Alan**
  **New Castle, DE 19720 (US)**

(56) References cited:
**EP-A- 0 428 434**
**EP-A- 0 474 561**
**EP-A- 0 515 240**
**EP-A- 0 559 538**
**EP-A- 0 630 887**

- **EMONDS-ALT X ET AL: "PHARMACOLOGICAL PROFILE AND CHEMICAL SYNTHESIS OF SR 48968, A NON -PEPTIDE ANTAGONIST OF THE NEUROKININ A (NK2) RECEPTOR" BIOORGANIC & MEDICINAL CHEMISTRY LETTERS, vol. 3, no. 5, 1 January 1993 (1993-01-01), pages 925-930, XP002068450 ISSN: 0960-894X**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 119 551 B1

Printed by Jouve, 75001 PARIS (FR)

## Description

## Background

The mammalian neurokinins comprise a class of peptide neurotransmitters which are found in the peripheral and central nervous systems. The three principal neurokinins are Substance P (SP), Neurokinin A (NKA) and Neurokinin B (NKB).

There are also N-terminally extended forms of at least NKA. At least three receptor types are known for the three principal neurokinins. Based upon their relative selectivities favoring the neurokinin agonists SP, NKA and NKB, the receptors are classifed as neurokinin 1 ($NK_1$), neurokinin 2 ($NK_2$) and neurokinin 3 ($NK_3$) receptors, respectively. In the periphery, SP and NKA are localized in C-afferent sensory neurons, which neurons are characterized by non-myelinated nerve endings known as C-fibers, and are released by selective depolarization of these neurons, or selective stimulation of the C-fibers. C-Fibers are located in the airway epithelium, and the tachykinins are known to cause profound effects which clearly parallel many of the symptoms observed in asthmatics. The effects of release or introduction of tachykinins in mammalian airways include bronchoconstriction, increased microvascular permeability, vasodilation, increased mucus secretion and activation of mast cells. Thus, the tachykinins are implicated in the pathophysiology and airway hyperresponsiveness observed in asthmatics; and blockade of the action of released tachykinins may be useful in the treatment of asthma and related conditions. A cyclopeptide antagonist (FK-224) selective for both $NK_1$ and $NK_2$ receptors has demonstrated clinical efficacy in human patients suffering from asthma and chronic bronchitis. M. Ichinose, et al., Lancet, 1992, 340, 1248.

## Description

This invention relates to naphthalenecarboxamide compounds N-substituted by an substituted phenylpiperidinylbutyl group, to pharmaceutical compositions containing such compounds, as well as to their uses and processes for their preparation. These compounds antagonize the pharmacological actions of the endogenous neuropeptide tachykinins known as neurokinins, particularly at the neurokinin 1 ($NK_1$) and the neurokinin 2 ($NK_2$) receptors. These compounds are useful whenever such antagonism is desired. Thus, such compounds are of value in the treatment of those diseases in which Substance P and Neurokinin A are implicated, for example, in the treatment of asthma, anxiety, depression, emesis, urinary incontinence and related conditions.

The N-substituted naphthalenecarboxamide compounds of the present invention show a high degree of both $NK_1$ and/or $NK_2$ receptor antagonist activity. Additionally, by manipulation of the substituents on the naphthalene and piperidine rings of the formula (I), the ratio of activity at the $NK_1$ and $NK_2$ receptors can be modified as desired, affording compounds that are predominantly active at either $NK_1$ or $NK_2$ receptors, or affording compounds with a balanced activity and, as such, are particularly useful when combined antagonism of both receptors is desired. In particular, the compounds of the present invention also possess a high degree of $NK_1$ and/or $NK_2$ antagonism upon oral administration.

Accordingly, the present invention provides the compounds of the general formula (**I**):

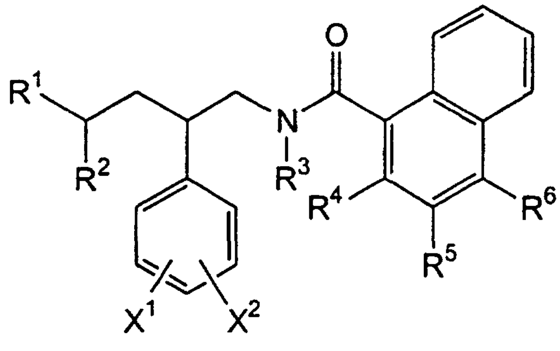

**(I)**

wherein:

$R^1$, in one respect, has the formula

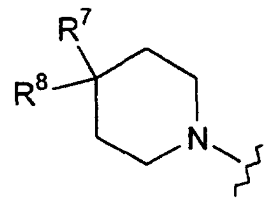

wherein $R^7$ and $R^8$ are as defined below to give general formula (**Ia**).

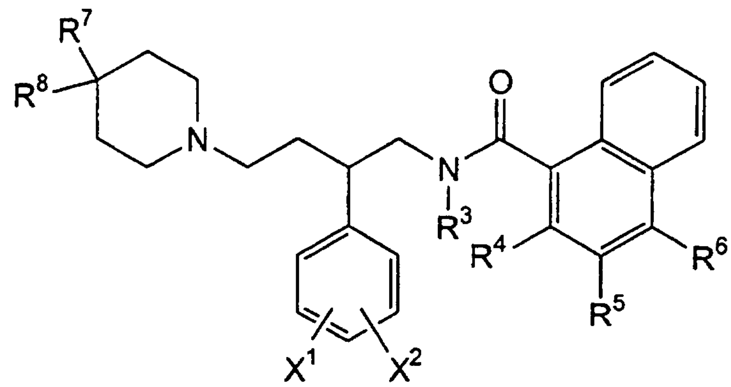

**(Ia)**

Intermediates, wherein $R^1$ is oxo ( =O, forming an aldehyde, -CHO) or $R^1$ is $OR^a$ are also active compounds in their own right.

In a further aspect $R^1$ is $-OR^a$ wherein $R^a$ is hydrogen or $C_{1-6}$alkyl. Preferably, $R^a$ is hydrogen, methyl or ethyl and in particular $R^a$ is hydrogen. In yet a further aspect $R^a$ may represent an ester forming group $-C(=O)R^b$, wherein $R^b$ is $C_{1-6}$alkyl for example methyl, phenyl or phenyl $C_{1-6}$alkyl for example benzyl.

$R^2$ is H or $R^1$ and $R^2$ together represent the ketal of an aldehyde, for example, of the formula $(R^cO)CH(OR^d)$- wherein $R^c$ and $R^d$ are independently selected from $C_{1-6}$alkyl, or together form a $C_{2-4}$methylene chain thus forming a dioxo ring. More suitably, $R^c$ and $R^d$ have the same value and are both methyl or are both ethyl

When $R^1$ is the piperidino species shown above (**Ia**), $R^2$ is H.

$R^3$ is hydrogen or $C_{1-6}$alkyl for example methyl, ethyl, n-propyl or cyclopropyl. Preferably, $R^3$ is methyl.

$R^4$, $R^5$ and $R^6$ are each, independently, hydroxy; cyano; nitro; trifluoromethoxy; trifluoromethyl; $C_{1-6}$alkylsulfonyl for example methylsulphonyl; halo for example chloro, bromo, fluoro or iodo; $C_{1-6}$alkoxy for example methoxy, ethoxy or propoxy; $C_{1-6}$alkyl for example methyl or ethyl; cyano$C_{1-6}$alkyl for example cyanomethyl; $C_{2-6}$alkenyl for example ethenyl, prop-1-enyl or prop-2-enyl; $C_{2-6}$alkynyl for example ethynyl; carboxy, $C_{1-6}$alkoxycarbonyl for example methoxycarbonyl; carbamoyl; $C_{1-6}$alkylcarbamoyl for example methylcarbamoyl or ethylcarbamoyl; di-$C_{1-6}$alkylcarbamoyl for example di-methylcarbamoyl; $C_{1-6}$alkanoyl for example acetyl or propionyl; $C_{1-6}$alkanoylamino for example acetylamino or propionylamino; aminosulfonyl; and substituted $C_{1-6}$alkyl for example methyl substituted by any of the hereinabove substituents. Additionally, $R^6$ may be hydrogen.

Favourably, $R^4$ is $C_{1-6}$alkyl for example methyl or ethyl; $C_{1-6}$alkoxy for example methoxy or ethoxy; or halo for example fluoro, chloro, bromo or iodo. Preferably, $R^4$ is methyl, ethyl, methoxy, ethoxy or fluoro. More preferably, $R^4$ is methoxy or ethyl, most preferably, methoxy.

Preferably, $R^5$ is cyano or nitro; more preferably, $R^5$ is cyano.

Preferably, $R^6$ is hydrogen, methoxy, cyano or nitro.

$R^7$ is a substituted phenyl group. "Substituted phenyl" means substituted in at least the ortho position by $C_{1-6}$alkylthio for example methylthio; $C_{1-6}$alkylsulfinyl for example methylsulfinyl, ethylsulfinyl or propyisulfinyl; $C_{1-6}$alkylsulfonyl for example methylsulfonyl or ethylsulfonyl; trifluoromethylthio; trifluoromethylsulfinyl; $C_{1-6}$alkanesulfonamido for example methanesulfonamido; $C_{1-6}$alkanoyl for example acetyl or propionyl; $C_{1-6}$alkoxycarbonyl for example methoxycarbonyl; succinamido; carbamoyl; $C_{1-6}$alkylcarbamoyl for example methylcarbamoyl; di-$C_{1-6}$alkylcarbamoyl for example dimethylcarbamoyl; $C_{1-6}$alkoxy-$C_{1-6}$alkylcarbamoyl for example N-methoxy, N-methylcarbamoyl; $C_{1-6}$alkanoylamino for example acetylamino; ureido, $C_{1-6}$ureido for example methylureido; di-$C_{1-6}$alkylureido for example dimethylureido; amino; $C_{1-6}$alkylamino for example methylamino or ethylamino; or

di-$C_{1-6}$alkylamino for example dimethylamino. Preferred values for the ortho-substituent are methylsulfinyl, ethylsulfinyl, propylsulfinyl, methylsulfonyl, trifluoromethylthio, trifluoromethylsulfinyl, methanesulfonamido, acetyl, methoxycarbonyl, succinamido, carbamoyl, methylcarbamoyl, dimethylcarbamoyl, N-methoxy, N-methylcarbamoyl, acetylamino, ureido, methylureido, dimethylureido, amino, methylamino or dimethylamino. In particular the ortho-substituent is methylsulfinyl, methylsulfonyl, methylureido, dimethylureido, amino, methylamino or dimethylamino. Of these methylsulfinyl is particularly preferred. The $R^7$ substituted phenyl group optionally may bear further substituents.

Suitable further substituents, which are optional, for the ortho substituted phenyl ring in $R^7$ include $C_{1-6}$alkyl for example methyl or ethyl; $C_{1-6}$alkylthio for example methylthio or ethylthio; $C_{1-6}$alkylsulfinyl for example methylsulfinyl, ethylsulfinyl or propoxysulfinyl; $C_{1-6}$alkylsulfonyl for example methylsulfonyl or ethylsulfonyl; $C_{1-6}$alkoxy for example methoxy, ethoxy or propoxy; halo for example bromo, fluoro, chloro or iodo; carboxy; $C_{1-6}$alkoxycarbonyl for example methoxycarbonyl; $C_{1-6}$alkanoyl for example acetyl or propionyl; nitro; amino; $C_{1-6}$alkylamino for example methylamino or ethylamino; di-$C_{1-6}$alkylamino where the alkyl groups may be the same or different, for example dimethylamino; trifluoromethyl; $CF_3S(O)_n$ wherein n is 0, 1 or 2, for example, trifluoromethylthio, trifluoromethylsulfinyl or trifluoromethylsulfonyl; $C_{1-6}$alkanoylamino for example acetylamino or propionylamino; $C_{1-6}$alkylsulphonamido for example methylsulphonamido; ureido; $C_{1-6}$alkylureido for example methylureido (MeNHC(=O)NH-), di-$C_{1-6}$alkylureido for example dimethylureido ($Me_2NC(=O)NH$-); carbamoyl; $C_{1-6}$alkylcarbamoyl for example methylcarbamoyl; di-$C_{1-6}$alkylcarbamoyl where the alkyl groups may be the same or different, for example dimethylcarbamoyl; and $C_{1-6}$alkyl for example methyl substituted by any of the hereinabove substituents.

Preferred further substituents if present, for the ortho-substituted phenyl ring, are methyl, methoxy, acetyl, acetylamino, methoxycarbonyl, methanesulfonylamino, methylsulfinyl, methylsulfonyl, trifluoromethyl, trifluoromethylthio, trifluoromethylsulfinyl, bromo, fluoro, chloro, hydroxy, carbamoyl, methylcarbamoyl, dimethylcarbamoylmethylureido and dimethylureido. In particular these preferred substituents may be at the 4-position of the phenyl ring.

Favourably, the ortho substituted phenyl ring is not substituted further or is substituted by up to three optional substituents. In particular the ortho-substituted phenyl ring is not substituted further or is substituted at the 4-position, that is the position para to the bond with the piperidine ring, so forming a 2,4-disubstituted phenyl group, preferably a 2-MeSO, 4-substituted phenyl group.

Thus a preferred class of compounds is that wherein $R^7$ is of the formula (Ib):

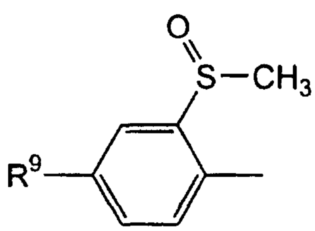

**(Ib)**

wherein $R^9$ is hydrogen, $C_{1-6}$alkoxy for example methoxy or ethoxy, halo for example bromo, chloro or fluoro, $C_{1-6}$alkylsulfinyl for example methylsulfinyl or carboxy. In particular $R^9$ is hydrogen, $C_{1-6}$alkoxy or halo. Most particularly $R^9$ is hydrogen, methoxy or fluoro.

The compounds of the invention have a number of chiral centers. It is preferred that the ortho-methylsulfinyl substituent, if present, has the stereochemistry depicted in formula (Ic):

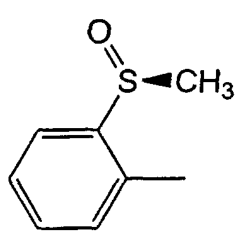

**(Ic)**

$R^8$ is hydrogen; hydroxy; $C_{1-6}$alkoxy for example methoxy or ethoxy; $C_{1-6}$alkanoyloxy for example acetyloxy or propionyloxy; $C_{1-6}$alkanoyl for example acethyl or propionyl; $C_{1-6}$alkoxycarbonyl for example methoxycarbonyl or ethoxycarbonyl; $C_{1-6}$alkanoylamino for example acetylamino; $C_{1-6}$alkyl for example methyl or ethyl; carbamoyl; $C_{1-6}$alkylcarbamoyl for example methylcarbamoyl or ethylcarbamoyl or di-$C_{1-6}$alkylcarbamoyl for example dimethylcarbamoyl.

Preferably $R^8$ is hydrogen, hydroxy, methoxycarbonyl, methylcarbamoyl or dimethylcarbamoyl. More preferably $R^8$ is hydrogen or hydroxy; most preferably $R^8$ is hydrogen.

The compounds of the present invention possess a number of chiral centres, at -CH(Ph-$X^1$,$X^2$)-, and possibly in the optional substituents (for example the MeSO-substituent) on either (or both) of the phenyl and naphth-1-yl groups. The present invention covers all isomers, diastereoisomers and mixtures thereof that antagonise $NK_1$ and/or $NK_2$.

The preferred configuration at -CH(Ph-$X^1$,$X^2$)- is shown in formula (**Id**) hereinbelow:

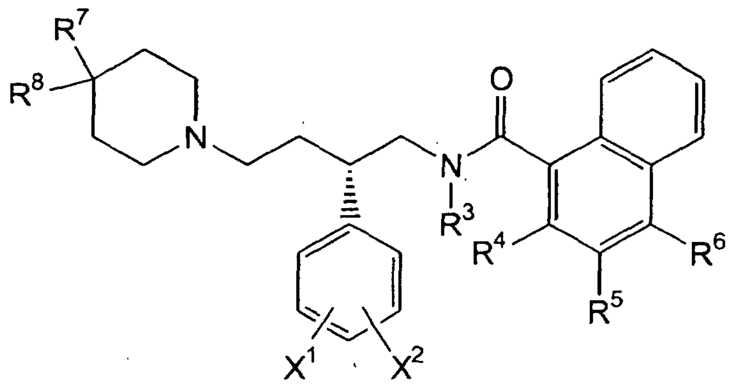

**(Id)**

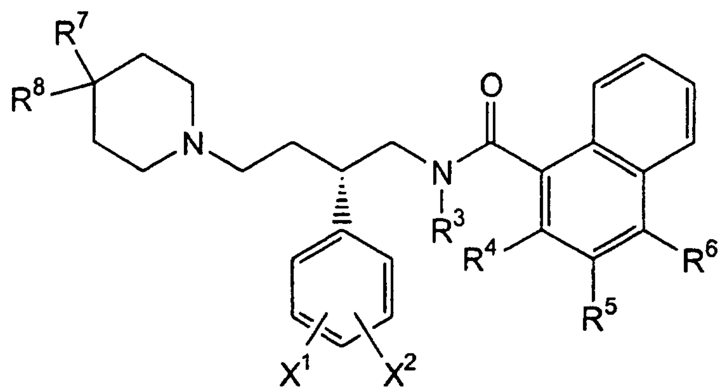

**(Id)**

$X^1$ and $X^2$ are independently hydrogen or halo, provided that at least one of $X^1$ or $X^2$ is halo. Favourably, $X^1$ and $X^2$ are both chloro. In a preferred aspect Ph-$X^1$,$X^2$ is 3,4-dichlorophenyl.

A preferred class of compounds is that of the formula (II):

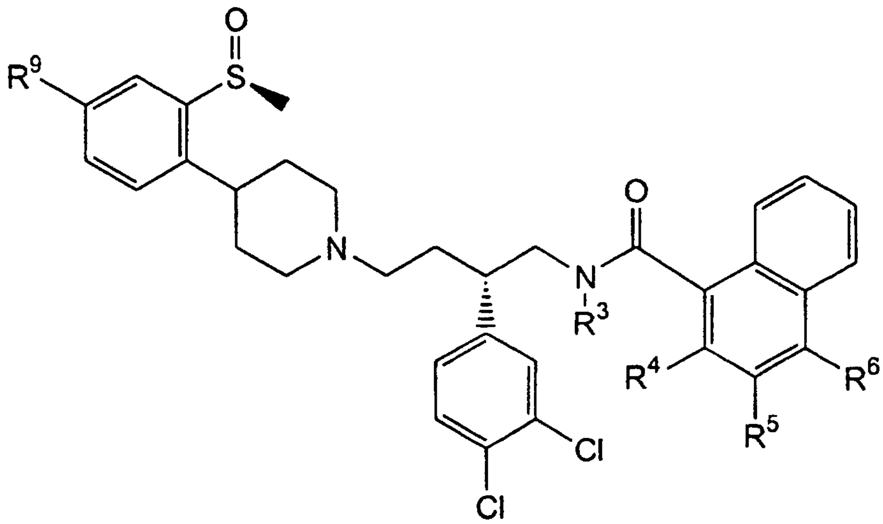

(II)

wherein $R^3$ is as hereinbefore defined and $R^4$-$R^6$ are selected from cyano, nitro, methoxy and fluoro additionally, $R_6$ may be hydrogen. In one particular aspect, in the compounds of the formula (II), $R^9$ is hydrogen, methoxy or fluoro, $R^4$ is hydrogen or fluoro, $R^6$ is hydrogen, and $R^5$ is cyano or nitro. In another particular aspect, $R^3$ is hydrogen, methoxy or fluoro, $R^4$ and $R^5$ are hydrogen, and $R^6$ is cyano or nitro. In a further particular aspect, $R^9$ is hydrogen, methoxy or fluoro, $R^4$ is methoxy, $R^6$ is hydrogen, and $R^5$ is cyano or nitro.

The most preferred structures are

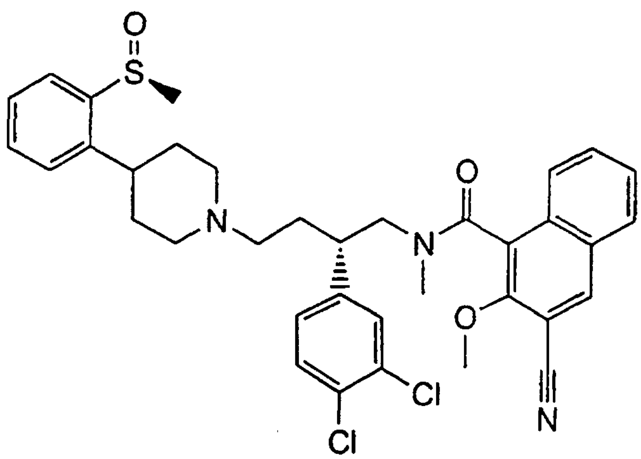

and

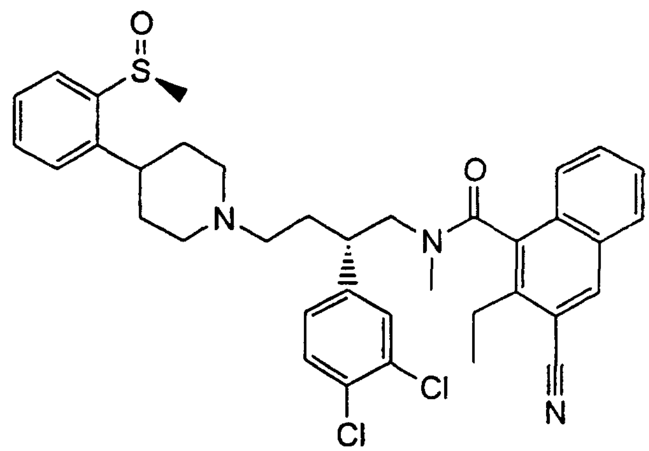

Particular compounds of this invention are provided as the Examples hereinbelow.

$C_{Y}$-$_{Z}$alkyl, unless otherwise specified, means an alkyl chain containing a minimum Y total carbon atoms and a maximum Z total carbon atoms. These alkyl chains may be branched or unbranched, cyclic, acyclic or a combination of cyclic and acyclic. For example, the following substituents would be included in the general description "$C_{4}$-$_{7}$alkyl":

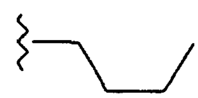

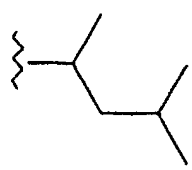

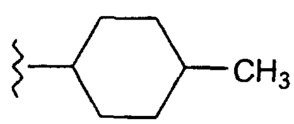

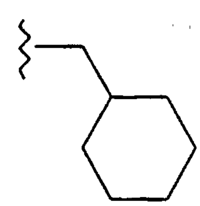

Pharmaceutically-acceptable salts may be prepared from the corresponding acid in conventional manner. Non-pharmaceutically-acceptable salts may be useful as intermediates and as such are another aspect of the present invention.

The compounds of the present invention are capable of forming salts with various inorganic and organic acids and bases and such salts are also within the scope of this invention. Examples of such acid addition salts include acetate, adipate, ascorbate, benzoate, benzenesulfonate, bisulfate, butyrate, camphorate, camphorsulfonate, citrate, cyclohexyl sulfamate, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, 2-naphthalenesulfonate, nitrate, oxalate, pamoate, persulfate, phenylacetate, phosphate, picrate, pivalate, propionate, quinate, salicylate, stearate, succinate, sulfamate, sulfanilate, sulfate, tartrate, tosylate (p-toluenesulfonate), and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quatemized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula (I) or a pharmaceutically acceptable salt thereof for the therapeutic treatment (including prophylactic treatment) of mammals including humans, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt and pharmaceutically acceptable carrier.

The pharmaceutical compositions of this invention may be administered in standard manner for the disease condition that it is desired to treat, for example by oral, topical, parenteral, buccal, nasal, vaginal or rectal adminstration or by inhalation or insufflation. For these purposes the compounds of this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

In addition to the compounds of the present invention the pharmaceutical composition of this invention may also contain, or be co-administered (simultaneously or sequentially) with, one or more pharmacological agents of value in treating one or more disease conditions referred to herein.

The pharmaceutical compositions of this invention will normally be administered to humans so that, for example, a daily dose of 0.01 to 25 mg/kg body weight (and preferably of 0.1 to 5 mg/kg body weight) is received. This daily dose may be given in divided doses as necessary, the precise amount of the compound received and the route of administration depending on the weight, age and sex of the patient being treated and on the particular disease condition being treated according to principles known in the art.

Typically unit dosage forms will contain about 1 mg to 500 mg of a compound of this invention. For example a tablet or capsule for oral administration may conveniently contain up to 250 mg (and typically 5 to 100 mg) of a compound of the formula (I) or a pharmaceutically acceptable salt thereof. In another example, for administration by inhalation, a compound of the formula (I) or a pharmaceutically acceptable salt thereof may be administered in a daily dosage range of 5 to 100 mg, in a single dose or divided into two to four daily doses. In a further example, for administration by intravenous or intramuscular injection or infusion, a sterile solution or suspension containing up to 10% w/w (and typically 5% w/w) of a compound of the formula (I) or a pharmaceutically acceptable salt thereof may be used.

The present invention also provides the use of a compound of the formula (I) or a pharmaceutically acceptable salt thereof in the preparation of a medicament for use in a disease condition wherein antagonism of the $NK_1$ and/or $NK_2$ receptors is beneficial.

The compounds of the formula (I) and their pharmaceutically acceptable salts may be made by processes as described and exemplified herein and by processes similar thereto and by processes known in the chemical art. If not commercially available, starting materials for these processes may be made by procedures which are selected from the chemical art using techniques which are similar or analogous to the synthesis of known compounds.

In another aspect the present invention provides a process for preparing a compound of the formula (I) or a pharmaceutically acceptable salt thereof which process comprises:

a) reacting a compound of the formula (III) with a compound of the formula (IV):

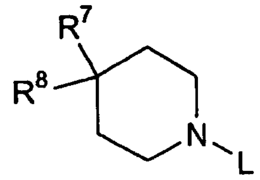

(III)

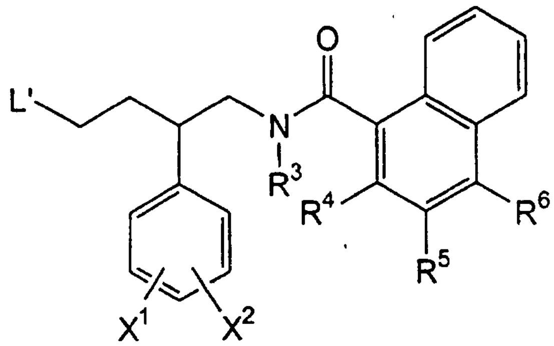

(IV)

wherein $R^3$ through $R^8$, and $X^1$ and $X^2$ are as hereinbefore defined; and L and L' are groups such that reductive amination of the compounds of the formulae (III) and (IV) forms a N-C bond; or

b) reacting a compound of the formula (V) with a compound of the formula (VI):

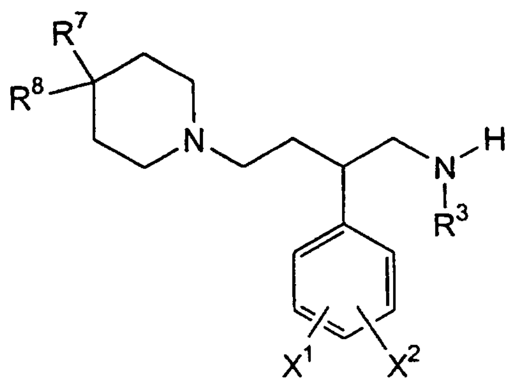

(V)

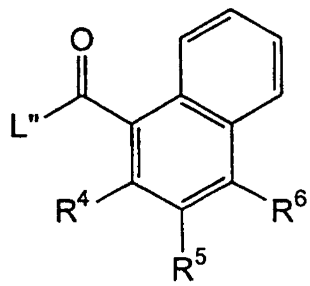

(VI)

wherein $R^3$ through $R^8$ and $X^1$ and $X^2$ are as hereinbefore defined; and L" is a leaving group; wherein any other functional group is protected, if necessary, and:

i) removing any protecting groups;
ii) optionally forming a pharmaceutically-acceptable salt.

Protecting groups may in general be chosen from any of the groups described in the literature or known to the skilled chemist as appropriate for the protection of the group in question, and may be introduced and removed by conventional methods; see for example Protecting Groups in Organic Chemistry; Theodora W. Greene. Methods of removal are chosen so as to effect removal of the protecting group with minimum disturbance of groups elsewhere in the molecule.

It will also be appreciated that certain of the various optional substituents in the compounds of the formula (I) may be introduced by standard aromatic substitution reactions or generated by conventional functional group modifications either prior to or immediately following the processes described hereinabove. The reagents and reaction conditions for such procedures are well known in the chemical art.

The compounds of the formulae (III) and (IV) are reacted under conditions of reductive amination. Typically in the compounds of the formula (III) L is hydrogen.

Typically in the compounds of the formula (IV) L' is an oxo group so forming an aldehyde moiety. The reaction is typically performed at a non-extreme temperature, for example 0 - 100 °C, suitably ambient temperature in a substantially inert solvent for example dichloromethane. Typical reducing agents include borohydrides such as sodium cyanoborohydride.

The compounds of the formula (III) are known or made be prepared in conventional manner. The compounds of the formula (IV) may be prepared, for example, by reacting a compound of the formula (VI) with a compound of the formula (VII):

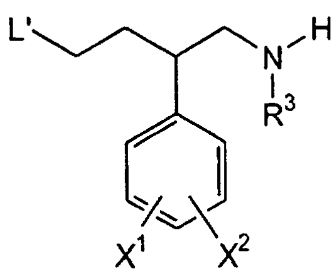

(VII)

wherein $R^3$, L', $X^1$ and $X^2$ are as hereinbefore defined under conventional acylation conditions.

The compounds of the formulae (V) and (VI) may be reacted under conventional acylation conditions wherein

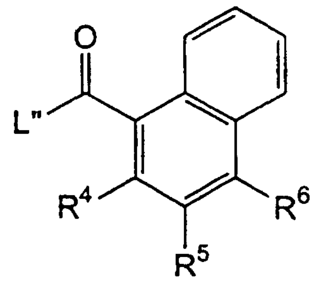

is an acid or an activated acid derivative. Such activated acid derivatives are well known in the literature. They may be formed in situ from the acid or they may be prepared, isolated and subsequently reacted. Typically L" is chloro thereby forming the acid chloride. Typically the acylation reaction is performed in the presence of a non-nucleophilic base, for example diisopropylethylamine, in a substantially inert solvent at a non-extreme temperature.

The compounds of the formula (VII) are known or may be prepared in conventional manner. Certain compounds of the formulae (IV) and (VI) are novel and form part of the present invention. In particular the compounds of the formula (VI) wherein the naphth-1-yl group is substituted by a methoxy group at the 2-position and by a cyano group at the 3-position are novel.

Accordingly, in another aspect the present invention provides a compound of the formula (VIII):

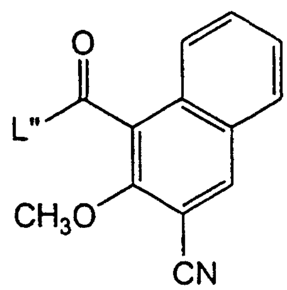

(VIII)

wherein L" is as hereinbefore defined; preferably L" is hydrogen or a leaving group such as chloro.

In another aspect the present invention provides a compound of the formulae (IX):

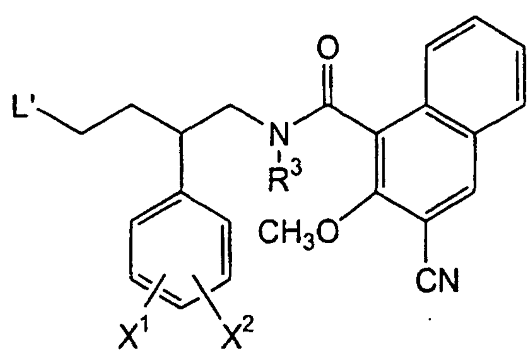

(IX)

wherein $R^3$, $X^1$, $X^2$ and L' are as hereinbefore defined.

It is well known in the art how to prepare optically-active forms (for example, by resolution of the racemic form or by synthesis from optically-active starting materials) and how to determine the $NK_1$ and $NK_2$ antagonist properties by the standard tests known in the art and those described hereinafter.

Some individual compounds within the scope of this invention may contain double bonds. Representations

of double bonds in this invention are meant to include both the E and the Z isomer of the double bond. Additionally, some species within the scope of this invention may contain one or more asymmetric centers. This invention includes the use of any of the optically pure stereoisomers as well as any combination of stereoisomers.

The following biological test methods, data and Examples serve to illustrate and further describe the invention.

The utility of a compound of the invention or a pharmaceutically acceptable salt thereof (hereinafter, collectively referred to as a "compound") may be demonstrated by standard tests and clinical studies, including those disclosed in the publications described below.

**SP Receptor Binding Assay (Test A)**

The ability of a compound of the invention to antagonize the binding of SP at the $NK_1$ receptor may be demonstrated using an assay using the human $NK_1$ receptor expressed in Mouse Erythroleukemia (MEL) cells. The human NK, receptor was isolated and characterized as described in: B. Hopkins, et al. "Isolation and characterization of the human lung $NK_1$ receptor cDNA" Biochem. Biophys. Res. Comm., 1991, 180, 1110-1117; and the NK, receptor was expressed in Mouse Erythroleukemia (MEL) cells using a procedure similar to that described in Test B below.

**Neurokinin A (NKA) Receptor Binding Assay (Test B)**

The ability of a compound of the invention to antagonize the binding of NKA at the $NK_2$ receptor may be demonstrated using an assay using the human $NK_2$ receptor expressed in Mouse Erythroleukemia (MEL) cells, as described in: Aharony, D., et al. "Isolation and Pharmacological Characterization of a Hampster Neurokinin A Receptor cDNA" Molecular Pharmacology, 1994, 45, 9-19.

The selectivity of a compound for binding at the $NK_1$ and the $NK_2$ receptors may be shown by determining its binding at other receptors using standard assays, for example, one using a tritiated derivative of NKB in a tissue preparation selective for NK3 receptors. In general, the compounds of the invention which were tested demonstrated statistically significant binding activity in Test A and Test B with a $K_i$ of 1 mM or much less typically being measured.

**Rabbit Pulmonary Artery: $NK_1$ in vitro functional assay (Test C)**

The ability of a compound of the invention to antagonize the action of the agonist Ac-[$Arg^6$, $Sar^9$, $Met(O_2)^{11}$] Substance P (6-11), ASMSP, in a pulmonary tissue may be demonstrated as follows.

Male New Zealand white rabbits are euthanized via i.v. injection into the ear vein with 60 mg/kg Nembutal (50 mg/mL). Preceding the Nembutal into the vein is Heparin (1000 units/mL) at 0.0025 mL/kg for anticoagulant purposes. The chest cavity is opened from the top of the rib cage to the sternum and the heart, lungs and part of the trachea are removed. The pulmonary arteries are isolated from the rest of the tissues and cut in half to serve as pairs.

The segments are suspended between stainless steel stirrups, so as not to remove any of the endothelium, and placed in water-jacketed (37.0 °C) tissue baths containing physiological salt solution of the following composition (mM): NaCl, 118.0; KCl, 4.7; $CaCl_2$, 1.8; $MgCl_2$, 0.54; $NaH_2PO_4$, 1.0; $NaHCO_3$, 25.0; glucose, 11.0; indomethacin, 0.005 (to inhibit cyclooxygenase); and *dl*-Propranolol, 0.001 (to block β receptors); gassed continuously with 95% $O_2$-5% $CO_2$. Responses are measured on a Grass polygraph via Grass FT-03 transducers.

Initial tension placed on each tissue is 2 grams, which is maintained throughout the 1.0 hour equilibration period. Tissues are washed with the physiological salt solution at 15 minute intervals. At the 30 and 45 minute wash the following treatments are added: $1 \times 10^{-6}$ M Thiorphan (to block E.C.3.4.24.11), $3 \times 10^{-8}$ M (S)-N-[2-(3,4-dichlorophenyl)-4-[4-(2-oxoperhydropyrimidin-1-yl)piperidino]butyl]-N-methylbenzamide (to block $NK_2$ receptors), and the given concentration of the compound being tested. At the end of the 1.0 h equilibration, $3 \times 10^{-6}$M Phenylephrine hydrochloride is added for 1.0 h. At the end of 1.0 h, a dose relaxation curve to ASMSP is done. Each tissue is treated as a individual and is considered finished when it fails to relax further for 2 consecutive doses. When a tissue is complete, $1 \times 10^{-3}$ M Papaverine is added for maximum relaxation.

Percent inhibition is determined when a tested compound produces a statistically significant ($p < 0.05$) reduction of the total relaxation which is calculated using the total relaxation of the Papaverine as 100%. Potencies of the compounds are determined by calculating the apparent dissociation constants ($K_B$) for each concentration tested using the standard equation:

$$KB= [antagonist]/ (dose\ ratio - 1)$$

where dose ratio = antilog[(agonist -log molar $EC_{50}$ without compound) - (-log molar $EC_{50}$ with compound)]. The $K_B$ values may be converted to the negative logarithms and expressed as -log molar KB (i.e. $pK_B$). For this evaluation,

complete concentration-response curves for agonist obtained in the absence and presence of the compound tested using paired pulmonary artery rings. The potency of the agonist is determined at 50% of its own maximum relaxation in each curve. The $EC_{50}$ values are converted to negative logarithms and expressed as -log molar $EC_{50}$.

**$NK_2$ in vitro functional assay (Test D)**

The ability of a compound of the invention to antagonize the action of the agonist [β-ala8] NKA (4-10), BANK, in a pulmonary tissue may be demonstrated as follows. Male New Zealand white rabbits are euthanized via i.v. injection into the ear vein with 60 mg/kg Nembutal (50 mg/mL). Preceding the Nembutal into the vein is Heparin (1000 units/mL) at 0.0025 mL/kg for anticoagulant purposes. The chest cavity is opened from the top of the rib cage to the sternum and a small incision is made into the heart so that the left and right pulmonary arteries can be cannulated with polyethylene tubing (PE260 and PE190 respectively). The pulmonary arteries are isolated from the rest of the tissues, then rubbed over an intimal surface to remove the endothelium, and cut in half to serve as pairs. The segments are suspended between stainless steel stirrups and placed in water-jacketed (37.0 °C) tissue baths containing physiological salt solution of the following composition (mM): NaCl, 118.0; KCl, 4.7; $CaCl_2$, 1.8; $MgCl_2$, 0.54; $NaH_2PO_4$, 1.0; $NaHCO_3$, 25.0; glucose, 11.0; and indomethacin, 0.005 (to inhibit cyclooxygenase); gassed continuously with 95% $O_2$-5% $CO_2$. Responses are measured on a Grass polygraph via Grass FT-03 transducers.

Initial tension placed on each tissue is 2 g, which is maintained throughout the 45 min equilibration period. Tissues are washed with the physiological salt solution at 15 min intervals. After the 45 min equilibration period, 3 x $10^{-2}$ M KCl is given for 60 min to test the viability of the tissues. The tissues are then washed extensively for 30 min. The concentration of the compound being tested is then added for 30 min. At the end of the 30 min, a cumulative dose response curve to BANK is performed. Each tissue is treated as a individual and is considered finished when it fails to contract further for 2 consecutive doses. When a tissue is complete, 3 x $10^{-2}$ M $BaCl_2$ is added for maximum contraction.

Percent inhibition is determined when a tested compound produces a statistically significant ($p < 0.05$) reduction of the total contraction which is calculated using the total contraction of the $BaCl_2$ as 100%. Potencies of the compounds are determined by calculating the apparent dissociation constants ($K_B$) for each concentration tested using the standard equation:

$$K_B = [\text{antagonist}] / (\text{dose ratio} - 1)$$

where dose ratio = antilog[(agonist -log molar $EC_{50}$ without compound) - (-log molar $EC_{50}$ with compound)]. The $K_B$ values may be converted to the negative logarithms and expressed as -log molar $K_B$ (i.e. $pK_B$). For this evaluation, complete concentration-response curves for agonist obtained in the absence and presence of the compound tested using paired pulmonary artery rings. The potency of the agonist is determined at 50% of its own maximum relaxation in each curve. The $EC_{50}$ values are converted to negative logarithms and expressed as -log molar $EC_{50}$.

**$NK_1$ and $NK_2$ in vivo functional assay (Test E)**

The activity of a compound as an antagonist of $NK_1$ and/or $NK_2$ receptors also may be demonstrated in vivo in laboratory animals as described in: Buckner et al. "Differential Blockade by Tachykinin $NK_1$ and $NK_2$ Receptor Antagonists of Bronchoconstriction Induced by Direct-Acting Agonists and the Indirect-Acting Mimetics Capsaicin, Serotonin and 2-Methyl-Serotonin in the Anesthetized Guinea Pig." J. Pharm. Exp. Ther.,1993, Vol 267(3), pp.1168-1175. The assay is carried out as follows.

Compounds are tested in anesthetized guinea pigs pretreated with i.v. indomethacin (10 mg/kg, 20 min), propranolol (0.5 mg/kg, 15 min), and thiorphan (10 mg/kg, 10 min).

Antagonists or vehicle are administered i.v. and orally, 30 and 120 min prior to increasing concentrations of agonist, respectively. The agonists used in these studies are ASMSP (Ac-[$Arg^6$,$Sar^9$,$Met(O_2)^{11}$]-SP(6-11)) and BANK (β-ala-8 NKA4-10).

Administered i.v., ASMSP is selective for NK, receptors, and BANK is selective for $NK_2$ receptors. Maximum response is defined as zero conductance ($G_L$, 1/Rp). $ED_{50}$ values are calculated (the dose of agonist resulting in a reduction of $G_L$ to 50% of baseline), and converted to the negative logarithm ($-\log ED_{50}$). The $ED_{50}$ values, obtained in the presence (P) and absence (A) of antagonist, are used to calculate a Dose Ratio (P/A), an expression of potency. Data are expressed as mean ± SEM and statistical differences were determined using ANOVA/Tukey-Kramer and Student's t-test , with $p < 0.05$ considered statistically significant.

Compounds of the present invention exhibit marked activity in the foregoing tests and are considered useful for the treatment of those diseases in which the $NK_1$ and/or $NK_2$ receptor is implicated, for example, in the treatment

of asthma and related conditions.

Results of testing of representative compounds of the present invention by the above methods are presented in the Table I

Table I

| | Rabbit Pulmonary Artery | |
|---|---|---|
| Example | $NK_1$ $pK_b$ (Test C) | $NK_2$ $pK_b$ (Test D) |
| 1 | 9.5 | 7.3 |
| 18 | 9.6 | 7.3 |

**Clinical Studies**

Clinical studies to demonstrate the efficacy of a compound of the invention may be carried out using standard methods. For example, the ability of a compound to prevent or treat the symptoms of asthma or asthma-like conditions may be demonstrated using a challenge of inhaled cold air or allergen and evaluation by standard pulmonary measurements such as, for example, $FEV_1$ (forced expiratory volume in one second) and FVC (forced vital capacity), analyzed by standard methods of statistical analysis.

It will be appreciated that the implications of a compound's activity in the above desribed Tests is not limited to asthma, but rather, that the Tests provide evidence of general antagonism of both SP and $NK_A$. SP and $NK_A$ have been implicated in the pathology of numerous diseases including: rheumatoid arthritis, Alzheimer's disease, cancer, schizophrenia, oedema, allergic rhinitis, inflammation, pain, gastrointestinal-hypermotility, anxiety, emesis, Huntington's disease, psychoses including depression, hypertension, migraine, bladder hypermotility and urticaria.

Accordingly, one feature of the invention is the use of a compound of formula I or a pharmaceutically acceptable salt thereof in the treatment of a disease in a human or other mammal in need thereof in which SP or $NK_A$ is implicated and antagonism of its action is desired.

Asthma is characterized by both chronic inflammation and hyperresponsiveness of the airways. The $NK_1$ receptor is known to mediate inflammation and mucus hypersecretion in airways; and the $NK_2$ receptor is involved in the control of the tone of bronchial smooth muscle. Thus, agents capable of antagonizing the actions of SP and NKA, at the $NK_1$ and $NK_2$ receptors, respectively, are capable of reducing both the chronic inflammation and the airway hyperresponsiveness which are symptomatic of asthma. It has been suggested that an antagonist having mixed affinity for $NK_1$ and $NK_2$ could be therapeutically superior to a receptor selective antagonist. C.M. Maggi "Tachykinin Receptors and Airway Pathophysiology" EUR. Respir. J., **1993**, 6, 735-742 at 739. Also, it has been suggested that a synergistic effect against bronchoconstriction may result from the simultaneous application of an $NK_1$ antagonist and an $NK_2$ antagonist. D.M. Foulon, et al. " $NK_1$ and $NK_2$ Receptors Mediated Tachykinin and Resiniferatoxin-induced Bronchospasm in Guinea Pigs" American Review of Respiratory Disease, 1993, 148, 915-921. Accordingly, another feature of the invention is the use of a compound of formula I or a pharmaceutically acceptable salt thereof in the treatment of asthma in a human or other mammal in need thereof. There is a possible role for Substance P antagonists in the treatment of depression. Accordingly, another feature of the invention is the use of a compound of formula (I) or a pharmaceutically acceptable salt thereof in the treatment of depression in a human or other mammal in need thereof.

Because of the range of effects attributable to the actions of SP and $NK_A$, compounds which are capable of blocking their actions may also be useful as tools for further evaluating the biological actions of other neurotransmitters in the tachykinin family. As a result, another feature of the invention is provided by the use of a compound of Formula I or a salt thereof as a pharmacological standard for the development and standardisation of new disease models or assays for use in developing new therapeutic agents for treating diseases in which SP or NKA are implicated or for assays for their diagnosis.

**EXAMPLES**

The invention will now be illustrated by the following non-limiting examples, in which, unless stated otherwise:

(i) temperatures are given in degrees Celsius (°C); unless otherwise stated, operations were carried out at room or ambient temperature, that is, at a temperature in the range of 18-25 °C;

(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60 °C;

(iii) chromatography means flash chromatography on silica gel; thin layer chromatography (TLC) was carried out

on silica gel plates;
(iv) in general, the course of reactions was followed by TLC and reaction times are given for illustration only;
(v) melting points are uncorrected and (dec) indicates decomposition;
(vi) final products had satisfactory proton nuclear magnetic resonance (NMR) spectra;
(vii) when given, NMR data is in the form of delta values for major diagnostic protons, given in parts per million (ppm) relative to tetramethylsilane (TMS) as an internal standard, determined at 300 MHz using deuterated chloroform ($CDCl_3$) as solvent; conventional abbreviations for signal shape are used; for AB spectra the directly observed shifts are reported; coupling constants (J) are given in Hz; Ar designates an aromatic proton when such an assignment is made;
(viii) reduced pressures are given as absolute pressures in pascals (Pa); elevated pressures are given as gauge pressures in bars;
(ix) solvent ratios are given in volume:volume (v/v) terms; and
(x) Mass spectra (MS) were run using an automated system with atmospheric pressure chemical ionization (APCI). Generally, only spectra where parent masses are observed are reported. The lowest mass major ion is reported for molecules where isotope splitting results in multiple mass spectral peaks (for example when chlorine is present).

Terms and abbreviations: Solvent mixture compositions are given as volume percentages or volume ratios. In cases where the NMR spectra are complex, only diagnostic signals are reported. atm; atmospheric pressure, Boc; t-butoxycarbonyl, Cbz; benzyloxycarbonyl, DCM; methylene chloride, DIPEA: diisopropylethylamine, DMF; N,N-dimethyl formamide, DMSO; dimethyl sulfoxide, $Et_2O$; diethyl ether, EtOAc; ethyl actate, h; hour(s), HPLC: high pressure liquid chromatography, min; minutes, NMR; nuclear magnetic resonance, psi; pounds per square inch, TFA; trifluoroacetic acid, THF; tetrahydrofuran.

Standard reductive amination refers to the typical procedure in which a solution of an amine (1-1.2 equivalents), an aldehyde (1-1.2 equivalents) and acetic acid (2 equivalents) are stirred in methanol for 5 to 60 min before adding $NaBH_3CN$ (1.7 equivalents). After 1-16 h the reaction is optionally concentrated, dissolved in DCM, and washed with saturated sodium bicarbonate and then purified by chromatography.

Standard Swem oxidation conditions refer to the oxidation of an alcohol to the corresponding aldehyde according to Mancuso, AJ; Huang, SL; Swem, D; J. Org. Chem.; 1978, 2840.

Standard formation of an acid chloride refers to the typical procedure in which a solution of a naphthoic or substituted naphthoic acid in DCM is stirred with 1-1.2 equivalents of oxalyl chloride and a catalytic amount of DMF for 1-12 h, concentrated under reduced pressure, and used without purification.

Standard acylation refers to the typical procedure in which an acid chloride (1-1.2 equivalents) is added to a stirred solution of an amine (1-1.2 equivalents) and triethylamine (2 equivalents) in DCM. After 1-16 h the reaction is optionally concentrated, dissolved in DCM, and washed with saturated sodium bicarbonate and then purified by chromatography.

Where noted that a final compound was converted to the citrate salt, the free base was combined with citric acid (1.0 equivalents) in methanol, concentrated under reduced pressure and dried under vacuum (25-70 °C). When indicated that a compound was isolated by filtration from $Et_2O$, the citrate salt of the compound was stirred in $Et_2O$ for 12-18 h, removed by filtration, washed with $Et_2O$, and dried under vacuum at 25-70 °C.

Where noted that a final compound was converted to the hydrochloride salt, a solution of HCl in $Et_2O$ was added with stirring to a solution of the purified free base in DCM or methanol. The resulting precipitate was collected by filtration and dried under vacuum.

Each compound bearing a 2-substituted naphthamide existed as a mixture of conformational isomers (atropisomers); this is believed to result from slow rotation about the amide and/or aryl bonds. Such compounds showed multiple peaks in HPLC chromatograms and highly complex NMR spectra. In some cases, the individual components of an atropiomeric mixture could be purified by reverse phase HPLC and the properties could be independently evaluated.

## Example 1

### N-[(S)-2-(3,4-Dichlorophenyl)-4-[4-[(S)-2-methylsulfinylphenyl]-1-piperidinyl]butyl]-N-methyl-2-methoxy-3-cyano-1-naphthamide citrate.

A solution of 2-methoxy-3-cyano-1-naphthoic acid (0.065 g), oxalyl chloride (0.039 g) and DMF (approximately 5 μL) was stirred for 1.5 h then concentrated to afford 2-methoxy-3-cyano-1-naphthoyl chloride as a white residue which was used directly. Using standard acylation conditions 2-methoxy-3-cyano-1-naphthoyl chloride (0.065 g) was reacted with N-[(S)-2-(3,4-dichlorophenyl)-4-[4-[(S)-2-methylsulfinylphenyl]-1-piperidinyl]butyl]-N-methylamine (0.136 g). The free base (0.170 g) was converted to the citrate salt. $^1H$ NMR (DMSO-$d_6$):δ 8.71-8.63 (m, 1H), 8.08-8.00 (m,

1H), 7.85-7.33 (m, 8.68, 1H), 7.11-6.85 (m, 0.6H), 6.31-6.28 (m, 0.47 1H), 4.57-0.96 (m, 22H); MS m/z 662 (M+H).

The requisite 2-methoxy-3-cyano-1-naphthoic acid was prepared as follows.

(a) 3-Hydroxy-4-iodo-2-naphthoic acid.

A mixture of NaOH (2.12 g) in methanol (100 mL) was stirred until the solution was homogeneous. Sodium iodide (3.98 g) and 3-hydroxy-2-naphthoic acid (5.00 g) were added and allowed to stir for 30 min. The resulting suspension was cooled to 0 °C and a 5.25% (w/v) aqueous solution of sodium hypochlorite was added dropwise and stirring continued for 1 h. Saturated sodium thiosulfate (25 mL) was added and after 5 min the solution was acidified to pH 2 by addition of 6 N HCl resulting in the formation of a yellow precipitate which was filtered and washed with water (50 mL). The precipitate was transferred to a round-bottomed flask, dissolved in methanol (70 mL) and toluene (100 mL), concentrated, redissolved in methanol (70 mL), concentrated, redissolved again in methanol (70 mL) and toluene (100 mL) and concentrated to afford the product as a yellow solid (6.26 g). MS m/z 313 (M-1). $^1$H NMR (DMSO-$d_6$): δ 12.41 (broad, 1 H), 8.63 (s, 1 H), 8.05-7.97 (m, 2 H), 7.70 (m, 1 H), 7.42 (m, 1H).

(b) Methyl 3-methoxy-4-iodo-2-naphthoate.

A solution of 3-hydroxy-4-iodo-2-naphthoic acid (8.0 g), dimethyl sulfate (8.03 g), powdered potassium carbonate (8.80 g), and dry acetone (150 mL) was heated under reflux for 18 h. The solution was cooled to room temperature, triethylamine (15 mL) was added, and stirring continued for 30 min. The solution was filtered through a pad of Celite and washed with dry acetone (50 mL). The filtrate was concentrated to a yellow oil, diluted with EtOAc, and washed successively with 1N HCl (100 mL), saturated aqueous sodium bicarbonate (100 mL), and brine (100 mL). The organic phase was dried (sodium sulfate), filtered, concentrated, and purified by chromatography (0-10% EtOAc in hexanes) to afford the product as a yellow oil (5.53 g). $^1$H NMR (DMSO-$d_6$) δ 8.47 (s, 1 H), 8.09 (m, 2 H), 7.74 (m, 1 H), 7.61 (m, 1 H), 3.94 (s, 3 H), 3.87 (s, 3 H).

(c) 1-Iodo-2-methoxy-3-cyanonaphthalene.

Based on the procedure of Wood, JL; Khatri, NA; Weinreb, SM; Tetrahedron Lett; 51, 4907 (1979), methyl 3-methoxy-4-iodo-2-naphthoate (5.0 g) was suspended in xylenes (100 mL), cooled to 0 °C, dimethylaluminum amide solution (approximately 37 mmol) was added and the solution heated under reflux for 2.5 h. The solution was then cooled to 0 °C and the solution was acidified to pH 2 by addition of IN HCl and extracted with EtOAc (3x100 mL).

The combined EtOAc extracts were washed with saturated aqueous sodium bicarbonate (150 mL) and brine (150 mL), dried (sodium sulfate), filtered, concentrated, and purified by chromatography (1:1 EtOAc:DCM, then 10-20% EtOAc in DCM) to afford the product as a white solid (3.29 g). $^1$H NMR (DMSO-$d_6$): δ 8.69 (s, 1 H), 8.24-8.04 (m, 2 H), 7.91-7.81 (m, 1 H), 7.76-7.65 (m, I H), 3.99 (s, 3 H); MS m/z 311 (M+H).

(d) Methyl 2-methoxy-3-cyano-1-naphthoate.

Through a suspension of 1-iodo-2-methoxy-3-cyanonaphthalene (0.250 g), $Pd(OAc)_2$ (0.018 g), triethylamine (0.081 g) and methanol (20 mL) was bubbled carbon monoxide for 25 min, then stirred at 70 °C under carbon monoxide (1 atm) for 18 h. The cooled solution was filtered, rinsed with methanol (20 mL) and DCM (20 mL), concentrated, preadsorbed onto silica (1 g) and purified by chromatography (0-10% EtOAc in hexanes) to afford the product as a white solid (0.113g). $^1$H NMR (DMSO-$d_6$): δ 8.78 (s, 1 H), 8.12-8.09 (m, 1 H), 7.84-7.78 (m, 2 H), 7.70-7.63 (m, 1 H), 4.02-4.01 (m, 6 H); IR (cm$^{-1}$): 2228, 1724, 1296, 1236, 1208, 1017.

(e) 2-Methoxy-3-cyano-1-naphthoic acid.

A solution of methyl 2-methoxy-3-cyano-1-naphthoate (0.113 g) and LiOH•$H_2O$ (0.0196 g) THF (3 mL), water (1 mL) and methanol (1 mL) was stirred overnight at room temperature. The solution was diluted with saturated sodium bicarbonate and extracted with $Et_2O$. The aqueous layer was acidified to pH 2 by addition of 1N HCl and extracted with $Et_2O$. The organic layer was washed with water (30 mL) and brine (40 mL), dried (sodium sulfate), filtered, and concentrated to a white solid. $^1$H NMR (DMSO-$d_6$): δ 14.06 (broad, 1 H), 8.08-8.02 (m, 1 H), 7.83-7.76 (m, 2 H), 7.69-7.63 (m, 1 H), 4.02 (s, 3 H); MS m/z: 226 (M-1).

The requisite N-[(S)-2-(3,4-dichlorophenyl)-4-[4-[(S)-2-methylsulfinylphenyl]-1-piperidinyl]butyl]-N-methylamine was prepared as follows.

(f) N-[(S)-2-(3,4-Dichlorophenyl)-4-[4-[(S)-2-methylsulfinylphenyl]-1-piperidinyl]butyl]-N-methyl-N-Boc-amine.

(S)-N-[2-(3,4-Dichlorophenyl)-4-oxobutyl]-N-methyl-N-Boc-amine (Miller, SC; WO 9505377) (51.7 g, 149.3 mmol), 4-[(S)-2-methylsulfinylphenyl]-piperidine (Shenvi, AB; Jacobs, RT; Miller, SC; Ohnmacht, CJ, Jr.; Veale, CA., WO 9516682) (36.7g, 164.3 mmol), and glacial acetic acid (9.9 g, 165.0 mmol) were dissolved in methanol (1000 mL), and the solution stirred for 15 min. Sodium cyanoborohydride (10.4 g, 165.5 mmol) was added in portions as a solid over 30 min. The mixture was stirred for 20 h, then treated with saturated sodium bicarbonate (500 mL). Methanol was removed in vacuo, and the aqueous residue was extracted with DCM (4x400 mL). The organic layer was washed with brine (300 mL), dried ($MgSO_4$), filtered, and concentrated in vacuo. The residue was purified by chromatography (0-6% methanol in DCM) to provide a white foam (77.2 g, 93%). MS: 553 (M+H). $^1$H-NMR ($CDCl_3$) δ 1.40 (s, 9H, t-$C_4H_9$); 1.61-2.04 (m, 9H, CH); 2.14-2.23 (m, 2H, CH); 2.62-2.79 (m, 6H, $NCH_3$, $SOCH_3$); 2.91-3.00 (m, 3H, CH); 3.27-3.54 (m, 2H, CH); 7.00-7.09 (m, 1H, aromatic); 7.21-7.53 (m, 5H, aromatic); 7.95-8.04 (m, 1H, aromatic).

(g) N-[(S)-2-(3,4-Dichlorophenyl)-4-[4-[(S)-2-methylsulfinylphenyl]-1-piperidinyl]butyl]-N-methylamine.

N-[(S)-2-(3,4-Dichlorophenyl)-4-[4-[(S)-2-methylsulfinylphenyl]-1-piperidinyl]butyl]-N-methylamine-N-Boc-amine (77.0 g, 139.0 mmol) was dissolved in DCM (1200 mL). To the stirred solution was added trifluoroacetic acid (160.0 g, 1.40 mol) dropwise over 15 min. The mixture was stirred for 4 h, then additional trifluoroacetic acid (80.0 g, 0.70 mol) was added, and the mixture stirred an additional 1.5 h. The mixture was washed with aqueous sodium carbonate (225 g, 1500 mL water), water (2x500 mL), then dried ($MgSO_4$). Filtration and concentration left the crude product as a yellow gum. Purification by chromatography (0-20% methanol/DCM) provided a light yellow foam (61.8 g, 98%). MS: 453 (M+H). $^1$H-NMR ($CDCl_3$) δ 1.64-2.09 (m, 7H, CH); 2.27-2.35 (m, 2H, CH); 2.46 (s, 3H, $NCH_3$); 2.68 (s, 3H, $SOCH_3$); 2.74-3.05 (m, 7H, CH); 3.39-3.78 (bs, 1H, NH); 7.07-7.10 (m, 1H, aromatic); 7.23-7.50 (m, 5H, aromatic); 7.95-7.99 (m, 1H, aromatic).

## Example 2

**N-[(S)-2-(3,4-Dichlorophenyl)-4-[4-[(S)-2-methylsulfinylphenyl]-1-piperidinyl]butyl]-N-methyl-2,3-dimethoxy-1-naphthamide citrate.**

Using standard acylation conditions N-[(S)-2-(3,4-dichlorophenyl)-4-[4-[(S)-2-methylsulfinylphenyl]-1-piperidinyl]butyl]-N-methylamine was reacted with 2,3-dimethoxy-1-naphthoyl chloride and converted to the citrate salt. MS m/z 667 (M+H); analysis for $C_{36}H_{40}Cl_2N_2O_4S$•1.0 $C_6H_8O_7$•1.2 $H_2O$; calculated: C, 57.23; H, 5.76; N, 3.14; found: C, 57.22; H, 5.76; N, 3.18.

The requisite carboxylic acid was prepared as follows.

(a) 2,3-Dihydroxy-1-naphthaldehyde.

A stream of HCl gas was passed through a mixture of 2,3-dihydroxynaphthalene (6.0 g, 37.4 mmol) and zinc cyanide (6.6 g, 56.1 mmol) in dry ether (28 mL) at 0 °C for 20 min, producing an insoluble yellow oil. Stirring was continued for 1 h at 0 °C, then at room temperature for 1 h. The yellow brown oil was separated and washed with ether. Water (120 mL) was added and the mixture was heated at 60 °C for 10 min. The yellow solid generated was filtered and washed with water to afford the titled product (5.48 g, containing 20% starting material). MS m/z 187 (M-H).

(b) 2,3-Dimethoxy-1-naphthaldehyde.

A mixture of 2,3-dihydroxyl-1-naphthaldehyde (4.87 g, 25.8 mmol), potassium carbonate (14.2 g, 102.9 mmol), and $CH_3I$ (16 mL, 258 mmol) in acetone (80 mL) was heated at 59 °C for 29 h. The solvent was evaporated and the mixture diluted with EtOAc. The organic layer was dried ($MgSO_4$), filtered, and concentrated. Following chromatography purification the product was recovered as white solid (3.7 g, 66%). $^1$H NMR ($CDCl_3$) δ 10.82 (s, 1H), 9.10 (d, 1H), 7.71 (d, 1H), 7.51 (m, 2H), 7.43 (s, 1H), 4.06 (s, 3H), 4.03 (s, 3H). MS m/z 217 (M+H).

(c) 2,3-Dimethoxy-1-naphthoic acid.

To a solution of 2,3-dimethoxy-1-naphthaldehyde (3.7 g, 17.1 mmol) in acetone was added sodium carbonate (1.81 g, 17.1 mmol) in water (9 mL). Potassium permanganate (2.7 g, 17.1 mmol) was added in portions. The solution was stirred at room temperature for 3 h and filtered. The filtrate was concentrated and extracted with EtOAc. The aqueous layer was acidified to pH 1 by adding 1N HCl, then extracted with EtOAc. The combined organic layers were

dried ($MgSO_4$), filtered, and concentrated to afford the product as yellow solid (2.41 g, 61%). $^1H$ NMR (DMSO) δ 13.46 (s, 1H), 7.86 (d, 1H), 7.63 (d, 1H), 7.51 (s, 1H), 7.42 (m, 2H), 3.96 (s, 3H), 3.83 (s, 3H). MS m/z 231 (M-H).

**Reference Example 3**

**N-[2-(S)-(3,4-Dichlorophenyl)-4-[4-[(S)-2-methylsulfinylphenyl]-1-piperidinyl]butyl]-N-methyl-2-ethoxy-1-naphthamide citrate hydrate.**

To a stirred solution of 2-ethoxy-1-naphthoic acid (106.5 mg, 0.492 mmol) in dry DCM (2 mL) was added oxalyl chloride (75.7 mg, 0.596 mmol) and DMF (5 µL). After 3 h at room temperature DCM was removed in vacuo to afford 2-ethoxy-1-naphthoyl chloride. Using standard acylation conditions (except that the order of addition was reversed) 2-ethoxy-1-naphthoyl chloride was reacted with N-[(S)-2-(3,4-dichlorophenyl)-4-[4-[(S)-2-methylsulfinylphenyl]-1-piperidinyl]butyl]-N-methylamine (203 mg, 0.448 mmol), converted to the citrate salt and isolated by filtration from $Et_2O$ to afford the title compound (301.3 mg) as a white powder. MS m/z 651 (M+H); analysis for $C_{36}H_{40}Cl_2N_2O_3S$ •$C_6H_8O_7$ •$H_2O$: calculated: C, 58.53; H, 5.85; N, 3.25; found: C, 58.70; H, 5.65; N, 3.17.

**Reference Example 4**

**N-[(S)-2-(3,4-Dichlorophenyl)-4-[4-[(S)-2-methylsulfinylphenyl]-1-piperidinyl]butyl]-N-methyl-2-methoxy-1-naphthamide citrate.**

According to the procedure described in Example 1, 2-methoxy-1-naphthoic acid (0.071 g) was converted to the acid chloride; 2-methoxy-1-naphthoyl chloride. Using standard acylation conditions N-[(S)-2-(3,4-dichlorophenyl)-4-[4-[(S)-2-methylsulfinylphenyl]-1-piperidinyl]butyl]-N-methylamine (0.238 g) was reacted with 2-methoxy-1-naphthoyl chloride. The free base (0.149 g) was converted to the citrate salt. MS m/z: 637 (M+H).

**Reference Example 5**

**N-[(S)-2-(3,4-Dichlorophenyl)-4-[4-[(S)-2-methylsulfinylphenyl]-1-piperidinyl]butyl]-N-methyl-2-methyl-1-naphthamide citrate.**

According to the procedure described in Example 1, 2-methyl-1-naphthoic acid (0.100 g) was converted to the acid chloride and reacted with N-[(S)-2-(3,4-dichlorophenyl)-4-[4-[(S)-2-methylsulfinylphenyl]-1-piperidinyl]butyl] -N-methylamine (0.2435 g) using standard acylation conditions. The free base (0.255 g) was converted to the citrate salt. MS m/z 621 (M+H).

**Reference Example 6**

**N-[(S)-2-(3,4-Dichlorophenyl)-4-[4-[(S)-2-methylsulfinylphenyl]-1-piperidinyl]butyl]-N-methyl-2-chloro-1-naphthamide citrate.**

Under standard conditions, 2-chloro-1-naphthoyl chloride was prepared from 2-chloro-1-naphthoic acid (Chatterjea, JN; et al; J. Indian Chem. Soc., 35, 41, (1958)) using oxalyl chloride. This material (0.10 g) was reacted with N-[(S)-2-(3,4-dichlorophenyl)-4-[4-[(S)-2-methylsulfinylphenyl]-1-piperidinyl]butyl]-N-methylamine (0.219 g) using standard acylation conditions. The product free base (0.174 g) was converted to the citrate salt. MS m/z 641 (M+H).

**Example 7**

**N-[(S)-2-(3,4-Dichlorophenyl)-4-[4-[4-methoxy-(S)-2-methylsulfinylphenyl]-1-piperidinyl]butyl]-N-methyl-3-cyano-2-methoxy-1-naphthamide citrate.**

Using standard reductive amination conditions N-[2-(S)-(3,4-dichlorophenyl)]-4-oxobutyl-N-methyl-3-cyano-2-methoxy-1-naphthamide (0.939 g) was reacted with 4-[4-methoxy-(S)-2-methylsulfinylphenyl]-piperidine (0.525 g) and converted to the citrate salt. MS: m/z 692 (M+); $^1H$ NMR (DMSO-$d_6$) δ 8.75-8.60 (m), 8.20-6.70 (m), 6.31 (d), 4.54 (t), 4.10-3.65 (m), 3.60-3.00 (m), 2.90-2.30 (m), 2.20-1.60 (m); analysis for $C_{37}H_{39}Cl_2N_3O_4S$•1.0 citric acid•1.5 $H_2O$•0.50 $Et_2O$: calculated; C, 56.96; H, 5.84; N, 4.43; found; C, 56.78; H, 5.52; N, 4.39.

The requisite [4-methoxy-(S)-2-methylsulfinylphenyl]-piperidine was prepared as follows.

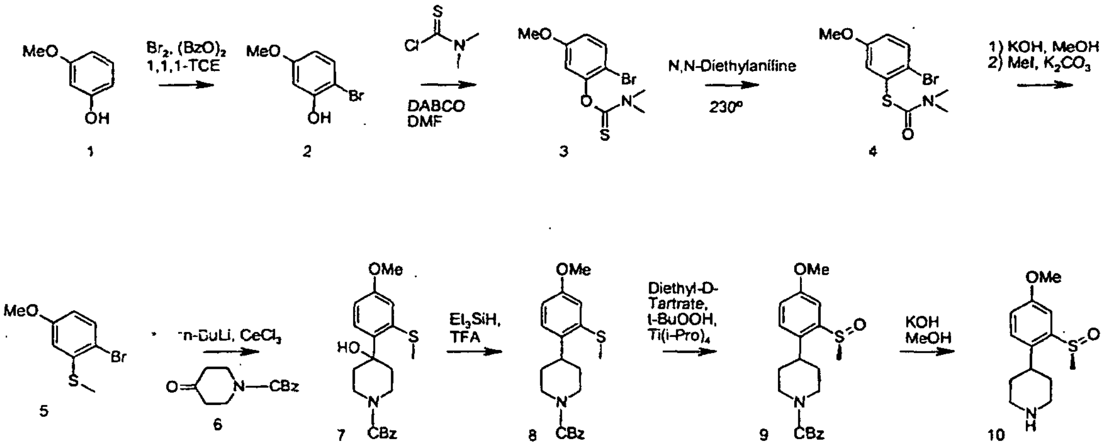


1 2 3 4 5 6 7 8 9 10

(a) 2-Bromo-5-methoxyphenol (2).

To a solution of 3-methoxyphenol (129.03 g) and benzoyl peroxide (1.00 g) in 500 mL 1,1,1-trichloroethane (TCE) was slowly added a solution of bromine (167.90 g in 150 mL in 1,1,1-TCE) over 1 h. During the addition the reaction flask was irradiated with a GE sunlamp

To a solution of 3-methoxyphenol (129.03 g) and benzoyl peroxide (1.00 g) in 500 mL 1,1,1-trichloroethane (TCE) was slowly added a solution of bromine (167.90 g in 150 mL in 1,1,1-TCE) over 1 h. During the addition the reaction flask was irradiated with a GE sunlamp (275 watt, 120 volt) which caused a gentle reflux to occur. The HBr released was trapped in a beaker containing a solution of 126.02 g $NaHCO_3$ and 800 mL water. When the addition of bromine was complete the reaction mixture was purged with nitrogen for 20 minutes. The reaction mixture was extracted with saturated $NaHCO_3$ until the pH of the aqueous extract was neutral. The organic layer was dried over anhydrous $Na_2SO_4$, filtered, and concentrated under reduced pressure to give a reddish oil. The crude product was purified by vacuum distillation (150 °C, 150 millitor) to give 161.78 g of a viscous liquid; $^1$H NMR ($CDCl_3$) δ 3.77 (s, 3), 5.57 (s, 1), 6.42 (m, 1), 6.60 (d, 1), 7.30 (d, 1).

(b) 2-Bromo-5-methoxy-(N,N-dimethylthiocarbamoyloxy)phenol (3).

To a stirred solution of 2-bromo-5-methoxyphenol (161.78 g) and 1,4-diazabicyclo-[2.2.2]octane (180.03 g) in 1 L DMF was slowly added 200 g of dimethylthiocarbamoyl chloride in four separate 50 g portions over 30 min. When the addition was complete the mixture was stirred overnight (18 h) under nitrogen atmosphere. At the end of this period the mixture was poured into 4 L distilled water with rapid stirring. The precipitated product was collected by filtration and washed with water. The crude product was air-dried for 4 h and crystallized from methanol to give white crystals (139.19 g); $^1$H NMR (300 MHz, $CDCl_3$) δ 3.38 (s, 3), 3.47 (s, 3), 3.79 (s,3 ), 6.71 (m, 2), 7.45 (m, 1); MS: m/z 290 (M+H).

(c) 4-Bromo-3-(N,N-dimethylcarbamoylthio)methoxybenzene (4).

A solution of 2-bromo-5-methoxy-(N,N-dimethylthiocarbamoyloxy)phenol (139.19 g) and N,N-diethyaniline (350 mL) was degassed (4 cycles) and then heated under reflux under nitrogen for 3.5 h. The resulting brown solution was concentrated (short path distillation) to approximately 100 mL and the residue was poured into 500 mL of ice cold 6N HCl with rapid stirring. The mixture was cooled to room temperature, 100 mL $Et_2O$ was added, and a heavy precipitate formed which was collected by filtration. This tan precipitate (crude product) was briefly air-dried and set aside. The filtrate was extracted with $Et_2O$. $Et_2O$ extracts were combined, dried over $MgSO_4$, filtered, and concentrated under reduced pressure to give a brownish solid (additional crude product). The crude product was purified by crystallization from methanol to give off-white crystals (82.04 g). 1H NMR (300 MHz, $CDCl_3$) δ 3.05 (br s, 3), 3.12 (br s, 3), 3.79 (s, 3), 6.82 (dd, 1), 7.19 (d, 1), 7.55 (d, 1); MS: 290 (M+H).

(d) 4-Bromo-3-(methylthio)-methoxybenzene (5).

To a stirred solution of KOH (120.01 g) in 500 mL methanol was added 82.04 g of 4-bromo-3-(N,N-dimethylcarbamoylthio)methoxybenzene. The mixture was heated under reflux under nitrogen atmosphere for 2 h, then cooled

to 0 °C and neutralized with 400 mL of 6N HCl. The mixture was cooled to 0 °C and extracted with DCM. The organic extracts were combined, dried over $Na_2SO_4$, filtered, and concentrated under reduced pressure to give a light brown liquid. This liquid was dissolved in 600 mL anhydrous DMF and treated with 80.90 g anhydrous $K_2CO_3$. The resulting mixture was stirred for 20 min and then 68.40 g of methyl iodide was slowly added over 15 min. The resulting mixture was stirred at room temperature under nitrogen atmosphere overnight (18 h). At the end of this period the reaction mixture was poured into 2.8 L distilled water and extracted with $Et_2O$. The organic layers were combined, dried over $MgSO_4$, filtered, and concentrated under reduced pressure to give a pale yellow liquid (65.03g). $^1H$ NMR ($CDCl_3$) δ 2.45 (s, 3), 3.80 (s, 3), 6.55 (dd, 1), 6.66 (d, 1), 7.39 (d, 1).

(e) 4-Hydroxy-4-(4-methoxy-2-methylthiophenyl)-N-Cbz-piperidine (7).

Cerium (III) chloride heptahydrate (181.38 g) was heated under high vacuum at 100 °C for 2 days then at 140 °C for two days. This material was transferred to a dry flask equipped with mechanical stirrer, suspended in 700 mL anhydrous THF, and stirred while cooling to -78° C. A solution of 4-bromo-2-(methylthio)methoxybenzene in 500 mL anhydrous THF was cooled to -78 °C and treated dropwise with n-butylithium (111.5 mL of a 2.5 M solution in hexane) over 1 hour. The temperature of the reaction flask was kept below -70 °C during the addition. This mixture was stirred at -78 °C for 1.5 hours and transferred via wide bore insulated cannula into the flask containing the stirred suspension of $CeCl_3$ at -78 °C. The resulting peach colored suspension was stirred for 1.5 h at -78 °C and then a solution of 1-benzyloxycarbonyl-4-piperidone (65.10g in 200 mL anhydrous THF) was added via cannula over 30 minutes. When the addition was complete the reaction mixture was warmed to room temperature and stirred overnight (18 h). At the end of this period the reaction mixture was quenched with 500 mL saturated $NH_4Cl$ and stirred for 30 minutes. The organic layer was decanted, concentrated under reduced pressure, and set aside. The remaining grayish suspension was stirred with 1 L DCM and filtered through Celite. The Celite filter cake was washed with DCM. All organic extracts were combined, dried over $Na_2SO_4$, filtered, and concentrated under reduced pressure to give a viscous oil which was purified by chromatography on silica (1:1, EtOAc:hexane) to give 85.00 g of an oil. $^1H$ NMR ($CDCl_3$) δ 1.99 (m,2), 2.12 (m, 2), 2.52 (s, 3), 3.39 (m, 2), 3.81 (s, 3), 4.10 (m, 3), 5.15 (s, 2), 6.71 (dd, 1), 6.95 (d, 1), 7.24 (d, 1), 7.37 (m, 5); MS: 387 (M+H).

(f) 4-(4-Methoxy-2-methylthiophenyl)-N-Cbz-piperidine (8).

To an ice-cooled, rapidly stirred slurry of 4-hydroxy-4-(4-methoxy-2-(methylthio)-phenyl)-N-Cbz-piperidine (50.09 g) in triethylsilane (29.12 g) was slowly added trifluoroacetic acid (29.60 g). When addition was complete the mixture was warmed to room temperature and stirred overnight (18 h). At the end of this period the mixture was poured into 300 mL saturated $NaHCO_3$ and extracted with DCM. Extracts were combined, dried over $Na_2SO_4$, filtered, and concentrated under reduced pressure to give an oil. The product was purified by chromatography on silica (40:1 to 20:1 gradient, DCM:EtOAc) to give 42.50 g of an oil. $^1H$ NMR ($CDCl_3$) δ 1.57 (m,2), 1.83 (d,2), 2.46 (s,3), 2.91 (m,2), 3.06 (tt,1), 3.80 (s,3), 4.33 (m,2), 5.14 (s,2), 6.68 (dd,1), 6.76 (d,1), 7.04 (d,1), 7.36 (m,5); MS: m/z 394 (M+Na).

(g) 4-(4-Methoxy-2-(S)-methylsulfinylphenyl)-N-Cbz-piperidine (9).

To a 500 mL flask was added 11.56 g diethyl-D-tartrate, 140 mL anhydrous DCM, 7.96 g titanium (IV) isopropoxide, and 0.50 g water. The pale yellow solution was stirred for 30 minutes and then treated with 10.78 g of 4-(4-methoxy-2-methylthiophenyl)-N-Cbz-piperidine dissolved in 40 mL DCM. The reaction mixture was then immersed in a water/glycol bath pre-chilled to -30 °C. After stirring for 30 min (bath temperature -36 °C) 5.6 mL of a 6M solution of *tert*-butylhydroperoxide in nonane was slowly added to the reaction mixture and stirred for 6 days under nitrogen atmosphere (bath temperature -38 °C). At the end of this period the reaction was quenched with 50 mL water and stirred vigorously for 1 hour while warming to room temperature. The mixture was then treated with 100 mL of 2.5 M NaOH and stirred for an additional 20 min, filtered through Celite and the layers were separated. The filter cake was washed with DCM twice and each portion was used to extract the aqueous layer. The organic extracts were combined, dried over $Na_2SO_4$, filtered, and concentrated under reduced pressure. This gave a light yellow oil which was purified by chromatography on silica (4:1, EtOAc:DCM). $^1H$ NMR ($CDCl_3$) δ 1.62 (m, 2), 1.79 (m, 2), 2.86 (m, 3), 2.87 (s, 3), 4.34 (m, 2), 5.16 (s, 2), 7.00 (dd, 1), 7.18 (d, 1), 7.36 (m, 5), 7.52 (d, 1); MS: m/z 410 (m+Na).

(h) 4-(4-Methoxy-2-(S)-methylsulfinylphenyl)piperidine (10).

To a solution of KOH (1.50 g) in 20 mL of 1:1 EtOH:water was added 1.23 g of 4-(4-methoxy-2-(S)-methylsulfinylphenyl)-N-Cbz-piperidine. The resulting mixture was heated reflux under nitrogen atmosphere for 18 h, evaporated, dissolved in 10 mL water, and extracted with $CHCl_3$. The organic extracts were combined, dried over $Na_2SO_4$,

filtered, and concentrated under reduced pressure. The resulting residue was purified by chromatography on silica (19:1, DCM:MeOH with 0.5% aqueous $NH_4OH$) to give 0.38 g of white solid. $^1H$ NMR ($CDCl_3$) δ 1.69 (m,2), 1.82 (m, 2), 2.38 (m,1), 2.70 (s,3), 2.75 (m,2), 3.22 (m,2), 3.88 (s,3), 7.01 (dd,1), 7.28 (d,1), 7.51 (d,1); MS: 254 (M+H).

(i) N-[2-(S)-(3,4-Dichlorophenyl)-4-oxobutyl]-N-methyl-3-cyano-2-methoxy-1-naphthamide.

N-[(S)-2-(3,4-Dichlorophenyl)-4-hydroxybutyl]amine (Miller, SC; WO 9512577) was reacted with 3-cyano-2-methoxy-1-naphthoic acid using Schotten Baumann conditions to afford N-[2-(S)-(3,4-dichlorophenyl)-4-hydroxybutyl]-N-methyl-3-cyano-2-methoxy-1-naphthamide [$^1H$ NMR (300 MHz, DMSO-$d_6$) δ 9.70-9.64 (m), 8.67-8.57 (m), 8.07-7.97 (m), 7.80 (s), 7.72-7.55 (m), 7.52-7.48 (m), 7.40-7.33 (m), 7.12-7.10 (d), 7.04-7.02 (d), 6.87-6.83 (m), 6.37-6.29 (d), 4.53-4.44 (t), 4.11-4.00 (m), 3.94 (s), 3.92 (s), 3.91-3.73 (m), 3.71 (s), 3.45-3.38 (m), 3.33 (s), 3.14 (s), 2.97-2.95 (d), 2.63 (s), 2.60 (s); MS APCI, m/z = 455 ($M^+$)]. The alcohol was oxidized using oxalyl chloride and DMSO under standard Swem conditions to afford the aldehyde N-[2-(S)-(3,4-dichlorophenyl)-4-oxobutyl]-N-methyl-3-cyano-2-methoxy-1-naphthamide [$^1H$ NMR (300 MHz, DMSO-$d_6$) δ 9.70-9.64 (m), 8.67-8.57 (m), 8.07-7.97 (m), 7.80 (s), 7.72-7.55 (m), 7.52-7.48 (m), 7.40-7.33 (m), 7.12-7.10 (d), 7.04-7.02 (d), 6.87-6.83 (m), 6.37-6.29 (d), 4.53-4.44 (t), 4.11-4.00 (m), 3.94 (s), 3.92 (s), 3.91-3.73 (m), 3.71 (s), 3.45-3.38 (m), 3.33 (s), 3.14 (s), 2.97-2.95 (d), 2.63 (s), 2.60 (s); MS APCI, m/z = 455 ($M^+$)].

## Example 8

**N-[(S)-2-(3,4-Dichlorophenyl)-4-[4-[(S)-2-methylsulfinylphenyl]-1-piperidinyl]butyl]-N-methyl-2-ethoxy-3-cyano-1-naphthamide citrate.**

A solution of 2-ethoxy-3-cyano-1-naphthoic acid (0.106 g), oxalyl chloride (0.067 g) and DMF (approximately 5 μL) was stirred for 1.5 h then concentrated to afford 2-ethoxy-3-cyano-1-naphthoyl chloride as a white residue which was used directly. Using standard acylation conditions 2-ethoxy-3-cyano-1-naphthoyl chloride (0.114 g) was reacted with N-[(S)-2-(3,4-dichlorophenyl)-4-[4-[(S)-2-methylsulfinylphenyl]-1-piperidinyl]butyl]-N-methylamine (0.220 g) to give the free base (0.264 g) which was converted to the citrate salt: $^1H$ NMR (300 MHz, $CDCl_3$) δ 8.70-8.62 (m), 8.08-6.25 (m), 4.64-4.56 (m), 4.23-3.91 (m), 3.17-1.79 (m), 1.37-1.32 (t, -$CH_3$), 1.24-1.17 (t, -$CH_3$); MS APCI, m/z = 676 ($M^+$).

The requisite 2-ethoxy-3-cyano-1-naphthoic acid was prepared as follows.

(a) Ethyl 3-ethoxy-4-iodo-2-naphthoate.

A solution of 3-hydroxy-4-iodo-2-naphthoic acid (2.0 g), diethyl sulfate (2.94 g), powdered potassium carbonate (3.52 g), and dry acetone (150 mL) was heated under reflux for 18 h. The solution was cooled to room temperature, triethylamine (5 mL) was added, and stirring continued for 30 min. The solution was filtered through a pad of Celite and washed with dry acetone (50 mL). The filtrate was concentrated to a yellow oil, diluted with EtOAc, and washed successively with 1N HCl (100 mL), saturated aqueous sodium bicarbonate (100 mL), and brine (100 mL). The organic phase was dried (sodium sulfate), filtered, concentrated, and purified by chromatography (0-10% EtOAc in hexanes) to afford the product as a yellow oil (2.29 g). $^1H$ NMR (300 MHz, DMSO-$d_6$) δ 8.43 (s, 1H), 8.09 (m, 2H), 7.75 (m, 1H), 7.62 (m, 1H), 4.35 (m), 4.04 (q), 1.39 (m); MS APCI, m/z = 393 (M+Na).

(b) 1-Iodo-2-ethoxy-3-cyanonaphthalene.

Based on the procedure of Wood, JL; Khatri, NA; Weinreb, SM; Tetrahedron Lett; 51, 4907 (1979), ethyl 3-ethoxy-4-iodo-2-naphthoate (2.29 g) was suspended in xylenes (100 mL), cooled to 0°C, dimethylaluminum amide solution (approximately 15.4 mmol) was added and the solution heated under reflux for 2.5 h. The solution was then cooled to 0 °C and acidified to pH 2 by addition of IN HCl and extracted with EtOAc (3x100 mL). The combined EtOAc extracts were washed with saturated aqueous sodium bicarbonate (150 mL) and brine (150 mL), dried (sodium sulfate), filtered, concentrated, and purified by chromatography (1:1 EtOAc:DCM, then 10-20% EtOAc in DCM) to afford the product as a white solid (0.778 g). $^1H$ NMR (300 MHz, DMSO-$d_6$) δ 8.68 (s, 1H), 8.25 (d, 1H), 8.13 (d, 1H), 7.86 (dd, 1H), 7.70 (dd, 1H), 4.21 (q, 2H), 1.50 (t, 3H).

(c) Methyl 2-ethoxy-3-cyano-1-naphthoate.

Through a suspension of 1-iodo-2-ethoxy-3-cyanonaphthalene (0.650 g), $Pd(OAc)_2$ (0.045 g), triethylamine (0.305 g) and methanol (30 mL) was bubbled carbon monoxide for 25 min, then stirred at 70 °C under carbon monoxide

(1 atm) for 18 h. The cooled solution was filtered, rinsed with methanol (20 mL) and DCM (20 mL), concentrated, preadsorbed onto silica (3 g) and purified by chromatography (0-10% EtOAc in hexanes) to afford the product as a white solid (0.252 g). $^1$H NMR (300 MHz, DMSO-$d_6$) δ 8.78 (s, 1H), 8.11 (d, 1H), 7.77 (m, 2H), 7.66 (m, 1H), 4.23 (q, 2H), 4.01 (s, 3H), 1.37 (t, 3H).

(d) 2-Ethoxy-3-cyano-1-naphthoic acid.

A solution of methyl 2-ethoxy-3-cyano-1-naphthoate (0.252 g) and LiOH (0.024 g), THF (5 mL), water (2 mL) and methanol (2 mL) was stirred overnight at room temperature. The solution was diluted with saturated sodium bicarbonate and extracted with $Et_2O$. The aqueous layer was acidified to pH 2 by addition of IN HCl and extracted with $Et_2O$. The organic layer was washed with water (30 mL) and brine (40 mL), dried (sodium sulfate), filtered, and concentrated to a white solid (0.141 g). $^1$H NMR (300 MHz, DMSO-$d_6$) δ 14.00 (b, 1H), 8.72 (s, 1H), 8.09 (d, 1H), 7.81 (m, 2H), 7.64 (m, 1H), 4.25 (q, 2H), 1.32 (t, 3H).

## Example 9

**N-[(S)-2-(3,4-Dichlorophenyl)-4-[4-[2-methylsulfonylphenyl]-1-piperidinyl]butyl]-N-methyl-2-methoxy-3-cyano-1-naphthamide citrate.**

4-(2-Methylsulfonylphenyl)piperidine (Shenvi, AB; Jacobs, RT; Miller, SC; Ohnmacht, CA; Veale, CA. WO 9516682) was reacted with N-[2-(S)-(3,4-dichlorophenyl)-4-oxobutyl]-N-methyl-3-cyano-2-methoxy-1-naphthamide according to standard reductive amination methodology to give the free base which was converted to the title compound. $^1$H NMR (300 MHz, DMSO-$d_6$) δ 8.74-8.64 (m), 8.08-7.98 (m), 7.94-7.90 (m), 7.81-7.70 (m), 7.67-7.48 (m), 7.39-7.34 (t), 7.10-7.05 (m), 6.90-6.83 (m), 6.31-6.28 (d), 4.59-4.51 (t), 4.04 (s), 4.01 (s), 3.95 (s), 3.96 (s), 3.89-3.65 (m), 3.27 (s), 3.23-3.08 (m), 2.72-2.57 (m), 2.44-2.07 (m), 1.88-1.61 (m), 0.84-0.81 (m); MS APCI, m/z = 678 ($M^+$).

## Example 10

**N-[(S)-2-(3,4-Dichlorophenyl)-4-[4-[(S)-2-methylsulfinylphenyl]-1-piperidinyl]butyl]-N-ethyl-3-cyano-2-methoxy-1-naphthamide.**

N-[(S)-2-(3,4-Dichlorophenyl)-4-[4-[(S)-2-methylsulfinylphenyl]-1-piperidinyl]butyl]-N-ethylamine was reacted with 3-cyano-2-methoxy-1-naphthoyl chloride according to standard acylation methodology to give the title compound which was converted to the hydrochloride salt; mp 160-180 °C (dec); $^1$H NMR (300 MHz, DMSO-$d_6$) δ 10.7 (br., 1H), 8.67 (m, 1H), 8.05 (m, 1H), 7.8-6.4 (m, 9H), 3.4 (s,3H), 2.6 (s, 3H), 2.0 (m, 6H), 1 (m, 3H); MS APCI, m/z = 676 ($M^+$).

The requisite N-[(S)-2-(3,4-dichlorophenyl)-4-[4-[(S)-2-methylsulfinylphenyl]-1-piperidinyl]butyl]-N-ethylamine was prepared as follows. N-[(S)-2-(3,4-Dichlorophenyl)-4-hydroxybutyl]amine (Miller, SC; WO 9512577) was acetylated with acetyl chloride in DCM under typical conditions. Using lithium aluminum hydride the acetamide was reduced to afford N-[(S)-2-(3,4-dichlorophenyl)-4-hydroxybutyl]-N-ethylamine [$^1$H NMR (300 MHz, $CDCl_3$) δ 7.4 (d, 1H, *J* = 10 Hz), 7.15 (d, 1H, *J* = 5 Hz), 7.0 (d,d, 1H, *J* = 10,5 Hz), 3.7 (m, 1H), 3.6 (m, 1H), 2.9-2.6 (m, 6H), 1.95 (m, 3H), 1.5 (m, 3H); MS APCI, m/z = 262 ($M^+$)]. Using di(*tert*-butyl)dicarbonate, the amine was converted to the N-Boc protected adduct [$^1$H NMR (300 MHz, $CDCl_3$) δ 7.4 (d, 1H, *J* = 10 Hz), 7.15-7.0 (m, 2H), 1.4 (s, 9H), 1.0 (br s, 3H); MS APCI, m/z = 262 ($M-C_5H_9O_2$)], the alcohol was oxidized using oxalyl chloride and DMSO under standard Swem conditions to afford the aldehyde [$^1$H NMR (300 MHz, $CDCl_3$) δ 9.7 (s, 1H), 7.4 (d, 1H, *J* = 10 Hz), 7.2-7.0 (m, 2H), 3.6-2.9 (m, 6H), 1.0 (br., 3H); MS APCI, m/z = 242 ($M- C_5H_9O_2$)]. This aldehyde was reacted with 4-[(S)-2-methylsulfinylphenyl]-piperidine (Shenvi, AB; Jacobs, RT; Miller, SC; Ohnmacht, CJ, Jr.; Veale, CA. WO 9516682) under standard reductive alkylation conditions to afford N-[(S)-2-(3,4-dichlorophenyl)-4-[4-[(S)-2-methylsulfinylphenyl]-1-piperidinyl]butyl]-N-ethyl-N-*tert*-butylcarbamate [$^1$H NMR (300 MHz, $CDCl_3$) δ 8.0 (m, 1H), 7.5-7.3 (m, 5H), 7.0 (m, 1H), 3.0 (m, 7H), 2.7 (s, 3H), 2.2 (m, 2H), 2.0-1.6 (m, 10H), 1.4 (s, 9H), 1.0 (m, 3H); MS APCI, m/z = 597 ($M^+$)]. This material was converted to the desired amine by removal of the Boc protecting group using trifluoroacetic acid under standard conditions to afford N-[(S)-2-(3,4-dichlorophenyl)-4-[4-[(S)-2-methylsulfinylphenyl]-1-piperidinyl]butyl]-N-ethylamine [$^1$H NMR (300 MHz, $CDCl_3$) δ 9.6 (br., 1H), 8.3 (br., 1H), 7.95 (d, 1H, *J* = 10 Hz), 7.5 (d, 1H, *J* = 10 Hz), 7.4 (d, 1H, *J* = 10 Hz), 7.3 (d, 1H, *J*= 5 Hz), 7.05 (d,d, 1H, *J*= 10,5 Hz), 3.85 (m, 1H), 3.4 (m, 3H), 3.2 (m, 3H), 2.9 (s, 3H), 2.2 (m, 4H), 1.4 (t, 3H, *J* = 10 Hz); MS APCI, m/z = 567 ($M^+$)]; this material was used without purification.

## Example 11

**N-[2-(S)-(3,4-Dichlorophenyl)-4-hydroxybutyl]-N-methyl-3-cyano-2-methoxy-1-naphthamide.**

A solution of N-[(S)-2-(3,4-dichlorophenyl)-4-hydroxybutyl]-N-methylamine (Miller, SC; WO 9512577) in DCM was combined with 10% aqueous sodium bicarbonate solution. The mixture was cooled to 0 °C and a solution of 3-cyano-2-methoxy-1-naphthoyl chloride in DCM was added dropwise over 30 min. After stirring overnight at room temperature, the organic phase was concentrated and purified by column chromatography to afford N-[2-(S)-(3,4-dichlorophenyl)-4-hydroxybutyl]-N-methyl-3-cyano-2-methoxy-1-naphthamide. 1H NMR (300 MHz, DMSO-d6) d 9.70-9.64 (m), 8.67-8.57 (m), 8.07-7.97 (m), 7.80 (s), 7.72-7.55 (m), 7.52-7.48 (m), 7.40-7.33 (m), 7.12-7.10 (d), 7.04-7.02 (d), 6.87-6.83 (m), 6.37-6.29 (d), 4.53-4.44 (t), 4.11-4.00 (m), 3.94 (s), 3.92 (s), 3.91-3.73 (m), 3.71 (s), 3.45-3.38 (m), 3.33 (s), 3.14 (s), 2.97-2.95 (d), 2.63 (s), 2.60 (s); MS APCI, m/z = 455 (M+).

## Example 12

**N-[2-(S)-(3,4-Dichlorophenyl)-4-oxobutyl]-N-methyl-3-cyano-2-methoxy-1-naphthamide.**

The product from Example 11 was oxidized using oxalyl chloride and DMSO under standard Swern conditions to afford the title compound. $^1$H NMR (300 MHz, DMSO-$d_6$) δ 9.70-9.64 (m), 8.67-8.57 (m), 8.07-7.97 (m), 7.80 (s), 7.72-7.55 (m), 7.52-7.48 (m), 7.40-7.33 (m), 7.12-7.10 (d), 7.04-7.02 (d), 6.87-6.83 (m), 6.37-6.29 (d), 4.53-4.44 (t), 4.11-4.00 (m), 3.94 (s), 3.92 (s), 3.91-3.73 (m), 3.71 (s), 3.45-3.38 (m), 3.33 (s), 3.14 (s), 2.97-2.95 (d), 2.63 (s), 2.60 (s); MS APCI, m/z = 455 (M+).

## Example 13

**N-[2-(S)-(3,4-Dichlorophenyl)-4,4-(dimethoxy)butyl]-N-methyl-3-cyano-2-methoxy-1-naphthamide.**

The product from Example 12 (300 mg) was reacted with 2,2-dimethoxypropane (0.16 mL) and 4-toluenesulfonic acid (6 mg) in DCM (5 mL) according to the methods of Lorette et al., J. Org. Chem., 1960,25, 521 to give the title compound. $^1$H NMR (300 MHz, DMSO-$d_6$ δ 8.64-8.62 (m), 8.08-7.98 (m), 7.75-7.58 (m), 7.39-7.37 (m), 7.13-7.06 (m), 6.96-6.92 (d), 6.88-6.84 (m), 6.35-6.32 (d), 4.50-4.42 (t), 4.14-4.11 (m), 3.94 (s), 3.92-3.75 (m), 3.69 (s), 3.45-3.39 (m), 3.23 (s), 3.16 (s), 3.05-3.00 (m), 2.93-2.85 (m), 2.60 (s), 2.04-1.92 (m); MS APCI, m/z = 471 (M-$OCH_3$).

## Example 14

**N-[2-(S)-(3,4-Dichlorophenyl)-4-methoxybutyl]-N-methyl-3-cyano-2-methoxy-1-naphthamide.**

The product from Example 11 (130 mg) was reacted with a 60% dispersion of sodium hydride (21 mg) and methyl iodide (0.019 mL) in DMF (2 mL) to give the title compound. $^1$H NMR (300 MHz, DMSO-$d_6$) δ 8.64-8.62 (m), 8.08-7.97 (m), 7.73-7.68 (m), 7.65-7.33 (m), 7.07-7.04 (m), 6.93-6.90 (d), 6.83-6.80 (m), 6.33-6.30 (d), 4.54-4.46 (t), 4.08-4.01 (m), 3.94 (s), 3.79-3.76 (m), 3.68 (s), 3.44-3.23 (m), 3.19 (s), 3.16-2.89 (m), 2.60 (s), 2.02-1.82 (m), 1.36-0.83 (m); MS APCI, m/z = 471 ($M^+$).

## Example 15

**N-[(S)-2-(3,4-Dichlorophenyl)-4-[4-[(S)-2-methylsulfinylphenyl]-1-piperidinyl]butyl]-N-methyl-3-cyano-2-methyl-1-naphthamide citrate.**

Using standard acylation conditions, 3-cyano-2-methyl-1-naphthoyl chloride (0.109 g) (prepared from 3-cyano-2-methyl-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.200 g), converted to the citrate salt to afford the title compound (0.138 g) as a light yellow powder. MS APCI, m/z = 646 (M+H); $^1$H NMR (300 MHz, $CDCl_3$) δ 8.23-8.17 (m), 8.01-7.96 (m), 7.84-7.80 (m), 7.62-7.29 (m), 7.10 (d), 6.96 (d), 6.79 (d), 6.50 (d), 4.60-4.52 (m), 4.19-4.11 (m), 3.85-3.79 (m), 3.56-3.50 (m), 3.34-3.15 (m), 3.04-2.88 (m), 2.74-2.53 (m), 2.32-1.60 (m); analysis calculated for $C_{36}H_{37}Cl_2N_3O_2S$, 1 $C_6H_8O_7$, 1.3 $H_2O$, C 58.51, H 5.56, N 4.87, found C 58.50, H 5.46, N 4.82.

The requisite 3-cyano-2-methyl-1-naphthoic acid was prepared as follows.

(a) Methyl-3-cyano-2-hydroxy-1-naphthoate.

A flame dried 250 mL 3-neck flask was charged with magnesium metal (2.42 g, 99.5 mmol). After cooling to room temperature, diethyl ether (80 mL), benzene (30 mL) and iodine (12.62 g, 49.7 mmol) were added. The reaction mixture was heated under reflux for 2 h and the iodine color dissipated. After cooling to room temperature, this solution was transferred to methyl-3-cyano-2-methoxy-1-naphthoate (10 g, 41.4 mmol) in benzene (30 mL) via syringe. The flask was washed with benzene (15 mL) and a yellow precipitate formed during the addition. The reaction mixture was heated under reflux for another 1 h. IN HCl and EtOAc were added and the aqueous layer was extracted with EtOAc. The combined organic layers were washed with saturated $Na_2S_2O_4$, NaCl, water, dried over $MgSO_4$, filtered and concentrated. The crude product was purified by chromatography (DCM) to afford the product (6.88 g, 73% yield) as a yellow solid. $^1H$ NMR ($CDCl_3$) δ 12.82 (s, 1H), 8.81-8.78 (d, 1H), 8.32 (s, 1H), 7.83-7.82 (d, 1H), 7.70 (t, 1H), 7.50 (t, 1H), 4.16 (s, 3H). MS (APCI, negative ion mode) m/z 225.92 (M-).

(b) Methyl-3-cyano-2-trifluoromethanesulfonyloxy-1-naphthoate.

To a solution of methyl-3-cyano-2-hydroxy-1-naphthoate (6.24 g, 27.5 mmol) in DCM (140 mL) was added triethylamine (4.21 mL, 30.2 mmol) followed by trifluoromethanesulfonic anhydride (5.05 mL, 30.2 mmol) at 0 °C. The reaction mixture was stirred at room temperature for 30 min. Saturated $NaHCO_3$ was added and the aqueous layer was extracted with DCM. The combined organic extracts were dried over $MgSO_4$, filtered and concentrated. The crude product was purified by chromatography (eluting with DCM) to give the product (9.6 g, 97% yield) as a yellow oil. $^1H$ NMR ($CDCl_3$) δ 8.44 (s, 1H), 8.29-8.04 (d, 1H), 7.01-7.98 (d, 1H), 7.84 (m, 2H), 4.10 (s, 3H).

(c) Methyl-3-cyano-2-methyl-1-naphthoate.

A stirred solution of methyl-3-cyano-2-trifluoromethanesulfonyloxy-1-naphthoate (0.28 g, 0.779 mmol), $K_3PO_4$ (0.33 g, 1.55 mmol), methylboronic acid (0.096 g, 1.55 mmol) and (1,1'-bis(diphenylphosphino)ferrocene)-dichloropalladium(II)$CH_2Cl_2$ (64 mg, 0.078 mmol) in THF (8 mL) was heated at 66 °C for 4.5 h. Saturated aqueous $NaHCO_3$ was added and the mixture was extracted with EtOAc (3x). The combined organic layers were dried over $MgSO_4$, filter and concentrated. The crude product was purified by chromatography (eluting with 5%, 8% EtOAc/hexane) to give the product (0.139 g, 78% yield) as a white solid. $^1H$ NMR ($CDCl_3$) δ 8.28 (s, 1H), 7.88 (d, 1H), 7.77 (d, 1H), 7.67 (t, 1H), 7.55 (t, 1H), 4.08 (s, 3H), 2.66 (s, 3H). MS m/z 226 (M+).

(d) 3-Cyano-2-methyl-1-naphthoic acid.

A solution of methyl-3-cyano-2-methyl-1-naphthoate (0.139 g) in THF (7.55 mL) and water (3 mL) was treated with a solution of NaOH (1.3 mL, 1N). Methanol (0.5 mL) was added, and the mixture was stirred under reflux for 27 hours. The mixture was concentrated, treated with additional water and extracted with DCM. The aqueous layer was acidified with IN HCl and extracted with EtOAc. The extracts were dried, filtered, and the solvent removed to afford the product (0.1 g, 77% yield) as a white solid. $^1H$ NMR (300 MHz, DMSO-$d_6$) δ 14.02 (s, 1H), 8.67 (s, 1H), 8.08 (d, 1H), 7.87-7.62 (m, 3H), 2.59 (s, 3H); MS APCI (negative ion mode) m/z 210.

**Example 16**

**N-[(S)-2-(3,4-Dichlorophenyl)-4-[4-[(S)-2-methylsulfinylphenyl]-1-piperidinyl]butyl]-N-methyl-3-bromo-2,4-dimethoxy-1-naphthamide citrate.**

Using standard acylation conditions, 3-bromo-2,4-dimethoxy-1-naphthoyl chloride (0.212 g) (prepared from 3-bromo-2,4-dimethoxy-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.271 g), converted to the citrate salt, and isolated by filtration from diethyl ether to afford the title compound (0.433 g) as a light yellow powder. MS APCI, m/z = 747 (M+H); $^1H$ NMR (300 MHz, $CDCl_3$) δ 8.08-7.97 (m), 7.57-7.20 (m), 7.01-6.93 (m), 6.60-6.56 (m), 4.46-4.38 (m), 4.04-3.57 (m), 3.37-2.94 (m), 2.74-2.60 (m), 2.33-1.61 (m); analysis calculated for $C_{36}H_{39}BrCl_2N_2O_4S$, 1 $C_6H_8O_7$, 0.8$H_2O$, C 52.93, H 5.14, N 2.94, found C 52.96, H 5.01, N 2.93.

The requisite 3-bromo-2,4-dimethoxy-1-naphthoic acid was prepared as follows.

(a) Ethyl-3-bromo-2,4-hydroxy-1-naphthoate.

To a solution of ethyl-2,4-dihydroxy-1-naphthoate (A. Bruggink and A. McKillop Tetrahedron Vol. 31, 2607,

1975) (0.1 g, 0.43 mmol) in acetonitrile (2 mL) was added N-bromosuccinimide (84 mg, 0.47 mmol). The mixture was stirred at room temperature for 30 min. Acetonitrile was evaporated and $CCl_4$ was added. The solution was filtered and concentrated. The crude product was purified by chromatography (eluting with DCM) to give the product (0.13 g, 93% yield) as a white solid. $^1H$ NMR ($CDCl_3$) δ 13.61 (s, 1H), 8.79 (d, 1H), 8.24 (d, 1H), 7.58 (t, 1H), 7.41 (t, 1H), 6.61 (s, 1H), 4.60 (q, 2H), 1.55 (t. 3H). MS APCI (negative mode) m/z 310.84.

(b) Ethyl-3-bromo-2,4-dimethoxy-1-naphthoate.

To a solution of ethyl-3-bromo-2,4-dihydroxy-1-naphthoate (5.8 g, 18.6 mmol) in acetone (93 mL) was added potassium carbonate (6.43 g, 46.6 mmol) and dimethyl sulfate (4.4 mL, 46.6 mmol). The mixture was heated under reflux overnight and solvent was evaporated. Water and EtOAc were added and the organic layer was dried over $MgSO_4$, filtered and concentrated. The crude product was purified by chromatography (eluting with 3-5% EtOAc/hexane) to give the product (6.23 g, 99% yield) as a light yellow oil. $^1H$ NMR ($CDCl_3$) δ 8.13 (d, 1H), 7.83 (d, 1H), 7.62-7.48 (m, 2H), 4.54 (q, 2H), 4.02 (s, 3H), 4.00 (s, 3H), 1.46 (t. 3H).

(c) 3-Bromo-2,4-dimethoxy-1-naphthoic acid.

A solution of ethyl-3-bromo-2,4-dimethoxy-1-naphthoate (0.613 g) in THF (6 mL) and water (4 mL) was treated with LiOH monohydrate (0.16 g). Methanol (0.5 mL) was added, and the mixture was stirred under reflux for 40 h. The mixture was concentrated, treated with additional water and extracted with DCM. The aqueous layer was acidified with 1N HCl and extracted with EtOAc. The extracts were dried, filtered, and the solvent removed to afford the product (0.33 g, 59% yield) as a white solid. $^1H$ NMR (300 MHz, DMSO-$d_6$) δ 13.73 (s, 1H), 8.09 (d, 1H), 7.82 (d, 1H), 7.71-7.56 (m, 2H), 3.97 (s, 3H), 3.91 (s, 3H).

## Example 17

**N-[(S)-2-(3,4-Dichlorophenyl)-4-[4-[(S)-2-methylsulfinylphenyl]-1-piperidinyl]butyl]-N-methyl-3-cyano-2-ethyl-4-methoxy-1-naphthamide citrate.**

Using standard acylation conditions, 3-cyano-2-ethyl-4-methoxy-1-naphthoyl chloride (0.15 g) (prepared from 3-cyano-2-ethyl-4-methoxy-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.23 g), converted to the citrate salt, and isolated by filtration from diethyl ether to afford the title compound (0.117 g) as a white powder. MS APCI, m/z = 690 (M+H); $^1H$ NMR (300 MHz, $CDCl_3$) δ 8.34-8.13 (m), 8.00-7.30 (m), 7.10-6.99 (m), 6.81-6.51 (m), 4.53-4.22 (m), 4.70-2.56 (m), 2.30-1.08 (m); analysis calculated for $C_{38}H_{41}Cl_2N_3O_3S$, 1 $C_6H_8O_7$, 1.6 $H_2O$, C 57.97, H 5.77, N 4.61, found C 57.98, H 5.62, N 4.46.

The requisite 3-cyano-2-ethyl-4-methoxy-1-naphthoic acid was prepared as follows.

(a) Ethyl-3-cyano-2,4-dimethoxy-1-naphthoate.

To a solution of ethyl-3-bromo-2,4-dimethoxy-1-naphthoate (Example 16) (11.2 g, 33.0 mmol) in DMF (88 mL) was added CuCN (3.00 g, 33.5 mmol) and 2 drops of pyridine. The reaction mixture was put in a preheated oil bath (185 °C) and heated for 6 h. The hot solution was poured into concentrated $NH_4OH$ (130 mL) containing 130 g of ice. The resulting suspension was extracted with DCM, washed with 1N HCl, water, dried over $MgSO_4$, filtered and concentrated. The crude product was purified by chromatography (eluting with 3-8% EtOAc/hexane) to give the product (3.81 g, 40% yield) as a white solid. $^1H$ NMR ($CDCl_3$) δ 8.20 (d, 1H), 7.87 (d, 1H), 7.65 (t, 1H), 7.50 (t, 1H), 4.52 (q, 2H), 4.36 (s, 3H), 4.09 (s, 3H), 1.48 (t. 3H).

(b) Ethyl-3-cyano-2-hydroxy-4-methoxy-1-naphthoate.

According to the method described for example 15 step (a), ethyl-3-cyano-2,4-dimethoxy-1-naphthoate (3.71 g, 13.0 mmol) was converted to the title compound (1.89 g, 54% yield) as a yellow solid. $^1H$ NMR ($CDCl_3$) δ 13.37 (s, 1H), 8.79 (d, 1 H), 8.24 (d, 1H), 7.66 (t, 1H), 7.42 (t, 1H), 4.58 (q, 2H), 4.49 (s, 3H), 1.54 (t, 3H); MS m/z 272.02 (M+).

(c) Ethyl-3-cyano-4-methoxy-2-trifluoromethanesulfonyloxy-1-naphthoate.

According to the method described for Example 15 step (b), ethyl-3-cyano-2-hydroxy-4-methoxy-1-naphthoate (1.89 g, 6.97 mmol) was converted to the title compound (2.85 g, quantitative) as a yellow oil. $^1H$ NMR ($CDCl_3$)

δ 8.31 (t, 2H), 7.80 (t, 1H), 7.69 (t, 1H), 4.54 (q, 2H), 4.45 (s, 3H), 1.46 (t, 3H).

(d) Ethyl-3-cyano-4-methoxy-2-vinyl-1-naphthoate.

To a solution of ethyl 3-cyano-4-methoxy-2-trifluoromethanesulfonyloxy-1-naphthoate (1.84 g, 4.5 6mmol) in 21 mL of dry dioxane was added tributylvinyltin (1.47 mL, 5.03 mmol), LiCl (0.58 g, 13.7 mmol), $Pd(PPh_3)_4$ (0.11 g, 0.09 mmol) and a few crystals of 2,6-di-t-butyl-4-methylphenol. The resulting suspension was heated under reflux for 3 h. The mixture was cooled to room temperature and treated with saturated KF solution (30 mL) and stirred for 30 min. After diluting with EtOAc, the mixture was filtered, washed with water and saturated NaCl, dried over $MgSO_4$, filtered and concentrated. Following chromatographic purification (hexane, 3% and 4% EtOAc/hexane), the product was afforded as a white solid (1.09 g, 85% yield). $^1$H NMR ($CDCl_3$) δ 8.24 (d, 2H), 7.86 (d, 1H), 7.63 (m, 2H), 7.01 (dd, 1H), 5.83 (d, 1H), 5.70 (d, 1H), 4.48 (q, 2H), 4.28 (s, 3H), 1.41 (t, 3H). MS m/z 282.04 (M+).

(e) Ethyl-3-cyano-2-ethyl-4-methoxy-1-naphthoate,

To a solution of ethyl-3-cyano-4-methoxy-2-vinyl-1-naphthoate (1.09 g, 3.87 mmol) in 75 mL of ethanol was added 5% Pd/C (0.16 g). The solution was shaken at room temperature for 2 h under hydrogen at 50 psi. The solution was filtered and washed with ethanol and evaporated to give the product as yellow solid (1.09 g, 99% yield). $^1$H NMR ($CDCl_3$) δ 8.20 (d, 1H), 7.74 (d, 1H), 7.66 (t, 1H), 7.55 (t, 1H), 4.53 (q, 2H), 4.27 (s, 3H), 2.96 (q, 2H), 1.46 (t, 3H), 1.35 (t, 3H); MS m/z 284.04 (M+).

(d) 3-Cyano-2-ethyl-4-methoxy-1-naphthoic acid.

A solution of ethyl-3-cyano-2-ethyl-4-methoxy-1-naphthoate (1.09 g) in THF (13 mL) and water (9 mL) was treated with LiOH monohydrate (0.34 g). Methanol (0.5 mL) was added, and the mixture was stirred under reflux for 22 h. The mixture was concentrated, treated with additional water and extracted with DCM. The aqueous layer was acidified with 1N HCl and extracted with EtOAc. The extracts were dried, filtered, and the solvent removed to afford the crude product. The crude product was purified by chromatography (eluting with 1-5% MeOH in DCM with 1% HOAc) to give the product (0.14 g, 14% yield) as a yellow solid. $^1$H NMR (300 MHz, DMSO-$d_6$) δ 13.86 (s, 1H), 8.20 (d, 1H), 7.84 (m, 2H), 7.69 (t, 1H), 4.19 (s, 3H), 2.88 (q, 2H), 1.31 (t, 3H). MS APCI (negative mode) m/z 253.88.

## Example 18

**N-[(S)-2-(3,4-Dichlorophenyl)-4-[4-[(S)-2-methylsulfinylphenyl]-1-piperidinyl]butyl]-N-methyl-3-cyano-2-ethyl-1-naphthamide citrate.**

Using standard acylation conditions, 3-cyano-2-ethyl-1-naphthoyl chloride (1.33 g) (prepared from 3-cyano-2-ethyl-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (2.29 g), and converted to the citrate salt to afford the title compound (3.57 g) as a light yellow powder. MS APCI, m/z= 660.32 (M+H); $^1$H NMR (300 MHz, $CDCl_3$) δ 8.31-8.19 (m), 7.99-7.82 (m), 7.57-7.31 (m), 7.14-7.11 (d), 7.00-6.98 (m), 6.80 (d), 6.53-6.50 (m), 4.60 (t), 4.38 (t), 3.69-3.49 (m), 3.32-2.55 (m), 2.37-1.61 (m), 1.39-1.10 (m); analysis calculated for $C_{37}H_{39}Cl_2N_3O_2S$, 1 $C_6H_8O_7$, 1.35 $H_2O$, C 58.88, H 5.71, N 4.79, found C 58.86, H 5.57, N 4.70.

The requisite 3-cyano-2-ethyl-1-naphthoic acid was prepared as follows.

(a) Methyl-3-cyano-2-ethyl-1-naphthoate.

To a solution of methyl-3-cyano-2-vinyl-1-naphthoate (Example 24) (5.98 g, 25.2 mmol) in 500 mL of ethanol was added 5% Pd on carbon (1.5 g). The solution was shaken at room temperature for 6 h under hydrogen at 50 psi. The solution was filtered and washed with ethanol. The ethanol was evaporated to give the product as a yellow solid (5.39 g, 89% yield). $^1$H NMR (300 MHz, $CDCl_3$) δ 8.28 (s, 1H), 7.89 (d, 1H), 7.76 (d, 1H), 7.67 (t, 1H), 7.55 (t, 1H), 4.07 (s, 3H), 2.96 (q, 2H), 1.37 (t, 3H); MS m/z 239.98 (M+H). Alternately, methyl-3-cyano-2-ethyl-1-naphthoate can be prepared by reaction of methyl-3-cyano-2-trifluoromethanesulfonyloxy-1-naphthoate (Example 15 (b)) according to the procedure described for Example 15 (b) except using triethylborane in place of methylboronic acid.

(b) 3-Cyano-2-ethyl-1-naphthoic acid.

A solution of methyl-3-cyano-2-ethyl-1-naphthoate (4.87 g, 20.4 mmol) in 10 g of trimethylsilyl iodide was

heated at 75 °C overnight. IN HCl was added and the mixture was extracted with EtOAc. The combined organic layers were dried , filtered and concentrated. The crude product was purified by chromatography (5% MeOH/DCM with 1% HOAc) to afford the product (4.35 g, 95% yield) as a yellow solid. $^1$H NMR (300 MHz, DMSO-$d_6$) δ 14.03 (s, 1H), 8.69 (s, 1H), 8.17-8.09 (dd, 1H), 7.89-7.77 (m, 2H), 7.69 (t, 1H), 2.91 (q, 2H), 1.29 (t, 3H); MS (APCI negative ion mode) m/z 223.90.

**Example 19**

**N-[(S)-2-(3,4-Dichlorophenyl)-4-[4-[2-methylsulfonylphenyl]-1-piperidinyl]butyl]-N-methyl-3-cyano-2-ethyl-1-naphthamide citrate.**

Using standard reductive amination conditions, N-[(S)-2-(3,4-dichlorophenyl)-4-oxobutyl]-N-methyl-3-cyano-2-ethyl-1-naphthamide (0.301 g) was reacted with 4-(2-methylsulfonylphenyl)-1-piperidine (0.162 g), and converted to the citrate salt to afford the title compound (0.359 g) as a white powder. MS APCI m/z=577 (M+H); $^1$H NMR (300 MHz, $CDCl_3$) δ 8.25-8.19 (m), 8.06 (m), 7.84-7.82 (m), 7.63-7.34 (m), 7.13 (d), 7.00-6.97 (d), 6.81-6.80 (d), 6.52-6.45 (m), 4.61(t), 4.44-4.35 (m), 3.68-3.64 (m), 3.50-2.55 (m), 2.32-1.74 (m), 1.59 (s), 1.39-1.07 (m); analysis calculated for $C_{37}H_{39}Cl_2N_3O_3S$, 1.0 $C_6H_8O_7$, 0.8 $H_2O$, C 58.47, H 5.55, N 4.76, found C 58.54, H 5.44, N 4.62.

The requisite N-[(S)-2-(3,4-dichlorophenyl)-4-oxobutyl]-N-methyl-3-cyano-2-ethyl-1-naphthamide was prepared as follows.

(a) N-[(S)-2-(3,4-Dichlorophenyl)-4-hydroxybutyl]-N-methyl-3-cyano-2-ethyl-1-naphthamide.

To a stirred, cooled (0 °C) mixture of (S)-2-(3,4-dichlorophenyl)-4-hydroxybutyl-N-methylamine (0.882 g, 3.55 mmol) in DCM (25 mL) and 1N NaOH (4.44 mL) was added dropwise a solution of 3-cyano-2-ethyl-1-naphthoyl chloride (0.866 g, 3.55 mmol) in DCM (10 mL). The mixture was stirred at 0 °C for 2.5 h, $H_2O$ and DCM were added and the mixture was extracted with DCM. The combined organic layers were dried, filtered, and concentrated. Purification by chromatography (0%, 50%, 100% EtOAc in $Et_2O$) provided the title compound (1.25 g, 77%) as a white solid. $^1$H NMR (300 MHz, $CDCl_3$) δ 8.28-8.18 (m), 7.86-7.79 (m), 7.61-7.45 (m), 7.37-7.31 (m), 7.08-7.05 (d), 6.96-6.94 (d), 6.76 (d), 6.50-6.44 (m), 4.64-4.56 (m), 4.42 (m), 3.71-3.03 (m), 2.73-2.69 (m), 2.58-2.55 (d), 2.05-1.63 (m), 1.52-1.16 (m); MS m/z 455.23 (M+H).

(b) N-[(S)-2-(3,4-Dichlorophenyl)-4-oxobutyl]-N-methyl-3-cyano-2-ethyl-1-naphthamide.

To a stirred cooled (-78 °C) solution of oxalyl chloride (0.33 mL, 3.78 mmol) in DCM (10 mL) was added DMSO (0.54 mL, 7.58 mmol) in DCM (5 mL) dropwise. The solution was stirred at -78 °C for 5 min and a solution of N-[(S)-2-(3,4-dichlorophenyl)-4-hydroxybutyl]-N-methyl-3-cyano-2-ethyl-1-naphthamide (1.15 g, 2.52 mmol) in DCM (6.0 mL) and DMSO (2.9 mL) was added dropwise. The solution was stirred at -78 °C for 15 min and triethylamine (2.11 mL, 15.1 mmol) was added. Stirring was continued at -78 °C for 30 min and then at room temperature for 2 h. 1N HCl (75 mL) and DCM (75 mL) were added and the organic layer was dried ($MgSO_4$), filtered, and concentrated. Purification by chromatography (50 % $Et_2O$ in DCM) provided the title compound (1.01 g, 89%) as a white pasty solid. $^1$H NMR (300 MHz, $CDCl_3$) δ 9.78 (s), 9.57 (s), 8.28-8.19 (m), 7.92-7.78 (m), 7.61-7.46 (m), 7.39-7.29 (m), 6.99-6.97 (d), 6.94-6.91 (d), 6.73 (d), 6.51-6.47 (dd), 6.38 (d), 4.68-4.54 (m), 3.82-3.80 (m), 3.61-3.45 (m), 3.34-3.27 (m), 3.08-2.91 (m), 2.71-2.52 (m), 2.06-2.02 (m), 1.35-1.29 (m), 1.08-1.03 (t); MS m/z 453.15 (M+H).

**Example 20**

**N-[2-(S)-(3,4-Dichlorophenyl)-4-[4-[(S)-2-methylsulfinylphenyl]-1-piperidinyl]butyl]-3-cyano-2-methoxy-1-naphthamide citrate.**

3-Cyano-2-methoxy-1-naphthoic acid (155 mg) was reacted with oxalyl chloride under standard conditions to afford the acid chloride and used without purification. The acid chloride was combined with N-[(S)-2-(3,4-dichlorophenyl)-4-[4-[(S)-2-methylsulfinylphenyl]-1-piperidinyl]butyl]-amine (300 mg) and triethyl amine under standard acylation conditions to afford the product as a white powder (300 mg, 67%) after extraction and chromatographic purification (5% methanol in DCM), and converted to the citrate salt under standard conditions. $^1$H NMR (300 MHz, DMSO-d6) d 8.76-8.61 (m), 8.56 (s), 8.02-8.00 (d), 7.84-7.80 (m), 7.69-7.55 (m), 7.53-7.47 (m), 7.45-7.25 (m), 7.19-7.17 (d), 3.89 (s), 3.86-3.75 (m), 3.56-3.50 (m), 3.44-3.27 (m), 3.07-3.04 (m), 2.94-2.90 (m), 2.82-2.79 (m), 2.65 (s), 2.22-2.15 (m), 2.13-1.92 (m), 1.76-1.39 (m); MS APCI, m/z = 648 (M+).

The requisite N-[(S)-2-(3,4-dichlorophenyl)-4-[4-[(S)-2-methylsulfinylphenyl]-1-piperidinyl]butyl]-amine was

prepared as follows.

(a) [2-(S)-(3,4-Dichlorophenyl)-4-[4-[(S)-2-(methylsulfinyl)phenyl]-1-piperidinyl]butyl]-phthalimide.

4-[(S)-2-methylsulfinylphenyl]-piperidine (5.06 g) was reacted with N-[(S)-2-(3,4-dichlorophenyl)-4-oxobutyl]-phthalimide (Bernstein, PR; Miller, SC. EP 709376, 1996) (8.2 g) in the presence of sodium cyanoborohydride under standard reductive amination conditions to afford the product (6.0 g, 46%) after extraction and chromatographic purification (5% methanol in DCM). 1H NMR (300 MHz, DMSO-d6) d 7.85-7.78 (m), 7.57-7.56 (d), 7.53-7.46 (m), 7.41-7.38 (m), 7.24-7.19 (dd), 3.86-3.75 (m), 3.39-3.34 (m), 3.19-3.16 (m), 2.89-2.85 (d), 2.79-2.75 (d), 2.64 (s), 2.62-2.57 (m), 2.19-2.15 (m), 2.10-2.08 (m), 1.90-1.79 (m), 1.69-1.54 (m); MS APCI, m/z = 569 (M+).

(b) N-[(S)-2-(3,4-Dichlorophenyl)-4-[4-[(S)-2-methylsulfinylphenyl]-1-piperidinyl]butyl]-amine.

[2-(S)-(3,4-Dichlorophenyl)-4-[4-[(S)-2-(methylsulfinyl)phenyl]-1-piperidinyl]butyl]-phthalimide (6.13 g) was dissolved in methanol (100 mL), hydrazine (0.8 mL) was added dropwise and the mixture was heated under reflux for 30 min. The reaction mixture was cooled to room temperature and quenched with water, diluted with DCM, extracted with brine, dried (MgSO4), and concentrated to afford the amine as a glassy solid (4.7 g, quantitative) after extraction and chromatographic purification (5-10% methanol, 1% $NH_4OH$, in DCM). 1 H NMR (300 MHz, DMSO-d6) d 7.85-7.81 (m), 7.56-7.40 (m), 7.25-7.19 (dd), 3.39-3.20 (m), 2.93-2.89 (m), 2.82-2.71 (m), 2.65 (s), 2.30-2.07 (m), 1.93-1.87 (m), 1.71-1.57 (m); MS APCI, m/z = 439 (M+).

## Example 21

**N-[2-(S)-(3,4-Dichlorophenyl)-4-[4-(S)-2-methylsulfinylphenyl]-1-piperidinyl]-butyl]-N-methyl-3-cyano-2-(1-propenyl)-1-naphthamide citrate.**

Using standard acylation conditions, 3-cyano-2-(1-propenyl)-1-naphthoyl chloride (0.028 g) (prepared from 3-cyano-2-(1-propenyl)-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.055 g), and converted to the citrate salt to afford title compound as a white powder. MS APCI, m/z = 672 (M+); 1H NMR (300 MHz, CDCl3) d 8.22 (d), 7.93-7.80 (m), 7.51 (m), 6.61-6.47 (m), 3.49 (m), 3.43-3.21 (m), 2.72-2.36 (m), 2.00-1.93 (m); mp 112 °C.

The requisite 3-cyano-2-(1-propenyl)-1-naphthoic acid was prepared as follows.

(a) Methyl-3-cyano-2-allyl-1-napthoate.

To a stirred solution of methyl-3-cyano-2-trifluoromethanesulfonyloxy-1-naphthoate (Example 15) (0.310 g, 0.86 mmol), anhydrous lithium chloride (0.307 g, 7.25 mmol), triphenylphosphine (0.136 g, 0.52 mmol) and dichlorobis (triphenylphosphine)palladium(II) (0.073 g, 0.10 mmol) in DMF (10 mL), was added allyltributyltin (0.535 mL, 1.73 mmol). A crystal of 2,6-di-tert-butyl-4-methylphenol was added and the mixture was heated under nitrogen at 120° C for 45 minutes. Water (15 mL) and diethyl ether (15 mL) were added and the organic phase was washed successively with IN HCl (3 x 50 mL) and saturated aqueous KF (3 x 50 mL), dried ($Na_2SO_4$), filtered, concentrated and purified by column chromatography (15% ether/hexane) to afford product as a colorless oil (0.040 g, 14%). 1H NMR (300 MHz, $CDCl_3$) δ 8.28 (s, 1H), 7.89 (d, 1H), 7.81 (d, 1H), 7.71 (m, 1H), 7.65 (m, 1H), 6.04-5.91 (m, 1H), 5.18 (m, 1H), 4.05 (s, 3H), 3.73 (m, 2H).

(b) 3-Cyano-2-(1-propenyl)-1-naphthoic acid.

A solution of methyl-3-cyano-2-allyl-1-naphthoate (0.040 g, 0.16 mmol) in THF (3 mL) and water (1 mL) was treated with LiOH monohydrate (0.020 g, 0.48 mmol) and MeOH (3 mL) and heated under reflux for 72 hours. The mixture was concentrated, treated with additional water and IN NaOH and extracted with DCM (3 x 25 mL). The aqueous layer was acidified (IN HCl) and the resulting white precipitate was extracted with DCM (3 x 50 mL). The combined organic extracts were dried ($Na_2SO_4$), filtered and concentrated to afford product as a yellow oil (0.026, 70 %). MS APCI, m/z = 236 (M+); $^1H$ NMR (300 MHz, $CDCl_3$) δ 8.68 (s, 1H), 8.16 (d, 1H), 7.81 (m, 2H), 7.70 (m, 1H), 6.69 (d, 1H), 6.44 (m, 1H), 1.95 (m, 3H).

**Example 22**

**N-[(S)-2-(3,4-Dichlorophenyl)-4-[4-[(S)-2-methylsulfinylphenyl]-1-piperidinyl]butyl]-N-methyl-3-bromo-2-methoxy-1-naphthamide citrate.**

Using standard acylation conditions, 3-bromo-2-methoxy-1-naphthoyl chloride (0.149 g, 0.50 mmol) (prepared from 3-bromo-2-methoxy-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.226 g, 0.50 mmol), and converted to the citrate salt to afford the title compound (0.150 g, 42%) as a light powder. $^1$H NMR (300 MHz, $CDCl_3$) δ 8.36 (m), 7.92-7.75 (m), 7.56-7.40 (m), 6.27 (m), 3.82 (s), 3.59-3.10 (m), 2.89-2.50 (m), 1.81 (m); MS APCI, m/z = 717 ($M^+$).

The requisite 3-cyano-2-methoxy-1-naphthoic acid was prepared as follows.

(a) Methyl-2-hydroxy-1-naphthoate

To a solution of 2-hydroxy-1-naphthoic acid (15.40 g, 0.082 mol) in acetone (400 mL) was added $NaHCO_3$ (6.88 g, 0.082 mol) followed by dimethylsulfate (23.23 mL, 0.246 mol) and heated under reflux for 1 h. The mixture was cooled to room temperature and quenched with water (150 mL), extracted with DCM, dried, and concentrated to afford the product (16.05 g, 97 %). $^1$H NMR (300 MHz, DMSO-$d_6$) δ 10.59 (s, 1H), 7.93 (d, 1H), 7.82 (t, 1H), 7.51 (t, 1H), 7.38 (t, 1H), 7.24 (d, 1H), 3.94 (s, 3H); MS APCI, m/z = 201 ($M^-$).

(b) Methyl-2-hydroxy-5,6,7,8-tetrahydro-1-naphthoate.

To a solution of methyl-2-hydroxy-1-naphthoate (16.50 g, 0.082 mol) in acetic acid (225 mL) was added 10% Pd on carbon (1.65 g, 10 wt %) and the mixture was shaken under hydrogen (50 psi) at 60° C for 48 h. The mixture was filtered, dried and concentrated to afford the product (15.99 g, 95%). $^1$H NMR (300 MHz, $CDCl_3$) δ 10.90 (s, 1H), 7.13 (d, 1H), 6.79 (d, 1H), 3.94 (s, 3H), 2.97 (m, 2H), 2.70 (m, 2H), 1.73 (m, 4H); MS (APCI, negative ion mode), m/z = 205 (M-).

(c) Methyl-3-bromo-2-hydroxy-5,6,7,8-tetrahydro-1-naphthoate.

To a solution of methyl-2-hydroxy-5,6,7,8-tetrahydro-1-naphthoate (10.40 g, 0.050 mol) and sodium acetate (7.44 g, 0.090 mol) in acetic acid (180 mL) was added a solution of bromine (10.47 g, 0.066 mol) in acetic acid (72 mL) dropwise. After heating at 80° C for 1 h, the reaction mixture was cooled and concentrated. The residue was diluted with water and extracted with EtOAc, dried, concentrated and purified by chromatography (2% EtOAc in hexane) to afford product as a white solid (11.13 g, 77%). $^1$H NMR (300 MHz, DMSO-$d_6$) δ 9.56 (s, 1H), 7.33 (s, 1H), 3.80 (s, 3H), 2.64 (m, 2H), 2.50 (m, 2H), 1.66 (m, 4H); MS APCI, m/z = 283 ($M^+$).

(d) Methyl-3-bromo-2-methoxy-5,6,7,8-tetrahydro-1-naphthoate.

To a solution of methyl-3-bromo-2-hydroxy-5,6,7,8-tetrahydro-1-naphthoate (11.13 g, 0.039 mol) in acetone (250 mL) was added dimethyl sulfate (4.43 mL, 0.047 mol) and potassium carbonate (6.48 g, 0.047 mol), and the mixture was heated under reflux overnight. The reaction was cooled to room temperature and triethylamine (25 mL) was added. After stirring for 0.5 h, the mixture was filtered, concentrated, diluted with EtOAc (100 mL), washed successively with IN HCl, saturated aqueous $NaHCO_3$, and brine, and extracted with EtOAc. The combined organic extracts were dried, filtered, concentrated and purified by chromatography (3% EtOAc in hexane) to afford the product as a white solid (7.3 g, 63 %). $^1$H NMR (300 MHz, $CDCl_3$) δ 7.31 (s, 1H), 3.92 (s, 3H), 3.85 (s, 3H), 2.71 (m, 2H), 2.62 (m, 2H), 1.75 (m, 4H).

(e) Methyl 3-bromo-2-methoxy-1-naphthoate.

A mixture of methyl 3-bromo-2-methoxy-5,6,7,8-tetrahydronaphthalene-1-carboxylate (1.00 g, 3.34 mmol), N-bromosuccinimide (1.31 g, 7.35 mmol) and 2,2'-azobisisobutyronitrile (50 mg) in 10 mL of carbon tetrachloride was stirred under reflux for 18 h. The succinimide was filtered from the cooled mixture and washed well with carbon tetrachloride, and the filtrate was concentrated to yield a yellow oil consisting primarily of methyl 3,5,8-tribromo-2-methoxy-5,6,7,8-tetrahydronaphthalene-1-carboxylate [$^1$H NMR ($CDCl_3$) δ 2.33 (d,2, J=9Hz), 2.68 (d,2, J=12Hz), 3.89 (s,3), 3.99 (s,3), 5.54 (bs,1), 5.83 (bs,1), 7.62 (s,1)]. The oil was dissolved in 10 mL of xylene and heated under reflux for 11 h during which time HBr was given off. The cooled mixture was concentrated under reduced pressure, the residue

was dissolved in DCM, and purified by chromatography (0-5% EtOAc in hexane) to afford the product (0.48 g, 48%). $^1$H NMR ($CDCl_3$) δ 3.98 (s,3), 4.06 (s,3), 7.46 (t,1, J=9 Hz), 7.53 (t,1, J=9Hz), 7.73 (d,1,J =9 Hz), 8.15 (s,1). MS APCI, m/z = 297 ($M^+$).

(f) 3-Bromo-2-methoxy-1-naphthoic acid

A solution of methyl-3-bromo-2-methoxy-1-naphthoate (0.250 g, 0.85 mmol) in THF (6 mL) and water (2 mL) was treated with LiOH monohydrate (0.079 g, 1.88 mmol) and MeOH (3 mL) and heated under reflux for 48 hours. The mixture was concentrated, diluted with water and 1N NaOH, then extracted with DCM. The aqueous layer was acidified (1N HCl) and the resulting white precipitate was extracted with DCM. The combined organic extracts were dried, filtered and concentrated to afford the product as a yellow oil (0.230 g, 97 %). $^1$H NMR (300 MHz, DMSO-$d_6$) δ 13.8 (s, 1H), 8.44 (s, 1H), 7.98 (d, 1H), 7.76 (d, 1H), 7.64-7.57 (m, 2H), 3.91 (s, 3H); MS APCI (negative ion mode), m/z = 281 (M-).

**Example 23**

**N-[2-(S)-(3,4-Dichlorophenyl)-4-[4-[2-(S)-methylsulfinylphenyl]-1-piperidinyl]butyl]-N-methyl-3-cyano-2-(2,2,2-trifluoroethoxy)-1-naphthamide citrate.**

A solution of 2-(2,2,2-trifluoroethoxy)-3-cyano-1-naphthoic acid was converted to the corresponding acid chloride using oxalyl chloride under standard conditions. This material (0.063 g) was reacted with N-[(S)-2-(3,4-dichlorophenyl)-4-[4-[(S)-2-methylsulfinylphenyl]-1-piperidinyl]butyl]-N-methylamine (0.096 g) using standard acylation conditions and converted to the citrate salt. $^1$H NMR (300 MHz, DMSO-$d_6$) δ 8.78-8.73 (m), 8.14-6.85 (m), 6.20-6.17 (m), 4.84-4.43 (m), 4.072-1.78 (m); MS APCI, m/z = 730 ($M^+$).
The requisite 2-(2,2,2-trifluoroethoxy)-3-cyano-1-naphthoic acid was prepared as follows.

(a) Methyl 2-(2,2,2-trifluoroethoxy)-3-cyano-1-naphthoate.

Methyl 3-cyano-2-hydroxy-1-naphthoate (Example 15, step (a)) (0.050 g) and powdered potassium carbonate (0.060 g) were combined in 4 mL dry acetone. 2,2,2-Trifluoroethyltriflate (0.102 g) was added and the suspension was heated under reflux for 2 h. The suspension was cooled, filtered, and concentrated under reduced pressure to yield a white solid. The solid was dissolved in EtOAc and filtered through a small column of silica gel. The solution obtained was concentrated under reduced pressure to afford the product (0.075 g) as a white solid. $^1$H NMR (300 MHz, $CDCl_3$) δ 8.31 (s), 7.92-7.88 (m), 7.75-7.69 (dd), 7.65-7.60 (dd), 4.65-4.57 (q), 4.07 (s); 19 F NMR (282 MHz, 1H decoupled, $CFCl_3$) δ -72.37 (s).

(b) 2-(2,2,2-Trifluoroethoxy)-3-cyano-1-naphthoate.

Methyl 2-(2,2,2-trifluoroethoxy)-3-cyano-1-naphthoate (0.075 g) and LiOH monohydrate (0.015 g) were dissolved in a solution of 3 mL THF, 1 mL water, and 1 mL MeOH. The solution was heated under reflux for 4 h. The solution was cooled to room temperature and acidified to pH 2 with IN HCl. The acidified solution was made basic (pH 8) with saturated aqueous $NaHCO_3$ and allowed to stir overnight. The solution was transferred to a separatory funnel, 15 mL water was added, and the solution was extracted with 30 mL diethyl ether. The aqueous phase was acidified to pH 2 with 1N HCl. The white suspension was extracted with EtOAc. The EtOAc phase was washed with brine, dried with $Na_2SO_4$, filtered, and concentrated under reduced pressure to yield the product (0.057 g) as a white solid. $^1$H NMR (300 MHz, DMSO-$d_6$) δ 14.30 (b), 8.79 (s), 8.18-8.12 (d), 7.94-7.91 (d), 7.86-7.81 (dd), 7.75-7.69 (dd), 4.90-4.79 (q).

**Example 24**

**N-[(S)-2-(3,4-Dichlorophenyl)-4-[4-[(S)-2-methylsulfinylphenyl]-1-piperidinyl]butyl]-N-methyl-3-cyano-2-vinyl-1-naphthamide citrate.**

Using standard acylation conditions, 3-cyano-2-vinyl-1-naphthoyl chloride (0.086 g) (prepared from 3-cyano-2- vinyl-1-naphthoic acid using oxalyl chloride under standard conditions) was reacted with N-[(S)-2-(3,4-dichlorophenyl)-4-[4-[(S)-2-methylsulfinylphenyl]-1-piperidinyl]butyl]-N-methylamine (0.167 g), and converted to the citrate salt to afford the title compound (0.220 g) as a light powder. $^1$H NMR (300 MHz, DMSO-$d_6$) δ 8.72-8.64 (m), 8.11-5.41 (m), 4.57-4.49 (m), 4.29-4.06 (m), 4.06 (b), 3.69-1.78 (m); MS APCI, m/z = 658 ($M^+$).
The requisite 3-cyano-2-vinyl-1-naphthoic acid was prepared as follows.

(a) Methyl 3-cyano-2-vinyl-1-naphthoate.

To a solution of methyl 3-cyano-2-trifluoromethanesulfonyloxy-1-naphthoate (Example 15) (0.150 g, 0.417 mmol) in 4 mL of dry dioxane was added tributylvinyltin (0.134 mL, 0.459 mmol), LiCl (0.053 g, 1.252 mmol), $Pd(PPh_3)_4$ (0.024 g, 0.020 mmol) and a few crystals of 2,6-di-*t*-butyl-4-methylphenol. The resulting suspension was heated under reflux for 2 h. The mixture was cooled to room temperature and treated with KF solution (1 g KF in 10 mL $H_2O$) and stirred for 30 min. After diluting with EtOAc, the mixture was filtered, washed with water and saturated NaCl, dried over $Na_2SO_4$, filtered and concentrated. Following chromatographic purification (0-10% EtOAc in hexane), the product was afforded as a white solid (0.088 g, 88%). $^1$H NMR (300 MHz, $CDCl_3$) δ 8.31 (s), 7.89-7.84 (d), 7.81-7.78 (d), 7.72-7.66 (dd), 7.64-7.56 (dd), 7.07-6.97 (dd), 5.90-5.84 (d), 5.75-5.71 (d), 4.01 (s).

(b) 3-Cyano-2-vinyl-1-naphthoate.

Methyl 3-cyano-2-vinyl-1-naphthoate (0.087 g, 0.366 mmol) and LiOH monohydrate (0.023 g, 0.550 mmol) were combined in 3 mL THF, 1 mL water, and 1 mL MeOH. The solution was heated under reflux for 6 h. The solution was cooled, 10 mL saturated aqueous $NaHCO_3$ was added. The solution was extracted with 20 mL $Et_2O$ and the aqueous phase was acidified with 1 N HCl to pH 2 and extracted with EtOAc. The organic phase was washed with brine, dried with $Na_2SO_4$, filtered, and concentrated under reduced pressure to afford a white solid. The solid was treated with MeOH and toluene and concentrated under reduced pressure. The solid was again treated with MeOH and toluene and concentrated under reduced pressure to afford the product (0.075 g, 91 % yield) as a white solid. $^1$H NMR (300 MHz, DMSO-$d_6$) δ 14.08 (b), 8.73 (s), 8.14-8.11 (d), 7.89-7.70 (m), 7.06-6.96 (dd), 5.96-5.90 (d), 5.81-5.77 (d).

## Example 25

**N-[(S)-2-(3,4-Dichlorophenyl)-4-[4-[2-thiazol-2-yloxyphenyl]-1-piperidinyl]butyl]-N-methyl-3-cyano-2-methoxy-1-naphthamide citrate.**

4-[2-Thiazol-2-yloxyphenyl]-piperidine trifluoroacetate was reacted with N-[2-(S)-(3,4-dichlorophenyl)-4-oxobutyl]-N-methyl-3-cyano-2-methoxy-1-naphthamide using standard reductive amination conditions and converted to the citrate salt (25% yield). $^1$H NMR (300 MHz, DMSO-$d_6$) δ 8.64 (d), 8.03 (m), 7.78-7.19 (m), 7.08-6.80 (m), 6.31 (d), 4.52 (t), 4.06-3.94 (m), 3.93 (d), 3.92-3.70 (m), 3.54-1.60 (m). MS APCI, m/z = ($M^+$); 699.

The requisite 4-[2-thiazol-2-yloxyphenyl]-piperidine was prepared as follows.

(a) 4-[2-[(5-Bromothiazol-2-yl)oxy]phenyl]-1-N-Cbz-1-piperidine.

2,5-Dibromothiazole (430 mg) was dissolved in DMF under nitrogen and to this was added 4-(2-hydroxyphenyl)-N-Cbz-piperidine (500 mg) and potassium carbonate (670 mg). The reaction was heated at 100 °C overnight and then allowed to cool. It was diluted with EtOAc; washed with water, brine; dried, filtered and concentrated under reduced pressure. The residue was purified via chromatography (15% EtOAc in hexane) to afford the desired product (500 mg) as an oil. $^1$H NMR (300 MHz, $CDCl_3$) δ 7.42-7.13 (m, 10H), 5.15 (s, 2H), 4.31 (bs, 2H), 3.00 (m, 1H), 2.85 (t, 2H), 1.79 (d, 2H), 1.63 (m, 2H). MS APCI, m/z= ($M^+$); 473.

(b) 4-[2-[Thiazol-2-yloxy]phenyl]-piperidine trifluoroacetate.

4-[2-[(5-Bromothiazol-2-yl)oxy]phenyl]-1-N-Cbz-1-piperidine (500 mg) was dissolved in 2-propanol (20 mL) under nitrogen and to this was added 10% palladium on carbon (220 mg). The mixture was shaken under hydrogen, (50 psi) overnight. It was filtered and concentrated under reduced pressure. The residue was then dissolved in TFA (10 mL), heated under reflux for 10 min, cooled and concentrated under reduced pressure. It was partitioned between EtOAc and aqueous sodium bicarbonate, the layers separated and the organic layer dried, filtered and concentrated under reduced pressure to afford the desired compound (370 mg) as a yellow solid. $^1$H NMR (300 MHz, $CDCl_3$) δ 7.46-7.16 (m, 5H), 6.82 (d, 1H), 3.52 (d, 2H), 3.13 (m, 1H), 2.95 (m, 2H), 2.02 (m, 4H). MS APCI, m/z = ($M^+$); 261.

**Example 26**

**N-[(S)-2-(3,4-Dichlorophenyl)-4-[4-[(S)-2-methylsulfinylphenyl]-1-piperidinyl]butyl]-3-cyano-2-methylsulfonyloxy-1-naphthamide citrate.**

4-[(S)-2-Methylsulfinyl-phenyl]-piperidine (0.215 g, 0.900 mmol) was reacted with N-[2-(S)-(3,4-dichlorophenyl)-4-oxobutyl]-3-cyano-2-methylsulfonyloxy-1-naphthamide (0.454 g, 0.900 mmol ) in the presence of sodium cyanoborohydride under standard reductive amination conditions. The crude product was purified by gradient chromatography (eluting with 2-4% MeOH/DCM ) to give product (0.513 g, 80% yield) as a white solid. $^1$H NMR (300 MHz, $CDCl_3$) δ 8.25 (s, 1H), 7.90 (m, 2H), 7.68 (m, 4H), 7.43 (m, 4H), 7.16 (dd, 1H), 3.97 (m, 1H), 3.69 (m, 1H), 3.51 (s, 3H), 3.08 (m, 1H), 2.84 (m, 2H), 2.61 (s, 3H), 2.57 (m, 1H), 2.38 (m, 2H), 2.03-1.70 (m, 4H), 1.68 (m, 1H), 1.31 (m, 3H); MS APCI, m/z = 712 ($M^+$); Analysis calculated for $C_{35}H_{35}N_3O_5S_2Cl_2$, 1.0 $C_6H_8O_7$, 1.0 water, C 53.36, H 4.91, N 4.55, found C 53.31, H 4.86, N 4.49.

(a) N-[2-(S)-(3,4-Dichlorophenyl)-4-hydroxybutyl]-3-cyano-2-methoxy-1-naphthamide.

Using Schotten Baumann conditions, 3-cyano-2-methoxy-1-naphthoyl chloride (3.99 g, 16.29 mmol) was reacted with N-[2-(S)-(3,4-dichlorophenyl)-4-hydroxybutyl]amine and purified by chromatography (0.5-5% MeOH in DCM) to give the product (5.51 g 77% yield) as a white solid. $^1$H NMR (300 MHz, $CDCl_3$) δ 8.16 (s, 1H), 7.82 (d, 1H), 7.65 - 7.32 (m, 5H), 7.14 (dd, 1H), 6.18 (t, 1H), 3.98 (s, 3H), 3.8 - 3.68 (m, 3H), 3.54 (m, 1H), 3.18 (m, 1H), 2.05 (m, 1H), 1.77 (m, 1H); MS APCI, m/z = 443 ($M^+$).

(b) N-[2-(S)-(3,4-Dichlorophenyl)-4-tert-butyldimethylsilyloxybutyl]-3-cyano-2-methoxy-1-naphthamide.

Using typical conditions, N-[2-(S)-(3,4-dichlorophenyl)-4-hydroxybutyl]-3-cyano-2-methoxy-1-naphthamide (5.51 g, 12.46 mmol) was reacted with tert-butyldimethylsilyl chloride (2.81 g, 18.69 mmol) and triethylamine (2.02 g) in DCM, then purified by chromatography (eluting with 50-70% $Et_2O$ in hexane) to give the product (6.48 g, 94% yield) as a white solid. $^1$H NMR (300 MHz, $CDCl_3$) δ 8.2 (s, 1H), 7.82 (d, 1H), 7.62-7.36 (m, 5H), 7.16 (dd, 1H), 6.14 (t, 1H), 4.01 (s, 3H), 3.88-3.78 (m, 2H), 3.64 (m, 1H), 3.47 (m, 1H), 3.20 (m, 1H), 2.03 (m, 1H), 1.84 (m, 1H), 0.86 (s, 9H), 0.016 (s, 6H); MS APCI, m/z = 557 ($M^+$).

(c) N-[2-(S)-(3,4-Dichlorophenyl)-4-tert-butyldimethylsilyloxybutyl]-3-cyano-2-hydroxy-1-naphthamide.

A 250 mL 3-neck flask containing a magnetic stirrer and magnesium chips (0.68 g, 27.96 mmol) was flame dried and allowed to cool to room temperature under nitrogen. After the addition of diethyl ether (30 mL), benzene (15 mL) and iodine (3.55 g, 13.98 mmol), the reaction mixture was heated under reflux for 2 h. After cooling, the solution was transferred via cannula to a flask containing N-[2-(S)-(3,4-dichlorophenyl)-4-tert-butyldimethylsilyloxybutyl]-3-cyano-2-methoxy-1-naphthamide (6.48 g, 11.65 mmol) in 108 mL benzene. Heating under reflux was continued for 1 h, then cooled to room temperature. 1N HCl and DCM were then introduced and allowed to stir for 15 min. The mixture was washed two times with water, dried over $Na_2SO_4$, filtered, and concentrated to give a quantitative yield of a light yellow solid. $^1$H NMR (300 MHz, $CDCl_3$) δ 11.91 (bs, 1H), 8.15 (s, 1H), 7.77 (m, 1H), 7.45-7.13 (m, 6H), 6.28 (m, 1H), 3.96 (m, 1H), 3.62-3.25 (m, 4H), 1.99 (m, 1H), 1.84 (m, 1H), 0.70 (s, 9H), .011 (s, 6H); MS APCI, m/z = 543 ($M^+$).

(d) N-[2-(S)-(3,4-Dichlorophenyl)-4-tert-butyldimethylsilyloxybutyl]-3-cyano-2-methanesuifonyloxy-1-naphthamide.

Using typical conditions, N-[2-(S)-(3,4-dichlorophenyl)-4-tert-butyldimethylsilyloxybutyl]-3-cyano-2-hydroxy-1-naphthamide (2.40 g, 4.42 mmol) was reacted with methanesulfonyl chloride (0.51 g, 4.95 mmol) and triethylamine (1.13 g) in DCM, then purified by chromatography (eluting with 40% hexane/$Et_2O$) to give the product (1.82 g, 67% yield) as a white solid. $^1$H NMR (300 MHz, $CDCl_3$) δ 8.29 (s, 1H), 7.92 (m, 1H), 7.69 (m, 2H), 7.47-7.4 (m, 3H), 7.20 (dd, 1H), 6.27 (t, 1H), 3.88 (m, 1H), 3.80 (m, 1H), 3.63 (m, 1H), 3.54 (s, 3H), 3.47 (m, 1H), 3.20 (m, 1H), 2.0 (m, 1H), 1.83 (m, 1H), 0.87 (s, 9H), 0.028 (s, 6H); MS APCI, m/z = 621 ($M^+$).

(e) N-[2-(S)-(3,4-Dichlorophenyl)-4-hydroxybutyl]-3-cyano-2-methanesulfonyloxy-1-naphthamide.

In a round bottom flask containing a 5% HF in $CH_3CN$ (composed from 44.2 mL 49% aqueous HF and 397.6 mL $CH_3CN$) was added a solution of N-[2-(S)-(3,4-dichlorophenyl)-4-tert-butyldimethylsilyloxybutyl]-3-cyano-2-methanesulfonyloxy-1-naphthamide (2.74 g, 4.42 mmol) in 40 mL $CH_3CN$. After stirring at room temperature for 4 h, the reaction was quenched by the addition of DCM, water, then $NaHCO_3$ was added to adjust to pH 7. The organic layer

was collected, washed twice with water, dried, filtered, and concentrated. The material was purified by chromatography (0.5-2.0% MeOH in DCM) to afford the product (2.14 g, 96% yield ) as a white solid. $^1H$ NMR (300 MHz, $CDCl_3$) δ 8.25 (s, 1H), 7.89 (m, 1H), 7.64 (m, 2H), 7.39 (m, 3H), 7.23 (dd, 1H), 6.37 (s, 1H), 3.82 (t, 2H), 3.65 (m, 1H), 3.51 (s, 3H), 3.46 (m, 1H), 3.15 (m, 1H), 2.04 (m, 1H), 1.86 (m, 1H), 1.64 (m, 1H); MS APCI, m/z = 507 ($M^+$).

(f) N-[2-(S)-(3,4-Dichlorophenyl)-4-oxobutyl]-3-cyano-2-methylsulfonyloxy-1-naphthamide.

N-[2-(S)-(3,4-Dichlorophenyl)-4-hydroxybutyl]-3-cyano-2-methanesulfonyloxy-1-naphthamide was reacted with oxalyl chloride and DMSO under standard Swem oxidizing conditions in DCM. Following aqueous extraction from DCM, and gradient chromatography (1%, 20%, and 50% $Et_2O$ in DCM) gave the product (24% yield ) as a white solid. $^1H$ NMR (300 MHz, $CDCl_3$) δ 8.35 (m, 1H), 7.99 (m, 2H), 7.82-7.69 (m, 3H), 7.42 (m, 1H), 7.24 (m, 1H), 6.19 (m, 1H), 3.83 (m, 2H), 3.59 (s, 3H), 3.44 (m, 1H), 2.34-2.16 (m, 2H); MS APCI, m/z = 505 ($M^+$).

## Example 27

**N-[(S)-2-(3,4-Dichlorophenyl)-4-[4-[2-(methoxy-1,2-dioxoethylamino)phenyl]-1-piperidinyl]butyl]-N-methyl-3-cyano-2-methoxy-1-naphthamide citrate.**

N-[2-(S)-(3,4-dichlorophenyl)]-4-oxobutyl-N-methyl-3-cyano-2-methoxy-1-naphthamide was reacted with 4-(2-(methoxy-1,2-dioxoethylamino)phenyl)piperidine hydrochloride under standard reductive amination conditions. The product was converted to the citrate salt and recovered as a white powder. $^1H$ NMR (300 MHz, DMSO-$d_6$) δ 8.62 (m), 8.04 (m), 7.80-6.83 (m), 6.33 (m), 4.52 (m), 3.95 (s), 3.85 (s), 3.33 (m), 3.20-2.60 (m), 2.65-2.40 (m), 2.30-1.65 (m); MS APCI, m/z = 701 (M+H).

The requisite 4-(2-(methoxy-1,2-dioxoethylamino)phenyl)piperidine hydrochloride was prepared as follows.

(a) 4-(2-Aminophenyl)piperidine.

4-(2-Nitrophenyl)pyridine hydrochloride (prepared by Ullmann coupling of 2-nitrobromobenzene and 4-bromopyridine according the method of Shimizu, N.; Kitamura, T.; Watanabe, K; Yamaguchi, T.; Shigyo, H.; Ohta, T.; Tetrahedron Letters 34 1993, 3421) (6.90 g) was dissolved in acetic acid (100 mL), platinum dioxide (1.60 g) was added and the mixture hydrogenated on a Parr-shaker at 50 psi hydrogen pressure for 4 h. The solution was filtered and evaporated to give the title compound as a yellow oil which was used directly in the next reaction. MS APCI, m/z = 177 (M+H).

(b) tert-Butyl 4-(2-aminophenyl)piperidine-1-carboxylate.

A solution of 4-(2-aminophenyl)piperidine (4.04 g) in water (200 mL) was adjusted to pH 9 by addition of potassium carbonate and cooled in an ice-water bath. A solution of di-tert-butyl dicarbonate (5.20 g) in 1,4-dioxane (80 mL) was added dropwise. The stirred mixture was allowed to warm gradually to room temperature over 3 h. Additional potassium carbonate was added as necessary to maintain pH 9. The reaction was extracted with ethyl ether. The organic extracts were dried and evaporated. The residue was purified by chromatography, with 3:1 hexanes:EtOAc as the eluant, to give the title compound (3.26 g). $^1H$ NMR (300 MHz, DMSO $d_6$) δ 6.87 (m, 2H), 6.65 (d, 1H), 6.52 (m, 1H), 4.91 (s, 2H, NH2), 4.04 (m, 2H), 2.76 (m, 3H), 1.70 (m, 2H), 1.42 (s, 9H), 1.34 (m, 2H); MS APCI, m/z = 177 (M-Boc), 299 (M+Na).

(c) tert-Butyl 4-(2-(methoxy-1,2-dioxoethylamino)phenyl)piperidine-1-carboxylate.

Methyloxalylchloride (0.094g) was added to a solution of tert-butyl 4-(2-aminophenyl)piperidine-1-carboxylate (0.172 g) and triethylamine (0.073 g) in DCM (4 mL) and stirred overnight then diluted with 1N aqueous hydrogen chloride. The organic phase was dried and evaporated to give the title compound (0.207 g) as an oil. MS APCI, m/z = 362 (M+H). $^1H$ NMR ($CDCl_3$) δ 7.83 (m, 1H), 7.21 (m, 3H), 6.30 (br, 1H, NH), 4.24 (m, 2H), 3.72 (s, 3H. OCH3), 2.77 (m, 3H), 1.67 (m, 4H), 1.51 (s, 9H).

(d) 4-(2-(methoxy-1,2-dioxoethylamino)phenyl)piperidine hydrochloride.

Hydrogen chloride gas was gently bubbled through an EtOAc solution (8 mL) containing tert-butyl 4-(2-(methoxy-1,2-dioxoethylamino)phenyl)piperidine-1-carboxylate (0.140 g) at 0 °C for 5 min. The reaction solution was evaporated to give the title compound as a white solid which was used directly in the next reaction.

**Example 28**

**N-[2-(S)-(3,4-Dichlorophenyl)-4-[4-[2-(N,N-dimethylaminocarbonylamino)phenyl]-1-piperidinyl]butyl]-3-cyano-2-methoxy-1-naphthamide citrate.**

N-[2-(S)-(3,4-Dichlorophenyl)]-4-oxobutyl-N-methyl-3-cyano-2-methoxy-1-naphthamide was reacted with 4-[2-(N,N-dimethylaminocarbonylamino)phenyl]-piperidine hydrochloride under standard reductive amination conditions. The product was converted to the citrate salt and recovered as a white powder. $^1H$ NMR (300 MHz, DMSO-$d_6$) d 8.64 (m), 8.02 (m), 7.87-7.19 (m), 6.33 (m), 4.53 (m), 3.95 (s), 3.33 (m), 2.92 (m), 2.65-2.49 (m), 1.85 (m), 1.75 (m); MS APCI, m/z = 686 (M+H).

The requisite 4-[2-(N,N-dimethylaminocarbonylamino)phenyl]-piperidine hydrochloride was prepared as follows.

(a) tert-Butyl 4-[2-(N,N-dimethylaminocarbonylamino)phenyl)]piperidine-1-carboxylate.

Triphosgene (0.305 g) was added to a solution of tert-butyl 4-(2-aminophenyl)-piperidine-1-carboxylate (Example 27) (0.260 g) in DCM (25 mL). Triethylamine (0.115 g) was added rapidly with vigorous stirring. After 15 min 2M dimethylamine in THF (5 mL) was added and the mixture stirred for 1h. The reaction was diluted with 1N HCl and extracted with DCM. The organic extracts were dried and evaporated to give the title compound (0.415 g) as a colorless oil. MS APCI, m/z = 248 (M-Boc); $^1H$ NMR (300 MHz, $CDCl_3$) δ 7.28 (m, 5H), 6.00 (s, 1H), 4.44 (m, 1H), 4.23 (br, 1H), 3.75 (m, 1H), 3.00 (m, 1H), 3.06 (s, 6H), 1.85 (m, 1H), 1.69 (m, 3H), 1.48 (s, 9H).

(b) 4-[2-(N,N-dimethylaminocarbonylamino)phenyl]-piperidine hydrochloride.

Hydrogen chloride gas was gently bubbled through an EtOAc solution (10 mL) containing tert-Butyl 4-[2-(N, N-dimethylaminocarbonylamino)phenyl)]piperidine-1-carboxylate (0.297 g) at 0 °C for 5 min. The reaction solution was evaporated to give the title compound as a white solid (0.240 g). MS APCI, m/z = 246 (M+H).

**Example 29**

**N-[(S)-2-(3,4-Dichlorophenyl)-4-[4-[2-(N-oxo-N,N-dimethylamino)phenyl]-1-piperidinyl]butyl]-N-methyl-3-cyano-2-methoxy-1-naphthamide trifluoroacetate.**

Using standard reductive amination conditions, 4-(2-(N-oxo-N,N-dimethylamino)-phenylpiperidine was reacted with N-[2-(S)-(3,4-dichlorophenyl)]-4-oxobutyl-N-methyl-3-cyano-2-methoxy-1-naphthamide, the product was purified by preparative HPLC and recovered as the trifluroacetate salt. $^1H$ NMR (300 MHz, $CDCl_3$) δ 8.30-8.10 (m), 8.00-7.00 (m), 6.95-6.55 (m), 4.45-4.10 (m), 4.09 (s), 4.02 (s), 3.96 (m), 3.91 (s), 3.72 (m), 3.50-2.75 (m), 2.58 (s), 2.54 (s), 2.50-1.90 (m); MS APCI, m/z = 659 (M+H).

The requisite 4-(2-(N-oxo-N,N-dimethylamino)phenyl)piperidine was prepared as follows.

(a) tert-Butyl 4-[2-N,N-dimethylaminophenyl]piperidinecarboxylate.

Formaldehyde (37% weight in water) was added to a solution of tert-butyl 4-(2-aminophenyl)piperidine-1-carboxylate (Example 27, step (b)) (0.065g) in methanol (2 mL). Acetic acid (0.01 mL) was added, the mixture stirred 5 min then sodium cyanoborohydride (0.100 g) in methanol (2 mL) was added. After 2 h, the solvent was evaporated and the residue partitioned between ethyl ether and aqueous sodium bicarbonate. The organic phase was dried and evaporated. to give the title compound (0.071 g) as a waxy solid. MS: m/z=305 (M+H). $^1H$ NMR ($CDCl_3$) δ 7.26 (m, 4H), 3.34 (m, 1H), 2.84 (m, 2H), 2.62 (s, 6H, N-$CH_3$), 1.62 (m, 6H), 1.54 (s, 9H).

(b) 4-(2-(N-oxo-N,N-Dimethylamino)phenyl)piperidine.

3-Chloroperoxybenzoic acid (0.125 g) in DCM (2 mL) was added to tert-butyl 4-[2-N,N-dimethylaminophenyl] piperidine-1-carboxylate (0.220 g) in DCM (10 mL) and stirred for 1 h. The reaction mixture was extracted sequentially with aqueous sodium sulfite and aqueous sodium bicarbonate. The organic phase was dried and evaporated to give tert-butyl 4-[2-(N-oxo-N,N-dimethylamino)phenyl]piperidine-1-carboxylate (0.206 g) as a foamy white solid. MS: m/z=321 (M+H). This material was N-deprotected with HCl (according to the procedure of Example 27) to afford 4-[2-(N-oxo-N,N-dimethylamino)phenyl]piperidine hydrochloride which was used without purification.

**Example 30**

**N-[2-(S)-(3,4-Dichlorophenyl)-4-[4-[2-methylsulfinyl-4-methoxycarbonylphenyl]-1-piperidinyl]butyl]-N-methyl-2-methoxy-3-cyano-1-naphthamide citrate.**

Using standard reductive amination conditions N-[2-(S)-(3,4-dichlorophenyl)]-4-oxobutyl-N-methyl-2-methoxy-3-cyano-1-naphthamide (0.160 g) was reacted with 4-[2-methylsulfinyl-4-methoxycarbonylphenyl]piperidine (0.093 g) and converted to the citrate salt. MS m/z 720 (M+); $^1$H NMR (DMSO $d_6$) δ 8.77-8.55 (m), 8.20-6.70 (m), 6.34 (d), 4.53 (t), 4.10-3.65 (m), 3.60-3.00 (m), 2.90-2.30 (m), 2.20-1.60 (m).

The requisite 4-[2-methylsulfinyl-4-methoxycarbonylphenyl]piperidine was prepared as follows.

(a) 4-[2-Methylsulfinyl-4-bromophenyl]piperidine.

To a stirred solution of 4-(2-methylsulfinylphenyl)piperidine (Jacobs, R; Shenvi, A; EP 630887) (0.496g) dissolved in 5 mL of acetic acid was added a solution of bromine (0.715 g in 15mL of acetic acid). The mixture was heated at 75 °C for 80 minutes. The cooled mixture was quenched with 3 mL water, the solvent was evaporated, and the residue was dissolved in water. The aqueous mixture was basified to pH 14 by addition of KOH and extracted with $CHCl_3$ (3x15 mL). The organic extracts were combined, dried over $Na_2SO_4$, evaporated, and purified by chromatography (19:1 DCM:methanol containing 0.5% aqueous $NH_4OH$) to afford the product (0.421 g) as a light yellow solid. MS m/z 302 (M+H). $^1$H NMR ($CDCl_3$) δ 7.86 (d, 1H), 7.61 (dd, 1H), 7.52 (d, 1H), 3.25-3.35 (m, 2H), 3.08-2.60 (m, 7H), 2.04-1.61 (m, 4H).

(b) 4-[2-Methylsulfinyl-4-methoxycarbonylphenyl]piperidine.

4-[2-Methylsulfinyl-4-bromophenyl]piperidine was N-protected using di(*tert*-butyl)dicarbonate in dioxane solvent using aqueous NaOH as the base, then isolated by extraction. A solution of the resulting N-Boc-4-[2-methylsulfinyl-4-bromophenyl]piperidine (1.17 g), dissolved in 1:1 methanol:DMSO (50 mL) with triethylamine (0.39 g), palladium acetate (0.092 g), and 1,3-bis(diphenylphosphino)propane (0.184 g), was stirred at 70 °C under carbon monoxide (1 atm) for 16 h. The mixture was cooled, diluted with EtOAc, and the organic layer was washed with water, dried, and purified by chromatography to afford N-Boc-4-[2-methylsulfinyl-4-methoxycarbonylphenyl]piperidine (0.52 g). $^1$H NMR ($CDCl_3$) δ 8.15-8.05 (m, 2H), 7.99-7.95 (m, 1H), 4.40-4.15 (m, 2H), 3.95 (s, 3H), 2.95-2.65 (m, 3H), 2.73 (s, 3H), 1.95-1.45 (m, 4H), 1.50 (s, 9H). N-deprotection of 4-[2-methylsulfinyl-4-methoxycarbonylphenyl]piperidine was accomplished using TFA under standard conditions to give 4-[2-methylsulfinyl-4-methoxycarbonylphenyl]piperidine. $^1$H NMR ($CDCl_3$) δ 8.15-8.03 (m, 3H), 3.94 (s, 3H), 3.30-3.15 (m, 2H), 2.90-2.65 (m, 4H), 2.72 (s, 3H), 1.95-1.50 (m, 4H); MS m/z 282 (M+H).

**Example 31**

**N-[2-(S)-(3,4-Dichlorophenyl)-4-[4-[2-methylsulfinyl-4-aminophenyl]-1-piperidinyl]-butyl]-N-methyl-2-methoxy-3-cyano-1-naphthamide citrate.**

Using standard reductive amination conditions N-[2-(S)-(3,4-dichlorophenyl)]-4-oxobutyl-N-methyl-3-cyano-2-methoxy-1-naphthamide (0.091 g) was reacted with 4-[2-methylsulfinyl-4-aminophenyl]-piperidine (0.048g) and converted to the citrate salt. MS m/z 677 (M+); $^1$H NMR (DMSO $d_6$) δ 8.75-8.55 (m), 8.20-7.25 (m), 7.15-6.00 (m), 4.51 (t), 4.10-3.20 (m), 3.18-2.30 (m), 2.15-1.60 (m).

The requisite 4-[2-methylsulfinyl-4-aminophenyl]-piperidine was prepared as follows.

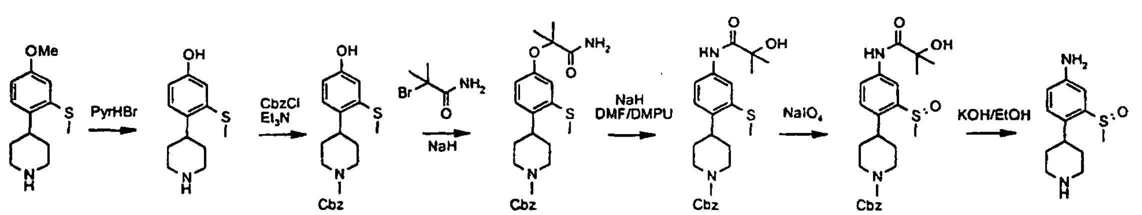


A B C D E F G

(a) 4-[4-Methoxy-2-methylthiophenyl]piperidine.

N-deprotection of 1-Cbz-4-[4-methoxy-2-methylthiophenyl]piperidine (Example 7, step f) was achieved by stirring in TFA for 1 h at 80 °C. The mixture was neutralized by addition of KOH, then extracted into chloroform to afford the product. $^1$H NMR ($CDCl_3$) δ 7.15 (d, 1H), 6.76 (d, 1H), 6.69 (dd, 1H), 3.80 (s, 3H), 3.18 (dm, 2H), 3.01 (tt, 1H), 2.78 (td, 2H), 2.45 (s, 3H), 1.82 (m, 2H), 1.66 (s, 1H), 1.58 (qd, 2H); MS m/z 238 (M+H).

(b) 4-[4-Hydroxy-2-methylthiophenyl]piperidine hydrobromide.

A mixture of pyridinium hydrobromide (20.76 g) and 4-[4-methoxy-2-methylthiophenyl]piperidine (6.16g) was heated to 225° C for 18 h. The reaction mixture was cooled, dissolved in 200 mL water, adjusted to pH 7 with IN KOH, and extracted with hexane. The aqueous layer was concentrated under reduced pressure to give an oil which was dissolved in 200 mL EtOH and stirred for 0.5 h. The precipitate filtered and washed with EtOH. The combined EtOH filtrates were concentrated under reduced pressure and purified by chromatography (9:1 DCM:MeOH) to give 6.06 g product. $^1$H NMR (DMSO $d_6$) δ 9.44 (s, 1H), 8.49 (m, 2H), 6.97 (d, 1H), 6.66 (d, 1H), 6.58 (dd, 1H), 3.43-3.30 (dm, 2H), 3.13-2.95 (m, 3H), 2.42 (s, 3H), 1.91-1.61 (m, 4H); MS m/z 225 (M+H).

(c) 1-Cbz-4-[4-Hydroxy-2-methylthiophenyl]piperidine.

To a rapidly stirred slurry of 4-[4-hydroxy-2-methylthiophenyl]piperidine hydrobromide.(2.57g) and triethylamine (4.00 mL) in 200 mL THF was slowly added 2.50 mL of benzyl chloroformate over 10 min. Aqueous $NaHCO_3$ was added, THF was removed under reduced pressure, the residue dissolved in DCM, washed with aqueous $NaHCO_3$, concentrated to an oil, and re-dissolved in 160 mL 1:1 THF:water. To this was added 0.26g LiOH and the mixture was stirred for 18 h. THF was evaporated under reduced pressure, the aqueous residue was acidified with 15 mL IN HCl, and extracted with DCM, dried, and concentrated under reduced pressure to give an oil which was purified by chromatography (2:3 EtOAc:hexane) to give 1.71g of solid product. $^1$H NMR ($CDCl_3$) δ 7.45-7.25 (m, 5H), 6.99 (d, 1H), 6.70 (d, 1H), 6.59 (dd, 1H), 5.16 (s, 2H), 5.03 (s, 1H), 4.41-4.25 (m, 2H), 3.04 (tt, 1H), 3.00-2.83 (m, 2H), 2.44 (s, 3H), 1.90-1.45 (m, 4H); MS m/z 358 (M+H).

(d) 1-Cbz-4-[4-aminocarbonyl(dimethyl)methyloxy)-2-methylthiophenyl]piperidine.

1-Cbz-4-[4-hydroxy-2-methylthiophenyl]piperidine (0.951 g) was reacted NaH (0.160 g 60% mineral oil dispersion) in dioxane solvent (15 mL) at room temperature for 2 h. This was treated with 2-bromo-2-methylpropanamide (Coutts and Southcott; *J. Chem. Soc., Perkin Trans. 1*, **1990**, 767) (0.662) and heated at 100 °C for 2 h. The mixture was poured into 30 mL saturated $NaHCO_3$ and extracted with DCM, dried, and concentrated under reduced pressure to give an oil which was purified by chromatography (40:1 DCM:MeOH) to afford 0.835 g of solid product. $^1$H NMR ($CDCl_3$) δ 7.45-7.25 (m, 5H), 7.03 (d, 1H), 6.78 (d, 1H), 6.70 (dd, 1H), 6.60 (m, 1H), 5.62 (m, 1H), 5.16 (s, 2H), 4.43-4.21 (m, 2H), 3.06 (tt, 1H), 3.00-2.87 (m, 2H), 2.44 (s, 3H), 1.90-1.45 (m, 4H), 1.54 (s, 6H); MS m/z 443 (M+H).

(e) 4-[4-(2-hydroxy-2-methylpropionamido)-2-methylthiophenyl]-N-Cbz-piperidine.

4-[4-aminocarbonyl(dimethyl)methyloxy)-2-methylthiophenyl]-N-Cbz-piperidine (0.835 g) was reacted with NaH (0.106 g 60% mineral oil dispersion) in 10 mL DMF containing 1.0 mL 1,3-dimethyl-3,4,5,6,-tetrahydro-2(1H)-pyrimidone and heated to 100°C for 2 h. The mixture was poured into 50 mL water and extracted with 1:1 EtOAc:$Et_2O$, washed with water, dried, and concentrated under reduced pressure to give an oil which was purified by chromatography (40:1 DCM:MeOH) to give 0.491 g of solid product; $^1$H NMR ($CDCl_3$) δ 8.65 (m, 1H), 7.70 (d, 1H), 7.45-7.25 (m, 5H), 7.17 (dd, 1H), 7.09 (d, 1H), 5.16 (s, 2H), 4.43-4.21 (m, 2H), 3.09 (tt, 1H), 3.03-2.87 (m, 2H), 2.50 (s, 3H), 1.90-1.45 (m, 4H), 1.56 (s, 3H), 1.56 (s, 3H); MS m/z 443 (M+H).

(f) 4-[4-(2-Hydroxy-2-methylpropionamido)-2-methylsulfinylphenyl]-N-Cbz-piperidine.

To a stirred solution of $NaIO_4$ (1.56g) in 100 mL 1:1 THF:water was added 4-[4-(2-hydroxy-2-methylpropionamido)-2-methylthiophenyl]-N-Cbz-piperidine (0.65g). After 18 h, THF was removed under reduced pressure, and the residue was diluted with 60 mL saturated $NaHCO_3$ and extracted with DCM, dried and evaporated to give 0.585g of an oil after chromatography (20:1 DCM:MeOH). $^1$H NMR ($CDCl_3$) δ 8.92 (m, 1H), 8.05 (dd, 1H), 7.84 (d, 1H), 7.45-7.25 (m, 5H), 7.26 (d, 1H), 5.16 (s, 2H), 4.43-4.22 (m, 2H), 2.97-2.78 (m, 3H), 2.71 (s, 3H), 1.90-1.45 (m, 4H), 1.57 (s, 3H), 1.55 (s, 3H); MS m/z 459 (M+H).

(g) 4-[4-Amino-2-methylsulfinylphenyl]piperidine.

To a solution of KOH (0.72 g) in 40 mL of 1:1 EtOH:water was added 0.585 g 4-[4-(2-hydroxy-2-methylpropionamido)-2-methylsulfinylphenyl]-N-Cbz-piperidine. The resulting mixture was heated under reflux under nitrogen for 36 h, evaporated, dissolved in 10 mL water, and extracted with $CHCl_3$, dried, filtered, and concentrated under reduced pressure. The resulting residue was purified by chromatography (9:1, DCM:MeOH with 2% aqueous $NH_4OH$) to give 0.148 g of white solid. The purified product was a 2:3 mixture of 4-[4-amino-2-methylsulfinylphenyl]piperidine and 4-[4-(2-hydroxy-2-methylpropionamido)-2-methylsulfinylphenyl]-piperidine. as determined by $^1H$ NMR. This mixture was used directly in the subsequent reaction. $^1H$ NMR ($CDCl_3$) δ 8.67 (m), 8.06 (dd), 7.82 (d), 7.35 (d), 7.13 (d, 1H), 6.76 (dd), 3.82 (m), 3.30-3.10 (m), 2.90-2.60 (m), 2.71 (s), 2.67 (s), 1.90-1.45 (m); MS m/z 239 (M+H).

## Example 32

**N-[(S)-2-(3,4-Dichlorophenyl)-4-[5-methoxy-2-methylsulfinylphenyl]-1-piperidinyl]-butyl]-N-methyl-3-cyano-2-methoxy-1-naphthamide citrate.**

Using standard reductive amination conditions N-[2-(S)-(3,4-dichlorophenyl)]-4-oxobutyl-N-methyl-3-cyano-2-methoxy-1-naphthamide (0.242 g) was reacted with 4-[5-methoxy-2-methylsulfinylphenyl]-1-piperidine (0.134 g) and converted to the citrate salt. MS: m/z 692 (M+); $^1H$ NMR (DMSO-$d_6$) δ 8.75-8.60 (m), 8.20-6.70 (m), 6.33 (d), 4.54 (t), 4.10-3.60 (m), 3.55-3.00 (m), 2.98-2.30 (m), 2.20-1.60 (m).

The requisite 4-[5-methoxy-2-methylsulfinylphenyl]-1-piperidine was prepared according to the procedures described in Example 7 except 4-methoxyphenol was used in place of 3-methoxyphenol; therefore, 4-methoxyphenol (11.38 g) was reacted with bromine (14.89g) to give 18.54 g of crude 2-bromo-4-methoxyphenol. $^1H$ NMR ($CDCl_3$) δ 7.01 (d, 1H), 6.94 (d, 1H), 6.80 (dd, 1H), 5.14 (s, 1H), 3.75 (s, 3H). Analytical data for all other intermediates follows. 2-Bromo-4-methoxy-(N,N-dimethylthiocarbamoyl)phenol; $^1H$ NMR ($CDCl_3$) δ 7.12 (d, 1H), 7.05 (d, 1H), 6.85 (dd, 1H), 3.80 (s, 3H), 3.47 (s, 3H), 3.36 (s, 3H); MS m/z 290 (M+). 5-Methoxy-2-(N,N-dimethylthiocarbamoyl)bromobenzene; $^1H$ NMR ($CDCl_3$) δ 7.50 (d, 2H), 7.24 (d, 1H), 6.87 (dd, 1H), 3.81 (s, 3H), 3.20-1.92 (m, 6H); MS m/z 290 (M+). 5-Methoxy-2-(methylthio)bromobenzene; $^1H$ NMR ($CDCl_3$) δ 7.19 (d, 1H), 7.15 (d, 1H), 6.87 (dd, 1H), 3.79 (s, 3H), 2.45 (s, 3H). 4-Hydroxy-4-(5-methoxy-2-methylthiophenyl)-1-N-Cbz-piperidine; $^1H$ NMR ($CDCl_3$) δ 7.43 (d, 1H), 7.43-7.25 (m, 5H), 6.89 (d, 1H), 6.80 (dd, 1H), 5.15 (s, 2H), 4.25-4.00 (m, 2H), 3.80 (s, 1H), 3.50-3.25 (m, 2H), 2.47 (s, 3H), 2.15-1.90 (m, 4H); MS m/z 370 (M-$H_2O$). 4-(5-Methoxy-2-methylthiophenyl)-1-N-Cbz-piperidine; $^1H$ NMR ($CDCl_3$) δ 7.50-7.28 (m, 6H), 6.80-6.65 (m, 2H), 5.14 (s, 2H), 4.42-4.20 (m, 2H), 3.79 (s, 3H), 3.28 (tt, 1H), 3.00-2.90 (m, 2H), 2.40 (s, 3H), 2.05-1.50 (m, 4H); MS m/z 372 (M+H). 4-(5-Methoxy-2-methylsulfinylphenyl)-1-N-Cbz-piperidine; $^1H$ NMR ($CDCl_3$) δ 7.91 (d, 1H), 7.42-7.30 (m, 5H), 6.98 (dd, 1H), 6.76 (d, 1H), 5.16 (s, 2H), 4.45-4.22 (m, 2H), 3.84 (s, 3H), 3.03 (tt, 1H), 3.00-2.85 (m, 2H), 2.69 (s, 3H), 1.98-1.55 (m, 4H); MS m/z 388 (M+H). 4-(5-Methoxy-2-methylsulfinylphenyl)-piperidine; $^1H$ NMR ($CDCl_3$) δ 7.91 (d, 1H), 6.97 (dd, 1H), 6.87 (d, 1H), 3.84 (s, 3H), 3.28-3.12 (m, 2H), 2.92 (tt, 3H), 2.74 (td, 1H), 2.68 (s, 3H), 1.88 (dm, 2H), 1.76 (qd, 2H); MS m/z 254 (M+H).

## Example 33

**N-[(S)-2-(3,4-Dichlorophenyl)-4-[4-methoxy-2-methylsulfinylphenyl]-1-piperidinyl]-butyl]-N-methyl-3-cyano-2-methylsulfonyl-1-naphthamide citrate.**

Using standard reductive amination conditions N-[2-(S)-(3,4-dichlorophenyl)]-4-oxobutyl-N-methyl-3-cyano-2-methylsulfonyl-1-naphthamide (0.255 g) was reacted with 4-[4-methoxy-2-methylsulfinylphenyl]-1-piperidine (0.132 g) and converted to the citrate salt. MS: m/z 740 (M+); $^1H$ NMR (DMSO-$d_6$) δ 9.00-8.82 (m), 8.32-6.80 (m), 6.47 (d), 4.66 (t), 4.20-3.00 (m), 2.95-2.21 (m), 2.20-1.60 (m); analysis for $C_{37}H_{39}Cl_2N_3O_5S_2$•1.0 citric acid•1.0 $H_2O$: calculated; C, 54.31; H, 5.19; N, 4.42; found; C, 54.03; H, 5.05; N, 4.36.

The requisite N-[2-(S)-(3,4-dichlorophenyl)]-4-oxobutyl-N-methyl-3-cyano-2-methylsulfonyl-1-naphthamide was prepared as follows.

(a) Methyl 3-cyano-2-methylthio-1-naphthoate.

Methyl-3-cyano-2-trifluoromethanesulfonyloxy-1-naphthoate (6.25 g) was reacted with sodium thiomethoxide (2.46 g) in toluene according to the method of Zheng; *J. Org. Chem.*, **1998**, *63*, 9606, except that stoichiometric amounts of Pd(OAc) (0.42 g) and (*S*)-(-)-2,2'-bis(diphenylphosphino)-1,1'-binaphthyl (1.25 g) used were used here. The mixture was heated to 100 °C for 66 h, diluted with 200 mL EtOAc and 100 mL DCM, extracted with 20% $K_2CO_3$, and brine, dried, and concentrated under reduced pressure to give an oil which was purified by chromatography (DCM) to give

3.40 g of solid product. $^1$H NMR ($CDCl_3$) δ 8.33 (s, 1H), 7.92 (m, 1H), 7.78-7.60 (m, 3H), 4.09 (s, 3H), 2.58 (s, 3H).

(b) 3-Cyano-2-methylthio-1-naphthoic acid.

Methyl 3-cyano-2-methylthio-1-naphthoate (3.20 g) was stirred with 20 g trimethylsilyl iodide and heated to 70°C for 2 h. The reaction was quenched by the slow addition of 20 mL water, diluted with 300 mL DCM and extracted with 150 mL 5% NaHSO. The organic layer was dried and concentrated under reduced pressure to give a yellow solid. $^1$H NMR ($CDCl_3$) δ 8.38 (s, 1H), 7.96 (m, 2H), 7.78 (m, 2H), 7.70 (m, 2H), 2.64 (s, 3H).

(c) N-[2-(S)-(3,4-dichlorophenyl)]-4-hydroxybutyl-N-methyl-3-cyano-2-methylthio-1-naphthamide.

3-Cyano-2-methylthio-1-naphthoyl chloride (2.36 g) (prepared from 3-cyano-2-methylthio-1-naphthoic acid using oxalyl chloride under standard conditions) was combined with N-[2-(S)-(3,4-dichlorophenyl)-4-hydroxybutyl]-N-methylamine (2.58 g) and triethyl amine under standard acylation conditions to afford the product (4.24 g) as a solid after purification by chromatography (4:1 EtOAc:DCM); MS: m/z 473 (M+); $^1$H NMR ($CDCl_3$) δ 8.45-8.20 (m), 8.00-7.20 (m), 7.05-6.40 (m), 4.47 (m), 4.20-3.05 (m), 2.70-2.22 (m), 2.18-1.40 (m).

(d) N-[2-(S)-(3,4-Dichlorophenyl)]-4-hydroxybutyl-N-methyl-3-cyano-2-methylsulfonyl-1-naphthamide.

To a solution of N-[2-(S)-(3,4-dichlorophenyl)]-4-hydroxybutyl-N-methyl-3-cyano-2-methylthio-1-naphthamide (0.804 g) in 50 mL HOAc was added 3.2 mL of 30% $H_2O_2$. The mixture was heated to 50 °C for 5 h, HOAc was evaporated under reduced pressure, the residue was slowly mixed with 30 mL saturated $NaHCO_3$, extracted with DCM, dried, and concentrated under reduced pressure to give a solid (0.604 g) after purification by chromatography (40:1 DCM:MeOH); MS: m/z 505 (M+); $^1$H NMR ($CDCl_3$) δ 8.55-8.38 (m), 8.10-7.10 (m), 7.00-6.50 (m), 4.69 (dd), 4.20 (dd), 3.81-3.15 (m), 2.75-2.55 (m), 2.22-1.40 (m).

(e) N-[2-(S)-(3,4-Dichlorophenyl)]-4-oxobutyl-N-methyl-3-cyano-2-methylsulfonyl-1-naphthamide.

N-[2-(S)-(3,4-Dichlorophenyl)]-4-hydroxybutyl-N-methyl-3-cyano-2-methylsulfonyl-1-naphthamide (0.604 g) was reacted with oxalyl chloride and DMSO under standard Swern oxidizing conditions in DCM (35 mL) to afford the product (0.512 g) as a solid after extraction with DCM and purification by chromatography (1:1 DCM:EtOAc); MS: m/z 503 (M+); $^1$H NMR ($CDCl_3$) δ 9.77 (s) 8.55-8.38 (m), 8.10-7.10 (m), 7.00-6.55 (m), 4.67 (dd), 4.20-4.00 (m), 3.85-3.55 (m), 2.70 (s), 2.60 (s), 3.40-2.40 (m).

## Example 34

**N-[(S)-2-(3,4-Dichlorophenyl)-4-[4-[4-chloro-2-methylsulfinylphenyl]-1-piperidinyl]-butyl]-N-methyl-3-cyano-2-methoxy-1-naphthamide citrate.**

Using standard reductive amination conditions N-[2-(S)-(3,4-dichlorophenyl)]-4-oxobutyl-N-methyl-3-cyano-2-methoxy-1-naphthamide (0.310 g) was reacted with 4-[4-chloro-2-methylsulfinylphenyl]-1-piperidine (0.179 g) to afford the product as a white powder (0.245 g) and converted to the citrate salt. MS: m/z 696 (M+); $^1$H NMR (DMSO-$d_6$) δ 8.75-8.60 (m), 8.15-7.92 (m), 7.82-6.75 (m), 6.32 (d), 4.53 (t), 4.15-3.65 (m), 3.60-2.91 (m), 2.90-2.30 (m), 2.20-1.50 (m); analysis for $C_{36}H_{36}Cl_3N_3O_3S$•1.0 citric acid•1.0 $H_2O$: calculated; C, 55.60; H, 5.11; N, 4.63; found; C, 55.82; H, 5.00; N, 4.75.

The requisite 4-(4-chloro-2-(R,S)-methylsulfinylphenyl)piperidine was prepared according to the procedures described in Example 7 except 3-chlorophenol was used in place of 3-methoxyphenol. The oxidation of the methylthio adduct was carried out according to the procedure described in Example 31 (f). 3-Chlorophenol (24.28 g) was reacted with bromine (29.78 g) to give 6.15 g of 2-bromo-5-chlorophenol (minor isomer) and 24.60 g 4-bromo-3-chlorophenol (major isomer) after purification by column chromatography (10:1 hexane:EtOAc); minor isomer-$^1$H NMR ($CDCl_3$) δ 7.37 (d, 1H), 7.04 (d, 1H), 6.82 (dd, 1H), 5.55 (s, 1H). Major isomer: $^1$H NMR ($CDCl_3$) δ 7.36 (d, 1H), 6.91 (d, 1H), 6.57 (dd, 1H), 5.75 (s, 1H). Analytical data for all other intermediates follows. 2-Bromo-5-chloro-(N,N-dimethylthiocarbamoyl) phenol; $^1$H NMR ($CDCl_3$) δ 7.52 (d, 1H), 7.18 (d, 1H), 7.13 (dd, 1H), 3.47 (s, 3H), 3.39 (s, 3H); MS m/z 296 (M+). 4-Chloro-2-(N,N-dimethylthiocarbamoyl)-bromobenzene; $^1$H NMR ($CDCl_3$) δ 7.68-7.55 (m, 2H), 7.23 (dd, 1H), 3.12 (s, 3H), 3.05 (s, 3H); MS m/z 296 (M+). 4-Chloro-2-(thiomethyl)bromobenzene; $^1$H NMR ($CDCl_3$) δ 7.43 (d, 1H), 7.06 (d, 1H), 6.97 (dd, 1H), 2.48 (s, 3H). 1-Benzyloxycarbonyl-4-hydroxy-4-(4-chloro-2-methylthiophenyl)piperidine; $^1$H NMR ($CDCl_3$) δ 7.43-7.30 (m, 6H), 7.26 (d, 1H), 7.15 (dd, 1H) 5.15 (s, 2H), 4.25-4.00 (m, 2H), 3.84 (s, 1H), 3.50-3.25 (m, 2H), 2.52 (s, 3H), 2.15-1.90 (m, 4H); MS m/z 414 (M+Na). 1-Benzyloxycarbonyl-4-(4-chloro-2-methylthiophenyl)-

piperidine; $^1H$ NMR ($CDCl_3$) δ 7.43-7.30 (m, 5H), 7.18-7.10 (m, 3H), 5.16 (s, 2H), 4.42-4.20 (m, 2H), 3.07 (tt, 1H), 3.00-2.80 (m, 2H), 2.47 (s, 3H), 1.91-1.45 (m, 4H); MS m/z 398 (M+Na). 1-Benzyloxycarbonyl-4-(4-chloro-2-(R,S)-methylsulfinylphenyl)piperidine; $^1H$ NMR ($CDCl_3$) δ 7.98 (d, 1H), 7.42 (dd, 1H), 7.41-7.30 (m, 5H), 7.21 (d, 1H), 5.16 (s, 2H), 4.43-4.21 (m, 2H), 2.96-2.78 (m, 3H), 2.71 (s, 3H), 1.92-1.51 (m, 4H). 4-(4-Chloro-2-(R,S)-methylsulfinylphenyl) piperidine; $^1H$ NMR ($CDCl_3$) δ 7.97 (d, 1H), 7.43 (dd, 1H), 7.28 (d, 1H), 3.30-3.10 (m, 2H), 2.71 (s, 3H), 2.83-2.61 (m, 3H), 1.92-1.51 (m, 5H); MS m/z 258 (M+H).

<u>Example 35</u>

**N-[(S)-2-(3,4-Dichlorophenyl)-4-[4-[4-(2-hydroxy-2-methylpropionamido)-2-methylsulfinylphenyl]-1-piperidinyl]butyl]-N-methyl-3-cyano-2-methoxy-1-naphthamide citrate.**

Using standard reductive amination conditions N-[2-(S)-(3,4-dichlorophenyl)]-4-oxobutyl-N-methyl-3-cyano-2-methoxy-1-naphthamide (0.137 g) was reacted with 4-[(2-hydroxy-2-methylpropionamido)-2-methylsulfinylphenyl]-piperidine (0.098 g) (prepared from 4-[4-(2-hydroxy-2-methylpropionamido)-2-methylsulfinylphenyl]-N-Cbz-piperidine [Example 31, step (f)] by N-deprotection according to the conditions described for Example 31, step (g)) to afford the product as a white powder (0.068 g) and converted to the citrate salt. MS: m/z 763 (M+); $^1H$ NMR (DMSO-$d_6$) δ 9.90-9.80 (m), 8.77-8.60 (m), 8.35-8.22 (m), 8.15-7.98 (m), 7.90-6.75 (m), 6.32 (d), 5.71 (s), 4.54 (t), 4.15-3.65 (m), 3.60-3.00 (m), 2.98-2.30 (m), 2.25-1.55 (m), 1.35 (s). The requisite 4-[(2-hydroxy-2-methylpropionamido)-2-methylsulfinylphenyl]-piperidine was prepared as described for example 31.

<u>Example 36</u>

**N-[(S)-2-(3,4-Dichlorophenyl)-4-[4-[4-methoxy-(S)-2-methylsulfinylphenyl]-1-piperidinyl]butyl]-N-methyl-3-cyano-2-methylthio-1-naphthamide citrate.**

N-[(S)-2-(3,4-Dichlorophenyl)-4-[4-[4-methoxy-(S)-2-methylsulfinylphenyl]-1-piperidinyl]butyl]-N-methylamine (0.270) was combined with 3-cyano-2-methylthio-1-naphthoyl chloride (0.143 g) (prepared from 3-cyano-2-methylthionaphthoic acid [Example 33] and oxalyl chloride) and triethyl amine under standard acylation conditions to afford a white powder (0.388 g) which was converted to the citrate salt. MS: m/z 708 (M+); $^1H$ NMR (DMSO-$d_6$) δ 8.78-8.70 (m), 8.17-7.97 (m), 7.86-6.77 (m), 6.42 (d), 4.48 (t), 4.15-3.65 (m), 3.63-2.91 (m), 2.90-1.50 (m); analysis for $C_{37}H_{39}Cl_2N_3O_3S_2$•1.0 citric acid•0.5 $H_2O$: calculated; C, 56.76; H, 5.32; N, 4.62; found; C, 56.95; H, 5.26; N, 4.59.

<u>Example 37</u>

**N-[(S)-2-(3,4-Dichlorophenyl)-4-[4-[(S)-2-methylsulfinylphenyl]-1-piperidinyl]butyl]-N-methyl-3-cyano-2-methylthio-1-naphthamide citrate.**

N-[(S)-2-(3,4-Dichlorophenyl)-4-[4-[(S)-2-methylsulfinylphenyl]-1-piperidinyl]butyl]-N-methylamine (0.248 g) was combined with 3-cyano-2-thiomethyl-1-naphthoyl chloride (0.145 g) and triethyl amine under standard acylation conditions to afford a white powder (0.351 g) which was converted to the citrate salt. MS: m/z 678 (M+); $^1H$ NMR (DMSO-$d_6$) δ 8.79-8.69 (m), 8.20-8.00 (m), 7.91-6.73 (m), 6.42 (d), 4.48 (t), 4.18 (dd), 3.71 (dd), 3.60-2.95, (m), 2.94-1.55 (m); analysis for $C_{36}H_{37}Cl_2N_3O_2S_2$•1.0 citric acid•0.5 $H_2O$: calculated; C, 57.33; H, 5.27; N, 4.78; found; C, 57.44; H, 5.26; N, 4.82.

<u>Example 38</u>

**N-[(S)-2-(3,4-Dichlorophenyl)-4-[4-[(S)-2-methylsulfinylphenyl]-1-piperidinyl]butyl]-N-methyl-3-cyano-2-methylsulfinyl-1-naphthamide citrate.**

Using standard reductive amination conditions N-[(S)-2-(3,4-dichlorophenyl)-4-oxobutyl]-N-methyl-2-methylsulfinyl-3-cyano-1-naphthamide (0.213 g) was reacted with 4-(2-(S)-methylsulfinylphenyl)piperidine (0.108 g) to afford the product as a white powder (0.254 g) which was converted to the citrate salt. MS: m/z 694 (M+); $^1H$ NMR (DMSO-$d_6$) δ 9.00-8.75 (m), 8.25-8.03 (m), 7.95-6.85 (m), 6.57 (d), 6.43 (d), 4.51 (q), 4.33 (dd), 3.91-3.80 (m), 3.64 (dd), 3.58-2.31, (m), 2.29-1.58 (m).

The requisite aldehyde was prepared as follows.

(a) N-[(S)-2-(3,4-Dichlorophenyl)-4-oxobutyl]-N-methyl-2-methylthio-3-cyano-1-naphthamide.

N-[(S)-2-(3,4-Dichlorophenyl)-4-hydroxybutyl]-N-methyl-2-methylthio-3-cyano-1-naphthamide (1.40 g) was reacted with oxalyl chloride and DMSO under standard Swem oxidizing conditions in DCM (80 mL) to afford N-[(S)-2-(3,4-dichlorophenyl)-4-oxobutyl]-N-methyl-2-thiomethyl-3-cyano-1-naphthamide (1.31 g) as a solid after aqueous extraction from DCM and purification by chromatography (10:1 DCM:EtOAc); MS: m/z 471 (M+); $^1$H NMR ($CDCl_3$) δ 9.85-9.72 (m), 8.40-8.18 (m), 8.10-7.21 (m), 7.10-6.95 (m), 6.92 (d), 6.76 (d), 6.24 (d), 6.54 (dd), 4.40 (dd), 4.25 (dd), 3.98-3.41 (m), 3.40-2.80 (d), 2.72-2.30 (m).

(b) N-[(S)-2-(3,4-Dichlorophenyl)-4-oxobutyl]-N-methyl-2-methylsulfinyl-3-cyano-1-naphthamide.

To a solution of N-[(S)-2-(3,4-dichlorophenyl)-4-oxobutyl]-N-methyl-2-methylthio-3-cyano-1-naphthamide (1.31 g) in 30 mL HOAc was added 4.5 mL of 30% aqueous $H_2O_2$. The mixture was heated to 35 °C for 2 h, HOAc was evaporated under reduced pressure, the residue was slowly mixed with 30 mL saturated aqueous $NaHCO_3$, and extracted with DCM to give a solid (1.35 g) after purification by chromatography (20:1 DCM:MeOH); MS: m/z 487 (M+); $^1$H NMR ($CDCl_3$) δ 9.85-9.52 (m), 8.60-8.20 (m), 8.18-6.57 (m), 6.61 (d), 6.37 (d), 4.69 (qm), 4.24 (dd), 3.37 (ddd), 4.50-2.40 (m), 2.25-1.80 (m).

**Example 39**

**N-[(S)-2-(3,4-Dichlorophenyl)-4-[4-[4-methoxy-(S)-2-methylsulfinylphenyl]-1-piperidinyl]butyl]-N-methyl-3-cyano-2-methylsulfinyl-1-naphthamide citrate.**

Using standard reductive amination conditions N-[(S)-2-(3,4-dichlorophenyl)-4-oxobutyl]-N-methyl-2-methylsulfinyl-3-cyano-1-naphthamide (0.215 g) was reacted with 4-(4-methoxy-2-(S)-methylsulfinylphenyl)piperidine (0.112 g) to afford the product as a white powder (0.320 g) which was converted to the citrate salt. MS: m/z 724 (M+); $^1$H NMR (DMSO-$d_6$) δ 9.00-8.78 (m), 8.25-8.03 (m); 7.91-6.80 (m), 6.57 (d), 6.43 (d), 4.51 (q), 4.33 (dd), 3.82 (s), 3.65 (dd), 3.60-2.30, (m), 2.29-1.55 (m).

**Example 40**

**N-[(S)-2-(3,4-Dichlorophenyl)-4-[4-[(S)-2-methylsulfinylphenyl]-1-piperidinyl]butyl]-N-methyl-3-cyano-2-methylsulfonyl-1-naphthamide citrate.**

Using standard reductive amination conditions N-[(S)-2-(3,4-dichlorophenyl)-4-oxobutyl]-N-methyl-2-methylsulfonyl-3-cyano-1-naphthamide (0.256 g) (Example 33) was reacted with 4-(2-(S)-methylsulfinylphenyl)piperidine (0.117 g) to afford the product as a white powder (0.268 g) and converted to the citrate salt. MS: m/z 710 (M+); $^1$H NMR (DMSO-$d_6$) δ 9.06-8.83 (m), 8.34-6.80 (m), 6.47 (d), 4.66 (t), 4.17-4.05 (m), 3.63-3.00 (m), 2.98-1.52 (m); analysis for $C_{36}H_{37}Cl_2N_3O_4S_2$•1.0 citric acid•1.0 $H_2O$: calculated; C, 54.78; H, 5.04; N, 4.52; found; C, 54.78; H, 5.04; N, 4.52.

**Example 41**

**N-[(S)-2-(3,4-Dichlorophenyl)-4-[4-[(S)-2-methylsulfinylphenyl]-1-piperidinyl]butyl]-N-methyl-3-aminocarbonyl-2-methoxy-1-naphthamide citrate.**

N-[(S)-2-(3,4-Dichlorophenyl)-4-[4-[(S)-2-methylsulfinylphenyl]-1-piperidinyl]butyl]-N-methylamine (0.267g) was reacted with 3-aminocarbonyl-2-methoxy-1-naphthoic acid (0.288g) and 1-(3-dimethylaminopropyl)-3-ethylcarbodiimide and converted to the citrate salt. MS m/z 680 (M+); $^1$H NMR (DMSO $d_6$) δ 8.25-8.20 (m, 1H), 8.1-6.8 (m, 10H), 6.2 (d, J= 3Hz, 1H), 4.6 (t, J = 10Hz, 1H), 3.8 (s, 3H), 2.2-1.6 (m, 5H); mp 160-170 (d).

The requisite 2-methoxy-3-carboxamido-1-naphthoic acid was prepared as follows.

(a) 1-Iodo-2-methoxy-3-naphthoic acid.

A solution of 1-iodo-2-methoxy-3-carbomethoxynaphthalene (22.2 g) (Example 1) in dioxane (200 mL) was treated with a solution of potassium hydroxide (7.27 g) in water (50 mL) followed by methanol (100 mL). The reaction mixture was stirred for 2 h , diluted with water, acidified with hydrochloric acid and extracted with DCM. The organic layer was dried and concentrated to afford the product. MS (APCI, negative ion mode) m/z = 328 ($M^-$).

(b) 1-Iodo-2-methoxy-3-naphthamide.

A solution of 1-iodo-2-methoxy-3-naphthoic acid (20.68 g) in DCM was cooled to 0 °C and treated with 5 drops of DMF followed by oxalyl chloride (11 mL) and the reaction mixture was stirred at room temperature for 16h. At the end of this period the reaction mixture was concentrated under reduced pressure, dissolved in THF, cooled to 0 °C and treated with aqueous ammonia (100 mL). The reaction mixture was diluted with EtOAc, washed with water, dried and concentrated under reduced pressure to afford the product. MS APCI, m/z = 328 ($M^+$); $^1H$ NMR (300 MHz, $CDCl_3$) δ 8.8 (s, 1H), 8.2 (d, 1H, J= 10 Hz), 7.9 (d, 1H, *J* = 10 Hz), 7.6 (m, 2H), 7.5 (m, 1H), 6 (br, 1H), 3.9 (s, 3H).

(c) Methyl 2-methoxy-3-carboxamido-1-naphthaoate.

A solution of 1-iodo-2-methoxy-3-naphthamide (1.57 g) in methanol was treated with palladium acetate (0.108 g), triethylamine (1 mL) and heated under an atmosphere of carbon monoxide for 16 h. At the end of this period the reaction mixture filtered through a pad of celite, concentrated under reduced pressure and purified by chromatography to afford the product. MS APCI, m/z = 260 ($M^+$); $^1H$ NMR (300 MHz, $CDCl_3$) δ 8.85 (s, 1H), 8.0 (d, 1H, *J* = 10 Hz), 7.8 (d, 1H, *J* = 10 Hz), 7.6 (m, 2H), 7.5 (t, 1H, *J* = 10 Hz), 6.0 (s, 1H), 4.1 (s, 3H), 4.0 (s, 3H).

(d) 2-Methoxy-3-carboxamido-1-naphthoic acid.

A solution of methyl 2-methoxy-3-carboxamido-1-naphthaoate (0.79 g) in methanol was treated with potassium hydroxide (0.36 g) in water (5 mL). The reaction mixture was heated under reflux for 72 h, diluted with water and extracted with ether. The aqueous layer was acidified with hydrochloric acid and extracted with EtOAc. Upon drying and concentration under reduced pressure the desired product was obtained; MS APCI, m/z = 244 ($M^-$); $^1H$ NMR (300 MHz, $CDCl_3$) d 8.8 (s, 1H), 8.0 (m, 1H), 7.7 (m, 1H), 7.6 (m, 1H), 7.5 (m, 1H), 4.0 (m, 3H).

**Example 42**

**N-[(S)-2-(3,4-Dichlorophenyl)-4-[4-[(S)-2-methylsulfinylphenyl]-1-piperidinyl]butyl]-N-ethyl-3-aminocarbonyl-2-methoxy-1-naphthamide citrate.**

N-[(S)-2-(3,4-Dichlorophenyl)-4-[4-[(S)-2-ethylsulfinylphenyl]-1-piperidinyl]butyl]-N-ethylamine (0.63 g) (Example 10) was reacted with 3-aminocarbonyl-2-methoxy-1-naphthoic acid (0.26 g) and 1-(3-dimethylaminopropyl)-3-ethylcarbodiimide, then converted to the citrate salt. MS m/z 694 (M+); $^1H$ NMR (DMSO $d_6$) δ 8.25-8.15 (m, 1H), 8.0-6.8 (m, 10H), 6.4(d, J= 10Hz, 1H), 4.4(t, J = 10Hz, 1H), 3.8(s, 3H), 2.2-1.6(m, 5H); mp 160-170 (d).

**Example 43**

**N-[(S)-2-(3,4-Dichlorophenyl)-4-[4-[4-methoxy-(S)-2-methylsulfinylphenyl]-1-piperidinyl]butyl]-3-cyano-2-methoxy-1-naphthamide.**

Using standard reductive amination conditions, a mixture of N-[2-(S)-(3,4-dichlorophenyl)]-4-oxobutyl-3-cyano-2-methoxy-1-naphthamide and N-[2-(S)-(3,4-dichlorophenyl)]-4-oxobutyl-N-carbomethoxymethyl-2-methoxy-3-cyano-1-naphthamide (1.14 g) was reacted 4-[4-methoxy-(S)-2-methylsulfinylphenyl]-1-piperidine (0.696 g). The resulting reaction mixture was purified by chromatography to afford the title compound. MS m/z 678 (M+); $^1H$ NMR ($CDCl_3$) δ 8.2 (s, 1H), 7.8(d, J= 10Hz, 1H), 7.7 (d, J= 10Hz, 1H), 7.6-7.4 (m, 6H), 7.2 (d,d, J1 = 3Hz, J2 = 10Hz, 1H), 6.95 (d,d, J1= 3Hz, J2=10Hz, 1H), 6.7 (d, J= 10Hz, 1H), 5.3 (s, 1H), 4.1 (s, 3H), 4.0 (m, 1H), 3.8 (s, 3H), 3.7 (m, 1H), 3.0 (m, 2H), 2.8 (m, 1H), 2.6 (s, 3H), 2.4 (m, 2H), 2.0 (m, 4H), 1.6-1.2 (m, 6H); mp 120-130 (d).

The mixture containing N-[2-(S)-(3,4-dichlorophenyl)]-4-oxobutyl-3-cyano-2-methoxy-1-naphthamide and N-[2-(S)-(3,4-dichlorophenyl)]-4-oxobutyl-N-carbomethoxymethyl-2-methoxy-3-cyano-1-naphthamide was prepared as follows.

(a) N-[2-(S)-(3,4-Dichlorophenyl)-4-hydroxybutyl]-N-carbomethoxymethylamine-3-cyano-2-methoxy-1-naphthalenecarboxamide.

A solution of (S)-2-(3,4-dichlorophenyl)-4-hydroxybutylamine (4.68 g) in methanol (50 mL) was treated with 2-hydroxy-2-methoxyacetic acid methyl ester (2.2 mL), acetic acid (2.36 mL) and sodium cyanoborohydride (2.0 g). After stirring for 16 h the reaction mixture was concentrated under reduced pressure, dissolved in DCM and washed with potassium carbonate solution, dried and concentrated to afford N-[2-(S)-(3,4-dichlorophenyl)-4-hydroxybutyl]-N-carbomethoxymethylamine. This material was treated with triethylamine (4.2 mL) and 3-cyano-2-methoxy-1-naphthoyl chloride and dimethylaminopyridine (0.28 g). After stirring for 16 h at room temperature the reaction mixture was diluted with DCM, washed with 5% hydrochloric acid followed by water, dried and concentrated, and purified by chromatography to afford the titled product. MS APCI, m/z = 515 ($M^+$); $^1H$ NMR (300 MHz, $CDCl_3$) δ 8.25 (m, 1H), 7.4 (m, 7H), 4.0 (m, 6H).

(b) N-[2-(S)-(3,4-Dichlorophenyl)-4-oxobutyl]-N-carbomethoxymethylamine-3-cyano-2-methoxy-1-naphthalenecarboxamide.

To a solution of oxalyl chloride (0.76 mL) in DCM (100 mL) at -78 C was added DMSO (1.06 mL) followed by after 5 min. a solution of N-[2-(S)-(3,4-dichlorophenyl)-4-hydroxybutyl]-N-carbomethoxymethylamine-3-cyano-2-methoxy-1-naphthalenecarboxamide (3.75 g) in DCM (100 mL). After 30 min the reaction mixture was treated with triethylamine (4.2 mL) and allowed to warm to the room temperature. Upon stirring for 1 h the reaction mixture was diluted with water and the organic layer was dried and concentrated under reduced pressure to afford a crude product. Purification by column chromatography afforded the desired product. $^1H$ NMR (300 MHz, $CDCl_3$) δ 9.6 (m, 1H), 8.3 (m, 1H), 7.5 (m, 7H), 4.0 (m, 6H).

<u>**Example 44**</u>

**N-[(S)-2-(3,4-Dichlorophenyl)-4-[4-[4-fluoro-(S)-2-methylsulfinylphenyl]-1-piperidinyl]-butyl]-3-cyano-2-methoxy-1-naphthamide.**

According to the procedure described for Example 43, the mixture containing N-[2-(S)-(3,4-dichlorophenyl)]-4-oxobutyl-N-(carbomethoxymethyl)-2-methoxy-3-cyano-1-naphthamide was reacted with 4-[4-fluoro-(S)-2-methylsulfinylphenyl]-1-piperidine to afford the product. MS (APCI, negative ion mode) m/z 666 (M-); $^1H$ NMR ($CDCl_3$) δ 8.2 (s, 1H), 7.8 (d, J= 10Hz, 1H), 7.7 (d, J= 10Hz, 1H), 7.6-7.4 (m, 6H), 7.2 (d,d J1 = 3Hz, J2 = 10Hz, 1H), 7.1 (m, 1H), 6.8 (m, 1H), 5.3 (s, 1H), 4.1 (s, 3H), 4.0 (m, 1H), 3.7 (m, 1H), 3.0 (m, 2H), 2.8 (m, 1H), 2.6 (s, 3H), 2.4 (m, 2H), 2.0 (m, 4H), 1.7-1.2 (m, 6H)); mp 125-140 (d). The requisite 4-[4-fluoro-(S)-2-methylsulfinylphenyl]-1-piperidine was prepared according to the procedure described in Example 7 except 2-bromo-5-methoxyphenol was replaced with 2-bromo-5-fluorophenol.

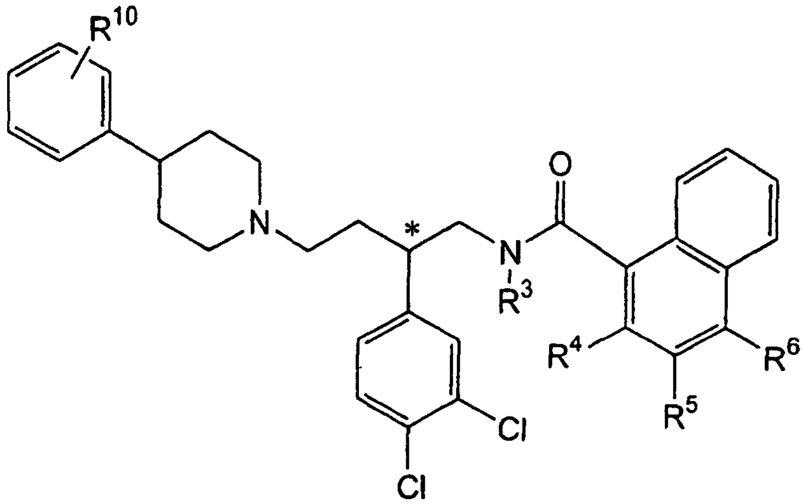

Table 1.

| Synthesis and selected experimental data for Tachykinin antagonists. 2-Dichlorophenyl-butyl chiral center is of the (S) configuration unless specified otherwise. Compounds containing a basic nitrogen were converted to citrate salts. | | | | | | | |
|---|---|---|---|---|---|---|---|
| | $R^3$ | $R^4$ | $R^5$ | $R^6$ | $R^{10}$ | MS (m/z) | Synth |
| 45 | -Me | -OMe | -CN | -H | 2-(P(O)(OEt)$_2$) | 736 | B (1) |

Table 1. (continued)

| Synthesis and selected experimental data for Tachykinin antagonists. 2-Dichlorophenyl-butyl chiral center is of the (S) configuration unless specified otherwise. Compounds containing a basic nitrogen were converted to citrate salts. | | | | | | | |
|---|---|---|---|---|---|---|---|
| | $R^3$ | $R^4$ | $R^5$ | $R^6$ | $R^{10}$ | MS (m/z) | Synth |
| 46 | -Me | -OMe | -CN | -H | 2-(2-oxazoline) | 669 | B (2) |
| 47 | -Me | -OMe | -CN | -H | 2-(2-pyridone) | 693 | B |
| 48 | -Me | -OMe | -CN | -H | 2-(S)-S(O)Me, 4-F | 680 | B |
| 49 | -Me | -OMe | -CN | -H | 2-S(O)Me, 4-S (O)Me | 724 | B |
| 50 | -Me | -OMe | -CN | -H | 2-S(O)Me, 4-OH | 678 | B |
| 51 | -Me | -OMe | -CN | -H | 2-S(O)Me, 4-C(O)$NH_2$ | 705 | B |
| 52‡ | -Me | -OMe | -CN | -H | 2-(S)-S(O)Me | 662 | B |
| 53 | -Et | -OMe | -CN | -H | 4-S(O)Me | 676 | |
| 54 | -tBu | -OMe | -CN | -H | 2-(S)-S(O)Me | 704 | A (3) |
| 55 | -tBu | -OMe | -CN | -H | 2-(S)-S(O)Me, 4-OMe | 734 | A (3) |
| 56 | -Et | -OMe | -CN | -H | 2-(R)-S(O)Me | 676 | A |
| 57 | -Et | -OMe | -CN | -H | 2-$S(O)_2$Me | 692 | A |
| 58 | -Me | -O-2-propyl | -CN | -H | 2-(S)-S(O)Me | 692 | A (4) |
| 59 | -Me | -OMe | -Me | -H | 2-(S)-S(O)Me | 651 | A (5) |
| 60 | -Me | -OMe | -$CH_2$CN | -H | 2-(S)-S(O)Me | 676 | A (6) |
| 61 | -Me | -Et | -CN | -H | 2-(S)-S(O)Me, 4-F | 678 | B |
| 62 | -Me | -Et | -CN | -H | 2-(S)-S(O)Me, 4-OMe | 690 | B |
| 63 | -Me | -$CH_2CH(Me)_2$ | -CN | -H | 2-(S)-S(O)Me | 688 | A (7) |
| 64 | -Me | -Et | -CN | -H | 2-(R)-S(O)Me | 660 | B |
| 65 | -Me | -$C(CH_2)$Me | -CN | -H | 2-(S)-S(O)Me | 672 | A (8) |
| 66 | -Me | -Me | -CN | -OMe | 2-(S)-S(O)Me | 676 | A (10) |
| 67 | -Me | -OMe | -CN | -OMe | 2-(S)-S(O)Me | 692 | A (11) |
| 68 | -Et | -OMe | -CN | -H | 2-S(O)Me, 4-OMe | 706 | A |
| 69† | -cylclopropyl | -OMe | -CN | -H | 2-(S)-S(O)Me | 688 | A (3) |
| 70† | -cylclopropyl | -OMe | -CN | -H | 2-(S)-S(O)Me, 4-OMe | 732 | A (3) |
| 71† | -methylcylclopropyl | -OMe | -CN | -H | 2-(S)-S(O)Me | 688 | A (3) |
| 72† | -methylcylclopropyl | -OMe | -CN | -H | 2-(S)-S(O)Me, | 732 | A (3) |
| 73 | -Me | -OH | -H | -H | 2-(S)-S(O)Me | 623 | A |
| 74 | -Me | -OH | -CN | -H | 2-S(O)Me | 648 | A (12) |
| 75 | -Me | -Ph | -CN | -H | 2-(S)-S(O)Me | 708 | A |

Table 1. (continued)

| Synthesis and selected experimental data for Tachykinin antagonists. 2-Dichlorophenyl-butyl chiral center is of the (S) configuration unless specified otherwise. Compounds containing a basic nitrogen were converted to citrate salts. | | | | | | | |
|---|---|---|---|---|---|---|---|
| | $R^3$ | $R^4$ | $R^5$ | $R^6$ | $R^{10}$ | MS (m/z) | Synth |
| 76‡ | -Me | -OMe | -CN | -H | 2-(R)-S(O)Me | 663 | A |
| 77 | -Me | -OMe | -CN | -H | 2-S(O)Me,<br>5-CH,Ph | 767 | B<br><br>(13) |
| 78 | -Me | $-OCH_2-$ | | -H | 2-(S)-S(O)Me | 651 | A<br>(14) |

A: Compound was prepared by combining the appropriate substituted naphthoyl chloride with the appropriate substituted amine according to the specified standard acylation conditions. B: Compound was prepared by combining the appropriate substituted piperidine with the appropriate substituted aldehyde according to the specified standard reductive amination conditions. ‡2-Dichlorophenyl-butyl chiral center is of the (R) configuration. †This compound is an epimeric mixture containing the (R) and (S) 2-dichlorophenyl-butyl isomers. (1) 4-(2-Trifluoromethanesulfonyloxyphenyl)-1-N-Cbz-piperidine was reacted with diethylphosphite according to the conditions described by Petrakis, et al.; J. Am. Chem. Soc., 1987, 2831 to afford 4-(2-(diethylphosphono)phenyl)-1-N-Cbz-piperidine which was N-deprotected by hydrogenation to afford the requisite 4-(2-(diethylphosphono)phenyl)-piperidine. (2) 4-(2-Carboxyphenyl)-1-N-Boc-piperidine was converted to the corresponding acid chloride using oxalyl chloride and reacted with 2-bromoethylamine, according to the conditions described by Elworthy, et al., J. Med. Chem., 1997, 2674, to provide the substituted oxazoline. This material was N-deprotected by treatment with 10% TFA in DCM to provide the requisite 4-(2-oxazolin-2-ylphenyl)-piperidine. (3) Material was prepared by reductive amination of the appropriate cyclopropyl or t-butyl substituted amine with 3,4-dichloro-alpha-2-propenylbenzene acetaldehyde (Shenvi, A; Jacobs, RT; Miller, SC; Ohnmacht, CJ; Veale, CA. EP 680962), followed by acylation with the appropriate substituted naphthoyl chloride using standard acylation conditions, then olefin oxidative cleavage using sodium periodate, oxidation of the resulting primary alcohol to the corresponding aldehyde using osmium tetroxide, then reductive amination with the appropriate substituted piperidine. (4) Methyl 3-cyano 2-isopropyloxy-1-naphthoate was prepared by heating methyl 3-cyano-2-hydroxy-1-naphthoate with potassium carbonate and isoproyl bromide in acetone, then saponified to afford 3-cyano 2-isopropyloxy-1-naphthoic acid. (5) Methyl 2-methoxy-3-methyl-1-naphthoate was prepared by treatment of 2-hydroxy-3-methoxynaphthalene (Ansink, HRW; Zelvelder, E; Cerfontain, H.; Recl. Trav. Chim. Pays-Bas, 1993, 216) with trifluoromethanesulfonic acid, then reacted with methyl boronic acid according to Example 15 (c) to afford 2-methyl-3-methoxynaphthalene. This material was treated with N-bromosuccinimide to afford 1-bromo-2-methoxy-3-methyl-naphthalene, then reacted according the conditions for Example 1 (d). The methyl ester was then saponified to afford 2-methoxy-3-methyl-1-naphthoate. (6) Methyl 2-methoxy-3-methyl-1-naphthoate was treated with N-bromosuccinimide in carbon tetrachloride, then reacted with potassium cyanide in ethanol-water, then saponified to afford 2-methoxy-3-cyanomethylnaphthoate. (7) Methyl 3-cyano-2-isobutyl-1-naphthoic acid was prepared according to the procedure described for Example 15, except 2-methylpropylboronic acid was used in place of methylboronic acid. This material was saponified to afford 3-cyano-2-isobutyl-1-naphthoic acid. (8) Methyl 3-cyano-2-propenyl-1-naphthoic acid was prepared according to the procedure described for Example 15, except propenylboronic acid was used in place of methylboronic acid. This material was saponified to afford 3-cyano-2-(propenyl)-1-naphthoic acid. (10) Ethyl-3-cyano-4-methoxy-2-trifluoromethanesulfonyloxy-1-naphthoate (Example 17) was reacted with methylboronic acid according to the procedure described for Example 15, then saponified to afford 3-cyano-2-methyl-4-methoxy-1-naphthoate. (11) Ethyl-3-bromo-2,4-dimethoxy-1-naphthoate Example 16) was reacted with CuCN according to the procedure for Example 17 (a)), then saponified to afford 3-cyano-2,4-dimethoxy-1-naphthoic acid. (12) The material from Example Example 1 was demethylated according to the conditions described for Example 15 (a) to afford the product. (13) N-Cbz-4-(4-bromo-2-(R,S)-methylsulfinylphenyl)piperidine was prepared according to the procedure described for N-Cbz-4-(4-chloro-2-(R,S)-methylsulfinylphenyl)-piperidine Example 34 except 3-bromophenol was used in place of 3-chlorophenol. This material was reacted with benzylamine, CuI, and $K_2CO_3$ in reflucing DMF for 2 h based on the procedure of Wisansky, WA; Ansbacher, S; J. Am. Chem. Soc.; 1941, 2532, then Cbz-deprotected by heating in TFA at 100 °C for 2 h to afford 4-(4-bromo-2-methylsulfinylphenyl)piperidine. (14) Naphtho[2,3-d]-1,3-dioxole-4-carboxylic acid was prepared according to Dallacker, F.; et al.; Z. Naturforsch; 1979, 1434.

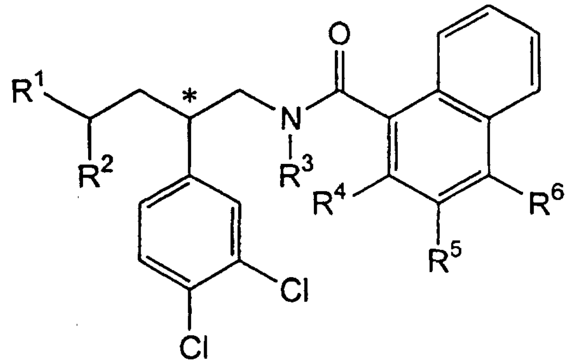

Table 2.

| Selected experimental data for Tachykinin antagonists. 2-Dichlorophenylbutyl chiral center is of the (S) configuration unless specified otherwise. These materials were prepared using procedures and intermediates as described within the above text or elsewhere. Compounds containing a basic nitrogen were converted to citrate salts. | | | | | | | |
|---|---|---|---|---|---|---|---|
| | $R^3$ | $R^4$ | $R^5$ | $R^6$ | $R^7$ | $R^{12}$ | MS (m/z) |
| 79 | -H | $-OSO_2Me$ | -CN | -H | -OH | -H | 507 |
| 80 | -Me | -SMe | -CN | -H | -OH | -H | 473 |
| 81 | -Me | -S(O)Me | -CN | -H | -OH | -H | 489 |
| 82 | -Me | $-SO_2Me$ | -CN | -H | -OH | -H | 505 |
| 83‡ | -Me | -OMe | -CN | -H | -OH | -H | 457 |
| 84‡ | -Me | -OMe | -CN | -H | =O | -H | 455 |
| 85 | -tBu | -OMe | -CN | -H | -OH | -H | 499 |
| 86 | -Me | -Et | -CN | -H | -OH | -H | 455 |
| 87 | -Me | -OMe | -H | -H | =O | -H | 430 |

‡2-Dichlorophenyl-butyl chiral center is of the (R) configuration.

## Claims

**1.** A compound having the formula

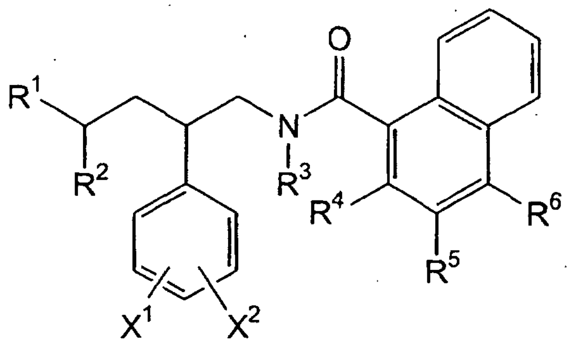

wherein:

$R^1$ is oxo, $-OR^a$, $-OC(=O)R^b$; or

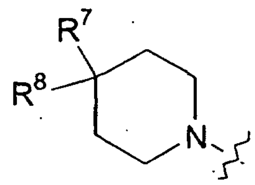

$R^2$ is H; or
$R^1$ is $-OR^c$ and $R^2$ is $-OR^d$; or
$R^1$ and $R^2$ together form $-O(CH_2)_mO-$;
$R^3$ is H or $C_{1-6}$alkyl;
$R^4$ is independently selected from hydroxy, halo, $C_{1-6}$alkoxy, $C_{1-6}$alkyl, cyano$C_{1-6}$alkyl, $C_{2-6}$alkenyl, $C_{2-6}$alkynyl, carboxy, $C_{1-6}$alkoxy-carbonyl, carbamoyl, $C_{1-6}$alkylcarbamoyl, di-$C_{1-6}$alkylcarbamoyl, $C_{1-6}$alkanoyl, $C_{1-6}$alkanoylamino and aminosulfonyl;
$R^5$ is independently selected from hydroxy, cyano, nitro, trifluoromethoxy, trifluoromethyl, $C_{1-6}$alkylsulfonyl, halo, $C_{1-6}$alkoxy, $C_{1-6}$alkyl, cyano$C_{1-6}$alkyl, $C_{2-6}$alkenyl, $C_{2-6}$alkynyl, carboxy, $C_{1-6}$alkoxy-carbonyl, carbamoyl, $C_{1-6}$alkylcarbamoyl, di-$C_{1-6}$alkylcarbamoyl, $C_{1-6}$alkanoyl, $C_{1-6}$alkanoylamino, aminosulfonyl, and substituted$C_{1-6}$alkyl; or
$R^4$ and $R^5$ together form $-OCH_2O-$ or $-OC(CH_3)_2O-$;
$R^6$ is selected from hydrogen, hydroxy, cyano, nitro, trifluoromethoxy, trifluoromethyl, $C_{1-6}$alkylsulfonyl, halo, $C_{1-6}$alkoxy, $C_{1-6}$alkyl, cyano$C_{1-6}$alkyl, $C_{2-6}$alkenyl, $C_{2-6}$alkynyl, carboxy, $C_{1-6}$alkoxy-carbonyl, carbamoyl, $C_{1-6}$alkylcarbamoyl, di-$C_{1-6}$alkylcarbamoyl, $C_{1-6}$alkanoyl, $C_{1-6}$alkanoylamino, aminosulfonyl, and substituted $C_{1-6}$alkyl
$R^7$ is phenyl substituted in at least the ortho position by a substituent selected from $C_{1-6}$alkylthio, $C_{1-6}$alkylsulfinyl, $C_{1-6}$alkylsulfonyl, trifluoromethylthio, trifluoromethylsulfinyl, $C_{1-6}$alkanesulfonamido, $C_{1-6}$alkanoyl, $C_{1-6}$alkoxy-carbonyl, succinamido, carbamoyl, $C_{1-6}$alkylcarbamoyl, di-$C_{1-6}$alkylcarbamoyl, $C_{1-6}$alkoxy-$C_{1-6}$alkylcarbamoyl, $C_{1-6}$alkanoylamino, ureido, $C_{1-6}$ureido, di-$C_{1-6}$alkylureido, amino, $C_{1-6}$alkylamino and di-$C_{1-6}$alkylamino;
$R^8$ is selected from hydrogen, hydroxy, $C_{1-6}$alkoxy, $C_{1-6}$alkanoyloxy, $C_{1-6}$alkanoyl, $C_{1-6}$alkoxycarbonyl, $C_{1-6}$alkanoylamino, $C_{1-6}$alkyl, carbamoyl, $C_{1-6}$alkylcarbamoyl, and bis($C_{1-6}$alkyl)carbamoyl;
$R^a$ is hydrogen or $C_{1-6}$alkyl;
$R^b$ is $C_{1-6}$alkyl, phenyl or phenyl $C_{1-6}$alkyl; $R^c$ and $R^d$ are independently selected from $C_{1-6}$alkyl;
m is 2, 3, or 4; and
$X^1$ and $X^2$ are independently H or halogen, wherein at least one of $X^1$ and $X^2$ are halogen; and

any pharmaceutically-acceptable salt thereof.

**2.** A compound according to Claim 1 wherein:

$R^1$ is oxo, $-OR^a$, or $-OC(=O)R^b$; or
$R^1$ is $-OR^c$ and $R^2$ is $-OR^d$.

**3.** A compound according to Claim 1 wherein:

$R^1$ is

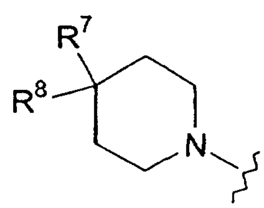

;

and
$R^2$ is H.

**4.** A compound according to Claim 3 wherein:

$R^7$ is phenyl substituted in the ortho position by a substituent selected from $C_{1-6}$alkylthio, $C_{1-6}$alkylsulfinyl, $C_{1-6}$alkylsulfonyl, trifluoromethylthio, trifluoromethylsulfinyl, $C_{1-6}$alkanesulfonamido, $C_{1-6}$alkanoyl, $C_{1-6}$alkoxycarbonyl, succinamido, carbamoyl, $C_{1-6}$alkylcarbamoyl, di-$C_{1-6}$alkylcarbamoyl, $C_{1-6}$alkoxy-$C_{1-6}$ alkylcarbamoyl, $C_{1-6}$alkanoylamino, ureido, $C_{1-6}$ureido, di-$C_{1-6}$alkylureido, amino, $C_{1-6}$alkylamino and di-$C_{1-6}$alkylamino; and substituted in the para position by a substituent selected from hydrogen, methyl, methoxy, acetyl, acetylamino, methoxycarbonyl, methanesulfonylamino, methyl-sulfinyl, methylsulfonyl, trifluoromethyl, trifluoromethylthio, trifluoromethylsulfinyl, bromo, fluoro, chloro, hydroxy, carbamoyl, methylcarbamoyl, dimethylcarbamoylmethylureido and dimethylureido; and
$R^8$ is selected from hydrogen, hydroxy, methoxycarbonyl, methylcarbamoyl and dimethylcarbamoyl.

**5.** A compound according to Claim 4 wherein:

$R^7$ is phenyl substituted in the ortho position by methylsulfinyl, methylsulfonyl, methylureido, dimethylureido, amino, methylamino or dimethylamino; and substituted in the para position by hydrogen, $C_{1-6}$alkoxy, halo, $C_{1-6}$alkylsulfinyl, or carboxy; and
$R^8$ is hydroxy or hydrogen,

**6.** A compound according to Claim 5 wherein:

$R^7$ is

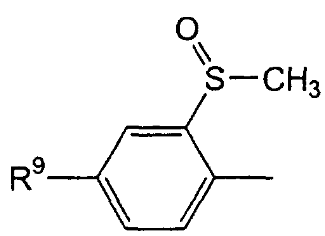

;

$R^8$ is hydrogen; and
$R^9$ is hydrogen, methoxy or fluoro.

**7.** A compound according to any one of Claims 2, 3, 4, or 6 wherein:

$R^3$ is hydrogen, methyl or ethyl;
$R^4$ is $C_{1-4}$alkoxy, $C_{1-4}$alkyl, halogen, halo$C_{1-2}$alkoxy, halo$C_{1-4}$alkyl, $-CH=CHCH_3$, $-S(O)_nCH_3$, or $-OS(O)_2CH_3$;
$R^5$ is cyano, nitro, hydrogen or halogen;
$R^6$ is hydrogen, methoxy, cyano or nitro; and
n is 0, 1 or 2.

**8.** A compound according to Claim 7 wherein:

$R^3$ is hydrogen, methyl or ethyl;
$R^4$ is methyl, ethyl, methoxy, ethoxy, hydroxy or fluoro;
$R^5$ is cyano or nitro; and
$R^6$ is hydrogen.

**9.** A process for preparing a compound according to Claim 3 which process comprises the step of:

reacting a compound of the formula (III) with a compound of the formula (IV) under reductive amination conditions:

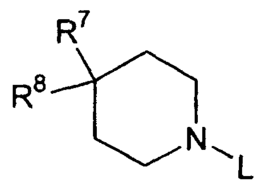

(III)

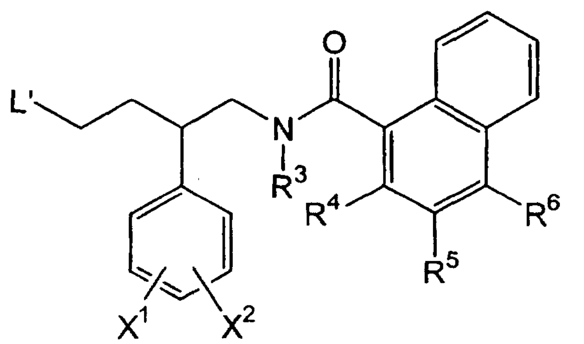

(IV)

wherein $R^3$ through $R^8$, $X^1$ and $X^2$ are as in Claim 3; and L and L' are groups such that reductive amination of the compounds of the formulae (III) and (IV) forms a N-C bond; or

reacting a compound of the formula (V) with a compound of the formula (VI):

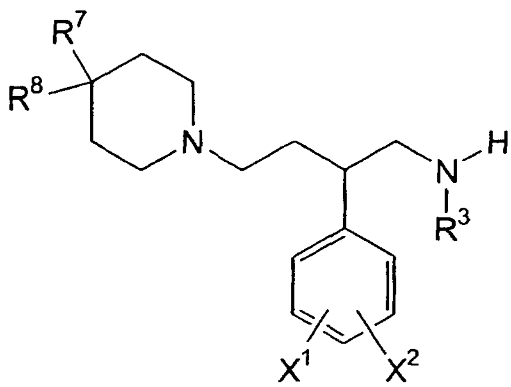

(V)

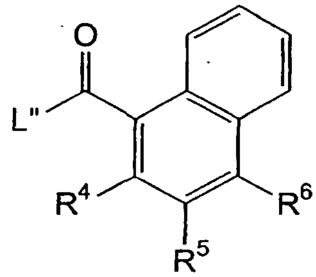

(VI)

wherein $R^3$ through $R^8$, $X^1$ and $X^2$ are as defined in Claim 3; and L" is a leaving group.

**10.** A pharmaceutical composition comprising a compound according to any one of Claims 1 through 8.

**11.** The use of an NK1 antagonist according to any one of Claims 1 through 8 for the manufacture of a medicament for treating depression, anxiety, asthma, rheumatoid arthritis, Alzheimer's disease, cancer, schizophrenia, oedema, allergic rhinitis, inflammation, pain, gastrointestinal-hypermotility, anxiety, emesis, Huntington's disease, psychoses including depression, hypertension, migraine, bladder hypermotility, or urticaria.

**Patentansprüche**

**1.** Verbindungen der Formel

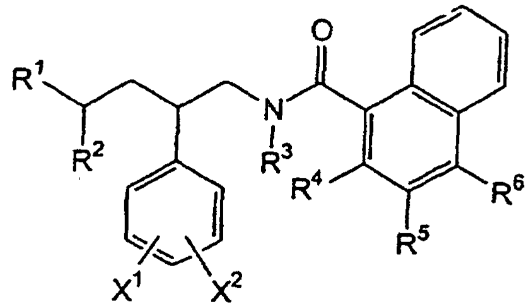

in welcher:

$R^1$ für Oxo, $-OR^a$, $-OC(=O)R^b$ oder

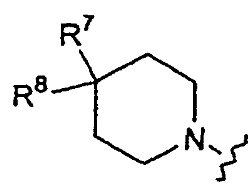

steht;

$R^2$ für H steht; oder

$R^1$ für $-OR^c$ und $R^2$ für $-OR^d$ steht; oder

$R^1$ und $R^2$ zusammen $-O(CH_2)_mO-$ bilden;

$R^3$ für H oder $C_{1-6}$-Alkyl steht;

$R^4$ unabhängig ausgewählt ist aus Hydroxy, Halogen, $C_{1-6}$-Alkoxy, $C_{1-6}$-Alkyl, Cyano-$C_{1-6}$-alkyl, $C_{2-6}$-Alkenyl, $C_{2-6}$-Alkinyl, Carboxy, $C_{1-6}$-Alkoxycarbonyl, Carbamoyl, $C_{1-6}$-Alkylcarbamoyl, Di-$C_{1-6}$-alkylcarbamoyl, $C_{1-6}$-Alkanoyl, $C_{1-6}$-Alkanoylamino und Aminosulfonyl;

$R^5$ unabhängig ausgewählt ist aus Hydroxy, Cyano, Nitro, Trifluormethoxy, Trifluormethyl, $C_{1-6}$-Alkylsulfonyl, Halogen, $C_{1-6}$-Alkoxy, $C_{1-6}$-Alkyl, Cyano-$C_{1-6}$-alkyl, $C_{2-6}$-Alkenyl, $C_{2-6}$-Alkinyl, Carboxy, $C_{1-6}$-Alkoxy-carbonyl, Carbamoyl, $C_{1-6}$-Alkylcarbamoyl, Di-$C_{1-6}$-alkylcarbamoyl, $C_{1-6}$-Alkanoyl, $C_{1-6}$-Alkanoylamino, Aminosulfonyl und substituiertem $C_{1-6}$-Alkyl; oder

$R^4$ und $R^5$ gemeinsam $-OCH_2O-$ oder $-OC(CH_3)_2O-$ bilden;

$R^6$ ausgewählt ist aus Wasserstoff, Hydroxy, Cyano, Nitro, Trifluormethoxy, Trifluormethyl, $C_{1-6}$-Alkylsulfonyl, Halogen, $C_{1-6}$-Alkoxy, $C_{1-6}$-Alkyl, Cyano-$C_{1-6}$-alkyl, $C_{2-6}$-Alkenyl, $C_{2-6}$-Alkinyl, Carboxy, $C_{1-6}$-Alkoxycarbonyl, Carbamoyl, $C_{1-6}$-Alkylcarbamoyl, Di-$C_{1-6}$-alkylcarbamoyl, $C_{1-6}$-Alkanoyl, $C_{1-6}$-Alkanoylamino, Aminosulfonyl und substituiertem $C_{1-6}$-Alkyl;

$R^7$ für Phenyl steht, das wenigstens in der Ortho-Stellung durch einen Substituenten ausgewählt aus $C_{1-6}$-Alkylthio, $C_{1-6}$-Alkylsulfinyl, $C_{1-6}$-Alkylsulfonyl, Trifluormethylthio, Trifluormethylsulfinyl, $C_{1-6}$-Alkansulfonamido, $C_{1-6}$-Alkanoyl, $C_{1-6}$-Alkoxy-carbonyl, Succinamido, Carbamoyl, $C_{1-6}$-Alkylcarbamoyl, Di-$C_{1-6}$-alkylcarbamoyl, $C_{1-6}$-Alkoxy-$C_{1-6}$-alkylcarbamoyl, $C_{1-6}$-Alkanoylamino, Ureido, $C_{1-6}$-Ureido, Di-$C_{1-6}$-alkylureido, Amino, $C_{1-6}$-Alkylamino und Di-$C_{1-6}$-Alkylamino substituiert ist;

$R^8$ aus Wasserstoff, Hydroxy, $C_{1-6}$-Alkoxy, $C_{1-6}$-Alkanoyloxy, $C_{1-6}$-Alkanoyl, $C_{1-6}$-Alkoxycarbonyl, $C_{1-6}$-Alkanoylamino, $C_{1-6}$-Alkyl, Carbamoyl, $C_{1-6}$-Alkylcarbamoyl und Bis($C_{1-6}$-alkyl)carbamoyl ausgewählt ist;

$R^a$ für Wasserstoff oder $C_{1-6}$-Alkyl steht;

$R^b$ für $C_{1-6}$-Alkyl, Phenyl oder Phenyl-$C_{1-6}$-alkyl steht;

$R^c$ und $R^d$ unabhängig voneinander aus $C_{1-6}$-Alkyl ausgewählt sind;

m für 2, 3 oder 4 steht; und

$X^1$ und $X^2$ unabhängig voneinander für H oder Halogen stehen, wobei wenigstens einer der Reste $X^1$ und $X^2$ für Halogen steht; und

deren pharmazeutisch unbedenklichen Salze.

2. Verbindungen nach Anspruch 1, wobei:

$R^1$ für Oxo, $-OR^a$, or $-OC(=O)R^b$; oder
$R^1$ für $OR^c$ und $R^2$ für $-OR^d$ steht.

3. Verbindungen nach Anspruch 1, wobei:

$R^1$ für

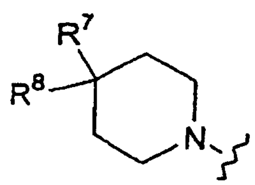

steht; und
$R^2$ für H steht.

**4.** Verbindungen nach Anspruch 3, wobei:

$R^7$ für Phenyl steht, das in der Ortho-Stellung durch einen Substituenten ausgewählt aus $C_{1-6}$-Alkylthio, $C_{1-6}$-Alkylsulfinyl, $C_{1-6}$-Alkylsulfonyl, Trifluormethylthio, Trifluormethylsulfinyl, $C_{1-6}$-Alkansulfonamido, $C_{1-6}$-Alkanoyl, $C_{1-6}$-Alkoxy-carbonyl, Succinamido, Carbamoyl, $C_{1-6}$-Alkylcarbamoyl, Di-$C_{1-6}$-alkylcarbamoyl, $C_{1-6}$-Alkoxy-$C_{1-6}$-alkylcarbamoyl, $C_{1-6}$-Alkanoylamino, Ureido, $C_{1-6}$-Alkylureido, Di-$C_{1-6}$-alkylureido, Amino, $C_{1-6}$-Alkylamino und Di-$C_{1-6}$-Alkylamino substituiert ist und in der Para-Stellung durch einen Substituenten ausgewählt aus Wasserstoff, Methyl, Methoxy, Acetyl, Acetylamino, Methoxycarbonyl, Methansulfonylamino, Methylsulfinyl, Methylsulfonyl, Trifluormethyl, Trifluormethylthio, Trifluormethylsulfinyl, Brom, Fluor, Chlor, Hydroxy, Carbamoyl, Methylcarbamoyl, Dimethylcarbamoylmethylureido und Dimethylureido substituiert ist; und

$R^8$ aus Wasserstoff, Hydroxy, Methoxycarbonyl, Methylcarbamoyl und Dimethylcarbamoyl ausgewählt ist.

**5.** Verbindungen nach Anspruch 4, wobei:

$R^7$ für Phenyl steht, das in der Ortho-Stellung durch Methylsulfinyl, Methylsulfonyl, Methylureido, Dimethylureido, Amino, Methylamino oder Dimethylamino substituiert ist und in der Para-Stellung durch Wasserstoff, $C_{1-6}$-Alkoxy, Halogen, $C_{1-6}$-Alkylsulfinyl oder Carboxy substituiert ist; und
$R^8$ für Hydroxy oder Wasserstoff steht.

**6.** Verbindungen nach Anspruch 5, wobei:

$R^7$ für

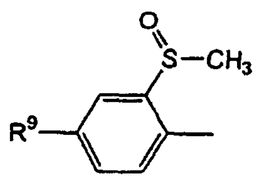

steht;
$R^8$ für Wasserstoff steht; und
$R^9$ für Wasserstoff, Methoxy oder Fluor steht.

**7.** Verbindungen nach einem der Ansprüche 2, 3, 4 oder 6, wobei:

$R^3$ für Wasserstoff, Methyl oder Ethyl steht;

$R^4$ für $C_{1-4}$-Alkoxy, $C_{1-4}$-Alkyl, Halogen, Halogen-$C_{1-2}$-alkoxy, Halogen-$C_{1-4}$-alkyl, $-CH=CHCH_3$, $-S(O)_nCH_3$ oder $-OS(O)_2CH_3$ steht;

$R^5$ für Cyano, Nitro, Wasserstoff oder Halogen steht;

$R^6$ für Wasserstoff, Methoxy, Cyano oder Nitro steht; und

n für 0, 1 oder 2 steht.

**8.** Verbindungen nach Anspruch 7, wobei:

$R^3$ für Wasserstoff, Methyl oder Ethyl steht;

$R^4$ für Methyl, Ethyl, Methoxy, Ethoxy, Hydroxy oder Fluor steht;

$R^5$ für Cyano oder Nitro steht; und

$R^6$ für Wasserstoff steht.

**9.** Verfahren zur Herstellung von Verbindungen nach Anspruch 3, bei dem man:

eine Verbindung der Formel (III) unter Bedingungen zur reduktiven Aminierung mit einer Verbindung der Formel (IV) umsetzt:

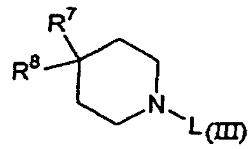

(III)

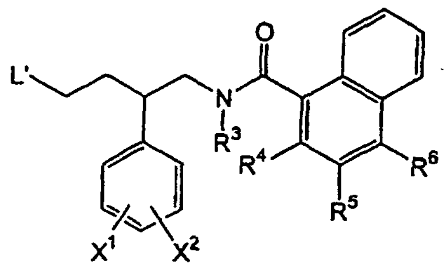

(IV)

wobei $R^3$ bis $R^8$, $X^1$ und $X^2$ wie in Anspruch 3 definiert sind und L und L' für Gruppen stehen, die bei der reduktiven Aminierung der Verbindungen der Formeln (III) und (IV) eine N-C-Bindung ausbilden; oder
eine Verbindung der Formel (V) mit einer Verbindung der Formel (VI) umsetzt:

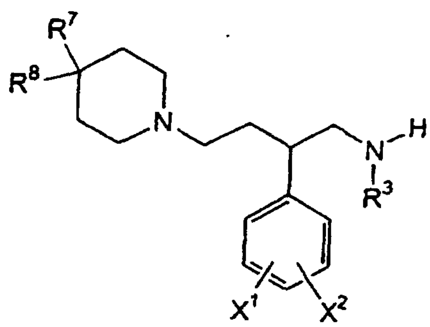

(V)

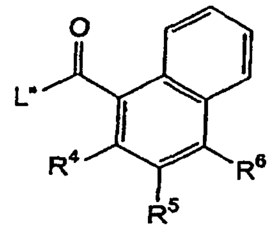

(VI)

wobei $R^3$ bis $R^8$, $X^1$ und $X^2$ wie in Anspruch 3 definiert sind und L" für eine Abgangsgruppe steht.

**10.** Pharmazeutische Zusammensetzung, enthaltend eine Verbindung nach einem der Ansprüche 1 bis 8.

**11.** Verwendung eines NK1-Antagonisten nach einem der Ansprüche 1 bis 8 zur Herstellung eines Medikaments zur Behandlung von Depression, Angstzuständen, Asthma, rheumatoider Arthritis, Alzheimer-Krankheit, Krebs, Schizophrenie, Ödemen, allergischer Rhinitis, Entzündungen, Schmerzen, gastrointestinaler Hypermotilität, Angstzuständen, Emesis, Chorea Huntington, Psychosen einschließlich Depression, Bluthochdruck, Migräne, Blasenhypermotilität oder Urticaria.

## Revendications

**1.** Composé de formule

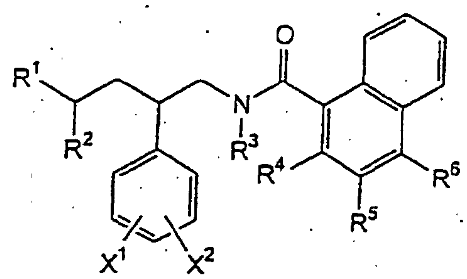

dans laquelle :

$R^1$ est oxo, $-OR^a$, $-OC(=O)R^b$ ; ou

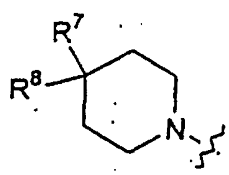

$R^2$ est H ; ou
$R^1$ est $-OR^c$ et $R^2$ est $-OR^d$ ; ou
$R^1$ et $R^2$ forment conjointement $-O(CH_2)_mO-$ ;
$R^3$ est H ou un alkyle en $C_{1-6}$ ;
$R^4$ est choisi indépendamment parmi un hydroxy, un halogéno, un alcoxy en $C_{1-6}$, un alkyle en $C_{1-6}$, un cyano-$C_{1-6}$-alkyle, un alcényle en $C_{2-6}$, un alcynyle en $C_{2-6}$, un carboxy, un $C_{1-6}$-alcoxycarbonyle, un carbamoyle, un $C_{1-6}$-alkylcarbamoyle, un di-$C_{1-6}$-alkylcarbamoyle, un alcanoyle en $C_{1-6}$, un $C_{1-6}$-alcanoylamino et un

aminosulfonyle ;
$R^5$ est choisi indépendamment parmi un hydroxy, un cyano, un nitro, un trifluorométhoxy, un trifluorométhyle, un $C_{1-6}$-alkylsulfonyle, un halogéno, un alcoxy en $C_{1-6}$, un alkyle en $C_{1-6}$, un cyano-$C_{1-6}$-alkyle, un alcényle en $C_{2-6}$, un alcynyle en $C_{2-6}$, un carboxy, un $C_{1-6}$-alcoxycarbonyle, un carbamoyle, un $C_{1-6}$-alkylcarbamoyle, un di-$C_{1-6}$-alkylcarbamoyle, un alcanoyle en $C_{1-6}$, un $C_{1-6}$-alcanoylamino, un aminosulfonyle et un alkyle en $C_{1-6}$ substitué ; ou
$R^4$ et $R^5$ forment conjointement $-OCH_2O-$ ou $-OC(CH_3)_2O-$ ;
$R^6$ est choisi parmi un hydrogène, un hydroxy, un cyano, un nitro, un trifluorométhoxy, un trifluorométhyle, un $C_{1-6}$-alkylsulfonyle, un halogéno, un alcoxy en $C_{1-6}$, un alkyle en $C_{1-6}$, un cyano-$C_{1-6}$-alkyle, un alcényle en $C_{2-6}$, un alcynyle en $C_{2-6}$, un carboxy, un $C_{1-6}$-alcoxycarbonyle, un carbamoyle, un $C_{1-6}$-alkylcarbamoyle, un di-$C_{1-6}$-alkylcarbamoyle, un alcanoyle en $C_{1-6}$, un $C_{1-6}$-alcanoylamino, aminosulfonyle et un alkyle en $C_{1-6}$ substitué ;
$R^7$ est un phényle substitué dans au moins la position ortho par un substituant choisi parmi un $C_{1-6}$-alkylthio, un $C_{1-6}$-alkylsulfinyle, un $C_{1-6}$-alkylsulfonyle, un trifluorométhylthio, un trifluorométhylsulfinyle, un $C_{1-6}$-alcanesulfonamido, un alcanoyle en $C_{1-6}$, un $C_{1-6}$-alcoxycarbonyle, un succinamido, un carbamoyle, un $C_{1-6}$-alkylcarbamoyle, un di-$C_{1-6}$-alkylcarbamoyle, un $C_{1-6}$-alcoxy-$C_{1-6}$-alkylcarbamoyle, un $C_{1-6}$-alcanoylamino, un uréido, un $C_{1-6}$-uréido, un di-$C_{1-6}$-alkyluréido, un amino, un $C_{1-6}$-alkylamino et un di-$C_{1-6}$-alkylamino ;
$R^8$ est choisi parmi un hydrogène, un hydroxy, un alcoxy en $C_{1-6}$, un $C_{1-6}$-alcanoyloxy, un alcanoyle en $C_{1-6}$, un $C_{1-6}$-alcoxycarbonyle, un $C_{1-6}$-alcanoylamino, un alkyle en $C_{1-6}$, un carbamoyle, un $C_{1-6}$-alkylcarbamoyle et un bis($C_{1-6}$-alkyl)carbamoyle ;
$R^a$ est un hydrogène ou un alkyle en $C_{1-6}$ ;
$R^b$ est un alkyle en $C_{1-6}$, un phényle ou un phényl-$C_{1-6}$-alkyle ; $R^c$ et $R^d$ sont choisis indépendamment parmi un alkyle en $C_{1-6}$ ;
m est 2, 3 ou 4 ; et
$X^1$ et $X^2$ sont indépendamment H ou un halogène, où au moins l'un parmi $X^1$ et $X^2$ est un halogène ; et

tout sel pharmaceutiquement acceptable de celui-ci.

**2.** Composé selon la revendication 1, dans lequel
$R^1$ est un oxo, $-OR^a$, ou $-OC(=O)R^b$ ; ou
$R^1$ est $-OR^c$ et $R^2$ est $-OR^d$.

**3.** Composé selon la revendication 1, dans lequel
$R^1$ est

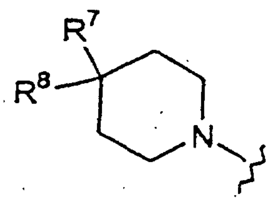

;

et
$R^2$ est H.

**4.** Composé selon la revendication 3, dans lequel :

$R^7$ est un phényle substitué dans la position ortho par un substituant choisi parmi un $C_{1-6}$-alkylthio, un $C_{1-6}$-alkylsulfinyle, un $C_{1-6}$-alkylsulfonyle, un trifluorométhylthio, un trifluorométhylsulfinyle, un $C_{1-6}$-alcanesulfonamido, un alcanoyle en $C_{1-6}$, un $C_{1-6}$-alcoxycarbonyle, un succinamido, un carbamoyle, un $C_{1-6}$-alkylcarbamoyle, un di-$C_{1-6}$-alkylcarbamoyle, un $C_{1-6}$-alcoxy-$C_{1-6}$-alkylcarbamoyle, un $C_{1-6}$-alcanoylamino, un uréido, un $C_{1-6}$-uréido, un di-$C_{1-6}$-alkyluréido, un amino, un $C_{1-6}$-alkylamino et un di-$C_{1-6}$-alkylamino ; et substitué dans la position para par un substituant choisi parmi un hydrogène, un méthyle, un méthoxy, un acétyle, un acétylamino, un méthoxycarbonyle, un méthanesulfonylamino, un méthylsulfinyle, un méthylsulfonyle, un trifluorométhyle, un trifluorométhylthio, un trifluorométhylsulfinyle, un bromo, un fluoro, un chloro, un hydroxy, un carbamoyle, un méthylcarbamoyle, un diméthylcarbamoylméthyluréido et un diméthyluréido ; et
$R^8$ est choisi parmi un hydrogène, un hydroxy, un méthoxycarbonyle, un méthylcarbamoyle et un diméthylcarbamoyle.

**5.** Composé selon la revendication 4, dans lequel :

$R^7$ est un phényle substitué dans la position ortho par un méthylsulfinyle, un méthylsulfonyle, un méthyluréido, un diméthyluréido, un amino, un méthylamino ou un diméthylamino ; et substitué dans la position para par un hydrogène, un alcoxy en $C_{1-6}$, un halogéno, un $C_{1-6}$-alkylsulfinyle ou un carboxy ; et
$R^8$ est un hydroxy ou un hydrogène.

**6.** Composé selon la revendication 5, dans lequel :

$R^7$ est

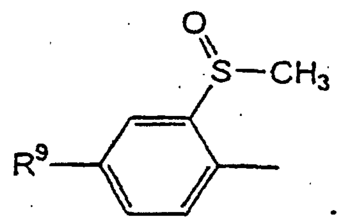

;

$R^8$ est un hydrogène ; et
$R^9$ est un hydrogène, un méthoxy ou un fluoro.

**7.** Composé selon l'une quelconque des revendications 2, 3, 4 ou 6, dans lequel :

$R^3$ est un hydrogène, un méthyle ou un éthyle ;
$R^4$ est un alcoxy en $C_{1-4}$, un alkyle en $C_{1-4}$, un halogène, un halogéno-$C_{1-2}$-alcoxy, un halogéno-$C_{1-2}$-alkyle, $-CH{=}CHCH_3$, $-S(O)_nCH_3$ ou $-OS(O)_2CH_3$ ;
$R^5$ est un cyano, un nitro, un hydrogène ou un halogène ;
$R^6$ est un hydrogène, un méthoxy, un cyano ou un nitro ; et
n vaut 0, 1 ou 2.

**8.** Composé selon la revendication 7, dans lequel :

$R^3$ est un hydrogène, un méthyle ou un éthyle ;
$R^4$ est un méthyle, un éthyle, un méthoxy, un éthoxy, un hydroxy ou un fluoro ;
$R^5$ est un cyano ou un nitro ; et
$R^6$ est un hydrogène.

**9.** Procédé de préparation d'un composé selon la revendication 3, lequel procédé comprend l'étape consistant à :

faire réagir un composé de formule (III) avec un composé de formule (IV) dans des conditions d'amination réductive :

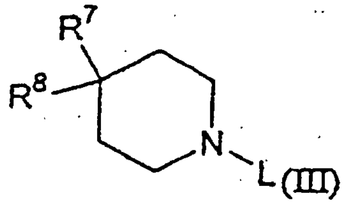

(III)

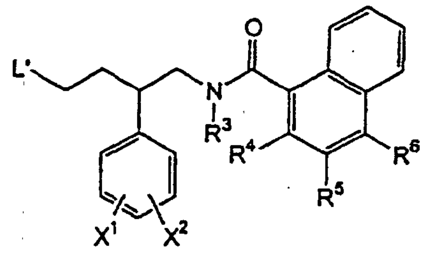

(IV)

dans lesquelles $R^3$ à $R^8$, $X^1$ et $X^2$ sont tels que dans la revendication 3 ; L et L' sont des groupes tels que l'amination réductive des composés de formules (III) et (IV) forment une liaison N-C ; ou

faire réagir un composé de formule (V) avec un composé de formule (VI) :

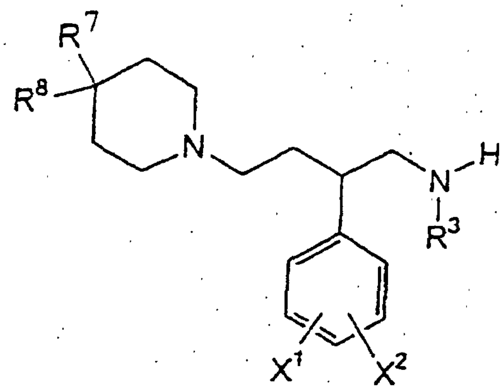

(V)

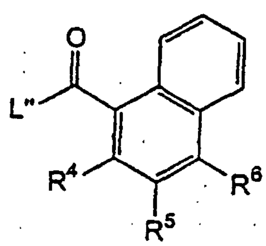

(VI)

dans lesquelles $R^3$ à $R^8$, $X^1$ et $X^2$ sont tels que définis dans la revendication 3 ; et L" est un groupe partant.

**10.** Composition pharmaceutique comprenant un composé selon l'une quelconque des revendications 1 à 8.

**11.** Utilisation d'un antagoniste de NK1 selon l'une quelconque des revendications 1 à 8, pour l'élaboration d'un médicament destiné au traitement de la dépression, de l'anxiété, de l'asthme, de la polyarthrite rhumatoïde, de la maladie d'Alzheimer, du cancer, de la schizophrénie, d'un oedème, de la rhinite allergique, de l'inflammation, de la douleur, de l'hypermotilité gastro-intestinale, du vomissement, de la maladie de Huntington, des psychoses y

compris la dépression, de l'hypertension, de la migraine, de l'hypermotilité vésicale ou de l'urticaire.